# EUROPEAN PATENT APPLICATION

(11) **EP 4 265 617 A1**
(43) Date of publication of application: **25.10.2023**
(21) Application number: 21911247.1
(22) Date of filing: 16.11.2021
(51) Int. Cl.: C07D 491/048, C07D 405/04, C07D 487/04, H01L 51/00, H01L 51/50

(54) **HETEROCYCLIC COMPOUND AND ORGANIC LIGHT-EMITTING DEVICE COMPRISING SAME**

(30) Priority: 21.12.2020 KR 20200179934
(71) Applicant: LT Materials Co., Ltd., Yongin-si, Gyeonggi-do 17118 (KR)
(72) Inventor: KIM, Ji Young, Yongin-si, Gyeonggi-do 17118 (KR); PARK, Min Ji, Yongin-si, Gyeonggi-do 17118 (KR); MO, Jun Tae, Yongin-si, Gyeonggi-do 17118 (KR); KIM, Dong Jun, Yongin-si, Gyeonggi-do 17118 (KR)
(74) Representative: Goddar, Heinz J.
(86) International application number: PCT/KR2021/016737
(87) International publication number: WO 2022/139180

(57) **Abstract**

The present invention provides a heterocyclic compound represented by Formula 1 and an organic light-emitting device comprising the same.

## Description

### [Technical Field]

This application claims the benefit of priority based on Korean Patent Application No. 10-2020-0179934 filed on December 21, 2020, the entire contents of which are incorporated herein as part of the present specification.

The present invention relates to a heterocyclic compound and an organic light-emitting device comprising the same.

### [Background Art]

An organic light-emitting device (organic light-emitting diode; OLED) has recently received a lot of attention due to an increase in demand for flat panel display devices. The organic light-emitting device is a device that converts electrical energy into light, and the performance of the organic light-emitting device is greatly affected by an organic material positioned between electrodes.

The organic light-emitting device has a structure in which an organic thin film is disposed between two electrodes. When a voltage is applied to the organic light-emitting device having such a structure, electrons and holes injected from the two electrodes combine in the organic thin film to form a pair, and then emit light while disappearing. The organic thin film may be composed of a single layer or multiple layers, if necessary.

The material for the organic thin film may have a light-emitting function, if necessary. For example, as a material for the organic thin film, a compound capable of constituting the light-emitting layer by itself may be used, or a compound capable of serving as a host or dopant of the host-dopant-based light-emitting layer may be used. In addition, as a material for the organic thin film, a compound capable of performing the roles of a hole injection layer, a hole transport layer, an electron-blocking layer, a hole-blocking layer, an electron transport layer, an electron injection layer, an electron-generating layer, and the like may be used.

In order to improve the performance, lifetime, or efficiency of the organic light-emitting device, there is a continuous demand for the development of materials for the organic thin film.

### [Prior Art References]

### [Patent Documents]

Korean Patent No. 10-1838693

### [Disclosure]

### [Technical Problem]

It is an object of the present invention to provide a heterocyclic compound capable of imparting a low driving voltage, excellent luminous efficiency, and excellent lifetime properties to an organic light-emitting device.

It is another object of the present invention to provide an organic light-emitting device comprising the heterocyclic compound.

### [Technical Solution]

The present invention provides a heterocyclic compound represented by following Formula 1: 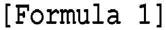 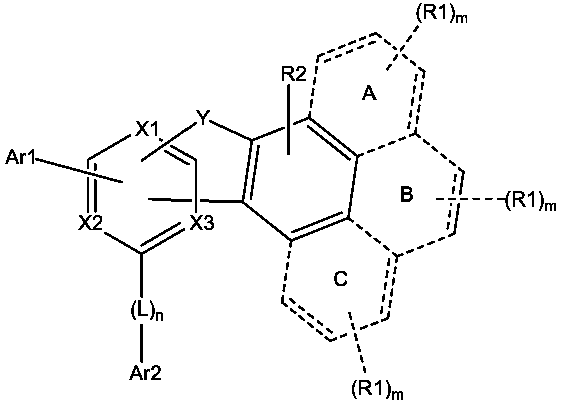 wherein,
X1, X2, and X3 are the same as or different from each other and are each independently N or C,
Y is NR20, O, or S, wherein R20 is hydrogen, deuterium, a substituted or unsubstituted C1 to C10 alkyl group, a substituted or unsubstituted C6 to C60 aryl group, or a substituted or unsubstituted C2 to C60 heteroaryl group,
rings A, B, and C are each independently present or absent, with the proviso that at least one of these rings is present,
L is a direct bond, a substituted or unsubstituted C6 to C60 arylene group, or a substituted or unsubstituted C2 to C60 heteroarylene group,
Ar1 is a substituted or unsubstituted C6 to C60 aryl group or a substituted or unsubstituted C2 to C60 heteroaryl group,
R1 and R2 are the same as or different from each other and are each independently hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; or -NR21R22, wherein R21 and R22 are the same as or different from each other and are each independently a substituted or unsubstituted C1 to C10 alkyl group, a substituted or unsubstituted C6 to C60 aryl group, or a substituted or unsubstituted C2 to C60 heteroaryl group, with the proviso that when two or more of R1 are present, each R1 is the same as or different from each other,
m is each independently an integer from 0 to 4, with the proviso that when m is 2 or more, each R1 is the same as or different from each other,
n is an integer from 0 to 3, with the proviso that when n is 2 or more, each L is the same as or different from each other, and
Ar2 is represented by following Formula 2 or 3: 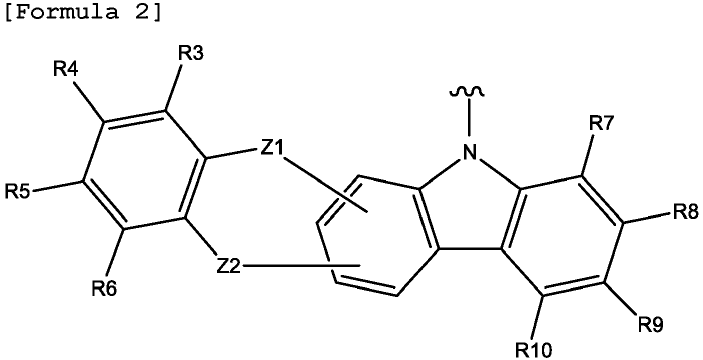 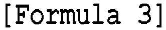 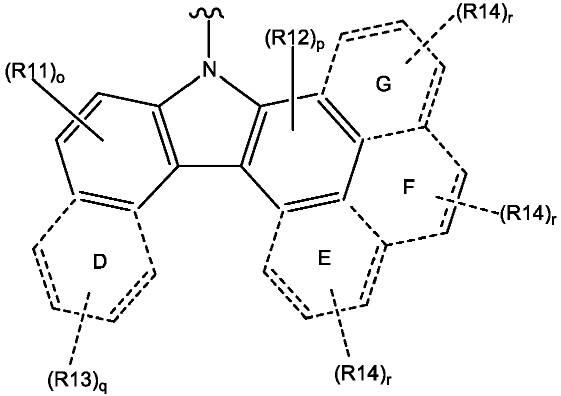 wherein,
   Z1 and Z2 are the same as or different from each other and are each independently a direct bond, NR23, O, S, or CR24R25, wherein R23 is hydrogen, deuterium, a substituted or unsubstituted C1 to C10 alkyl group, a substituted or unsubstituted a C6 to C60 aryl group, or a substituted or unsubstituted C2 to C60 heteroaryl group, and wherein R24 and R25 are the same as or different from each other and are each independently a substituted or unsubstituted C1 to C10 alkyl group, a substituted or unsubstituted C6 to C60 aryl group, or a substituted or unsubstituted C2 to C60 heteroaryl group, or R24 and R25 may be combined with each other to form a substituted or unsubstituted C6 to C60 aromatic hydrocarbon ring or a substituted or unsubstituted C2 to C60 heterocycle,
   R3 to R14 are the same as or different from each other and are each independently hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; or -NR21R22, wherein R21 and R22 are the same as or different from each other and are each independently a substituted or unsubstituted C1 to C10 alkyl group, a substituted or unsubstituted C6 to C60 aryl group, or a substituted or unsubstituted C2 to C60 heteroaryl group, with the proviso that when two or more of R14 are present, each R14 is the same as or different from each other,
   rings D, E, F, and G are each independently present or absent, with the proviso that at least one of these rings is present,
   o, p, q, and r are the same as or different from each other and are each independently an integer from 0 to 4, with the proviso that when each of o, p, q, and r is 2 or more, R11, R12, R13, and R14 is each independently selected.

In addition, the present invention provides an organic light-emitting device comprising:
a first electrode;
a second electrode provided to face the first electrode; and
one or more organic layers provided between the first electrode and the second electrode, wherein the organic layers comprise the heterocyclic compound represented by Formula 1.

In addition, the present invention provides a composition for an organic layer of an organic light-emitting device, comprising the heterocyclic compound represented by Formula 1 above.

### [Advantageous Effects]

The heterocyclic compound of the present invention may be usefully used as an material for an organic layer of an organic light-emitting device. In particular, this is used as a host material, thereby providing remarkable effects of lowering the driving voltage, improving the luminous efficiency, and improving the lifetime properties, of the organic light-emitting device.

In addition, the heterocyclic compound of the present invention provides excellent thermal stability.

The organic light-emitting device of the present invention comprises the heterocyclic compound, thereby providing excellent driving voltage, luminous efficiency, and lifetime properties.

### [Description of Drawings]

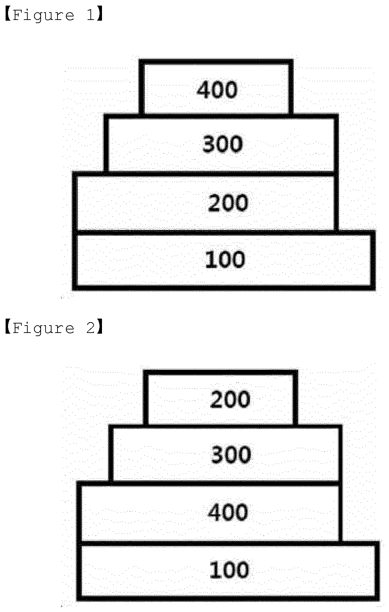

Figs. 1 to 3 are drawings schematically showing a stacked structure of an organic light-emitting device according to one embodiment of the present invention, respectively.

### [Best Mode]

Hereinafter, the present invention will be described in detail.

In the present invention, the term "substituted" means that a hydrogen atom bonded to a carbon atom of a compound is replaced with another substituent, and the position to be substituted is not limited as long as it is the position at which a hydrogen atom is substituted, that is, the position at which it may be substituted with a substituent. When substituted with two or more substituents, the two or more substituents may be the same as or different from each other.

In the present invention, the term "substituted or unsubstituted" means that it is unsubstituted or substituted with one or more substituents selected from the group consisting of C1 to C60 linear or branched alkyl; C2 to C60 linear or branched alkenyl; C2 to C60 linear or branched alkynyl; C3 to C60 monocyclic or polycyclic cycloalkyl; C2 to C60 monocyclic or polycyclic heterocycloalkyl; C6 to C60 monocyclic or polycyclic aryl; C2 to C60 monocyclic or polycyclic heteroaryl; -SiRR'R"; -P(=O)RR'; C1 to C20 alkylamine; C6 to C60 monocyclic or polycyclic arylamine; and C2 to C60 monocyclic or polycyclic heteroarylamine, or that it is unsubstituted or substituted with a substituent in which two or more substituents selected from the above-exemplified substituents are connected to each other.

In the present invention, the alkyl group includes a linear or branched chain having 1 to 60 carbon atoms, and may be further substituted with another substituent. The number of carbon atoms in the alkyl group may be 1 to 60, specifically 1 to 40, more specifically 1 to 20. Specific examples include, but are not limited to, a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like.

In the present invention, the alkenyl group includes a linear or branched chain having 2 to 60 carbon atoms, and may be further substituted with another substituent. The number of carbon atoms in the alkenyl group may be 2 to 60, specifically 2 to 40, more specifically 2 to 20. Specific examples include, but are not limited to, a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like.

In the present invention, the alkynyl group includes a linear or branched chain having 2 to 60 carbon atoms, and may be further substituted with another substituent. The number of carbon atoms in the alkynyl group may be 2 to 60, specifically 2 to 40, more specifically 2 to 20.

In the present invention, the cycloalkyl group includes a monocyclic or polycyclic ring having 3 to 60 carbon atoms, and may be further substituted with another substituent. In this case, the polycyclic ring refers to a group in which a cycloalkyl group is directly connected or condensed with another cyclic group. In this case, the another cyclic group may be a cycloalkyl group, but may be a different type of cyclic group, for example, a heterocycloalkyl group, an aryl group, a heteroaryl group, or the like. The number of carbon atoms in the cycloalkyl group may be 3 to 60, specifically 3 to 40, more specifically 5 to 20. Specifically, it includes, but is not limited to, a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, 4-tert-butylcyclohexyl group, a cycloheptyl group, a cyclooctyl group, and the like.

In the present invention, the heterocycloalkyl group includes O, S, Se, N, or Si as a heteroatom, includes a monocyclic or polycyclic ring having 2 to 60 carbon atoms, and may be further substituted with another substituent. In this case, the polycyclic ring refers to a group in which a heterocycloalkyl group is directly connected or condensed with another cyclic group. In this case, another cyclic group may be a heterocycloalkyl group, but may be a different type of cyclic group, for example, a cycloalkyl group, an aryl group, a heteroaryl group, or the like. The number of carbon atoms in the heterocycloalkyl group may be 2 to 60, specifically 2 to 40, more specifically 3 to 20.

In the present invention, the aryl group includes a monocyclic or polycyclic ring having 6 to 60 carbon atoms, and may be further substituted with other substituents. In this case, the polycyclic ring refers to a group in which an aryl group is directly connected or condensed with another cyclic group. In this case, another cyclic group may be an aryl group, but may be a different type of cyclic group, for example, a cycloalkyl group, a heterocycloalkyl group, a heteroaryl group, or the like. The aryl group includes a spiro group. The number of carbon atoms in the aryl group may be 6 to 60, specifically 6 to 40, more specifically 6 to 25. Specific examples of the aryl group may include, but are not limited to, a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, tetracenyl group, a pentacenyl group, a fluorenyl group, an indenyl group, an acenaphthylenyl group, a benzofluorenyl group, a spirobifluorenyl group, a 2,3-dihydro-1H-indenyl group, condensed cyclic groups thereof, and the like.

In the present invention, the fluorenyl group may be substituted, and adjacent substituents may be bonded to each other to form a ring.

When the fluorenyl group is substituted, it may be, but is not limited to, 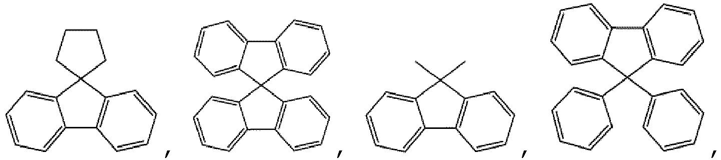 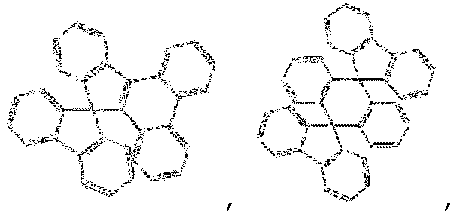 or the like.

In the present invention, the heteroaryl group includes S, O, Se, N, or Si as a heteroatom, includes a monocyclic or polycyclic ring having 2 to 60 carbon atoms, and may be further substituted with other substituents. In this case, the polycyclic group refers to a group in which a heteroaryl group is directly connected or condensed with another cyclic group. In this case, another cyclic group may be a heteroaryl group, but may be a different type of cyclic group, for example, a cycloalkyl group, a heterocycloalkyl group, an aryl group, or the like. The number of carbon atoms in the heteroaryl group may be 2 to 60, specifically 2 to 40, more specifically 3 to 25. Specific examples of the heteroaryl group may include, but are not limited to, a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a quinozolylyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophenyl group, a benzofuranyl group, a dibenzothiophenyl group, a dibenzofuranyl group, a carbazolyl group, a benzocarbazolyl group, a dibenzocarbazolyl group, a phenazinyl group, a dibenzosilol group, a spirobi(dibenzosilole) group, a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepinyl group, a 9,10-dihydroacridinyl group, a phenanthrazinyl group, a phenothiazinyl group, a phthalazinyl group, a naphthylidinyl group, a phenanthrolinyl group, a benzo[c][1,2,5]thiadiazolyl group, a 5,10-dihydrodibenzo[b,e][1,4]azasilinyl group, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group, and the like.

In the present invention, the amine group may be selected from the group consisting of a monoalkylamine group, a monoarylamine group, a monoheteroarylamine group, -NH₂, a dialkylamine group, a diarylamine group, a diheteroarylamine group, an alkylarylamine group, an alkylheteroarylamine group, and an arylheteroarylamine group; and the number of carbon atoms is not particularly limited, but is preferably 1 to 30. Specific examples of the amine group include, but are not limited to, a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group, and the like.

In the present invention, the arylene group refers to a group having two bonding positions on the aryl group, that is, a divalent group. The above description of the aryl group may be applied, except that each of them is a divalent group. In addition, the heteroarylene group refers to a group having two bonding positions on the heteroaryl group, that is, a divalent group. The above description of the heteroaryl group may be applied, except that each of them is a divalent group.

In the present invention, an "adjacent" group may refer to a substituent substituted on an atom directly connected to the atom on which that substituent is substituted, a substituent which is sterically closest to related substituent, or a substituent substituted on the atom on which that related substituent is substituted. For example, two substituents substituted at an ortho position on a benzene ring and two substituents substituted at the same carbon on an aliphatic ring may be interpreted as "adjacent" groups to each other.

In the present invention, "when a substituent is not indicated in the chemical formula or compound structure" means that a hydrogen atom is bonded to a carbon atom. However, since deuterium (²H) is an isotope of hydrogen, some hydrogen atoms may be deuterium.

In one embodiment of the present invention, "when a substituent is not indicated in the chemical formula or compound structure" may mean that hydrogen or deuterium is present at all positions that may be substituted with a substituent. That is, deuterium is an isotope of hydrogen, and thus, some hydrogen atoms may be deuterium that is an isotope, and in this case, the content of deuterium may be 0% to 100%.

In one embodiment of the present invention, in the case of "when a substituent is not indicated in the chemical formula or compound structure," hydrogen and deuterium may be used interchangeably in compounds unless deuterium is explicitly excluded, such as "the content of deuterium is 0%," "the content of hydrogen is 100%," and "all substituents are hydrogen".

In one embodiment of the present invention, deuterium is one of the isotopes of hydrogen and is an element having a deuteron consisting of one proton and one neutron as a nucleus, and may be expressed as hydrogen-2, and its element symbol may also be written as D or 2H.

In one embodiment of the present invention, an isotope refers to an atom having the same atomic number (Z) but a different mass number (A), and may also be interpreted as an element having the same number of protons but a different number of neutrons.

In one embodiment of the present invention, the meaning of the T% content of a specific substituent may be defined as an equation: T2/T1×100 = T%, wherein T1 is defined as the total number of substituents that the basic compound can have and T2 is defined as the number of specific substituents substituted among them.

That is, in one example, the 20% content of deuterium in the phenyl group represented by 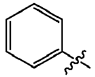 may mean that the total number of substituents that the phenyl group can have is 5 (T1 in the equation) and the number of deuterium among them is 1 (T2 in the equation). That is, the 20% content of deuterium in the phenyl group may be represented by the following structural formulas:

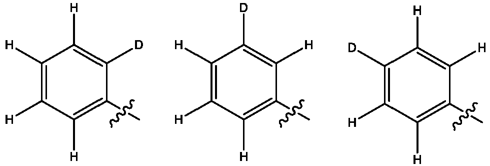

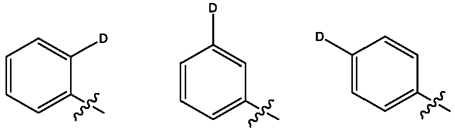

In addition, in one embodiment of the present invention, the case of "a phenyl group having a deuterium content of 0%" may mean a phenyl group that does not contain deuterium atoms, that is, a phenyl group having 5 hydrogen atoms.

In the present invention, the content of deuterium in the heterocyclic compound represented by Formula 1 may be 0 to 100%, more preferably 10 to 50%.

The present invention provides a heterocyclic compound represented by following Formula 1: 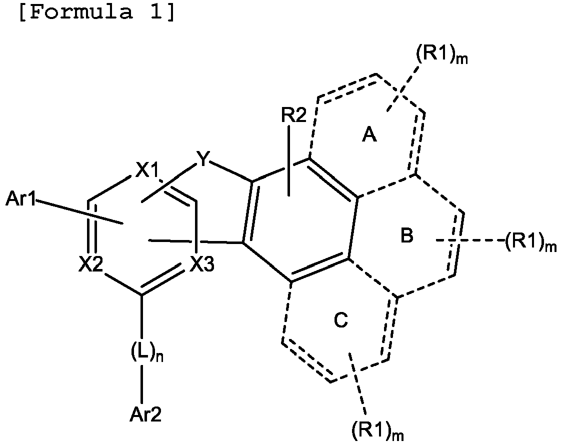 wherein,
X1, X2, and X3 are the same as or different from each other and are each independently N or C,
Y is NR20, O, or S, wherein R20 is hydrogen, deuterium, a substituted or unsubstituted C1 to C10 alkyl group, a substituted or unsubstituted C6 to C60 aryl group, or a substituted or unsubstituted C2 to C60 heteroaryl group,
Rings A, B, and C are each independently present or absent, with the proviso that at least one of these rings is present,
L is a direct bond, a substituted or unsubstituted C6 to C60 arylene group, or a substituted or unsubstituted C2 to C60 heteroarylene group,
Ar1 is a substituted or unsubstituted C6 to C60 aryl group or a substituted or unsubstituted C2 to C60 heteroaryl group,
R1 and R2 above are the same as or different from each other and are each independently hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; or -NR21R22, wherein R21 and R22 are the same as or different from each other and are each independently a substituted or unsubstituted C1 to C10 alkyl group, a substituted or unsubstituted C6 to C60 aryl group, or a substituted or unsubstituted C2 to C60 heteroaryl group, with the proviso that when two or more of R1 are present, each R1 is the same as or different from each other,
m is each independently an integer from 0 to 4, with the proviso that when m is 2 or more, each R1 is the same as or different from each other,
n is an integer from 0 to 3, with the proviso that when n is 2 or more, each L is the same as or different from each other, and
Ar2 is represented by following Formula 2 or 3: 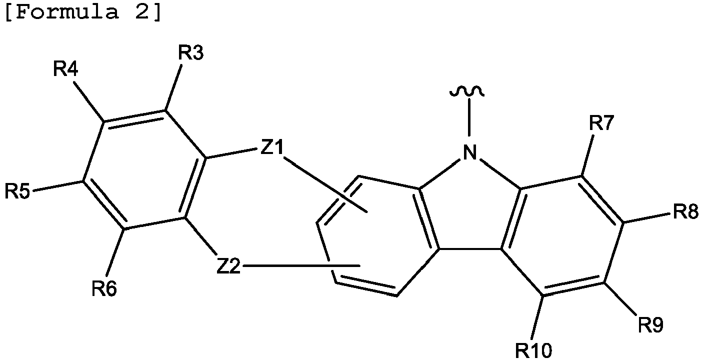 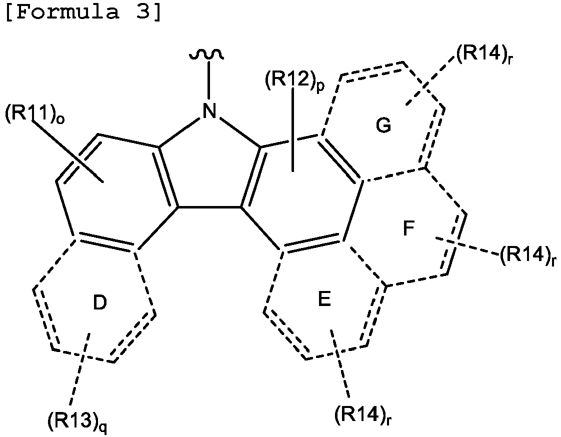 wherein,
   Z1 and Z2 are the same as or different from each other and are each independently a direct bond, NR23, O, S, or CR24R25, wherein R23 is hydrogen, deuterium, a substituted or unsubstituted C1 to C10 alkyl group, a substituted or unsubstituted a C6 to C60 aryl group, or a substituted or unsubstituted C2 to C60 heteroaryl group, and wherein R24 and R25 are the same as or different from each other and are each independently a substituted or unsubstituted C1 to C10 alkyl group, a substituted or unsubstituted C6 to C60 aryl group, or a substituted or unsubstituted C2 to C60 heteroaryl group, and R24 and R25 above may be combined with each other to form a substituted or unsubstituted C6 to C60 aromatic hydrocarbon ring or a substituted or unsubstituted C2 to C60 heterocycle,
   R3 to R14 are the same as or different from each other and are each independently hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; or -NR21R22, wherein R21 and R22 are the same as or different from each other and are each independently a substituted or unsubstituted C1 to C10 alkyl group, a substituted or unsubstituted C6 to C60 aryl group, or a substituted or unsubstituted C2 to C60 heteroaryl group, with the proviso that when two or more of R14 are present, each R14 is the same as or different from each other,
   Rings D, E, F, and G are each independently present or absent, with the proviso that at least one of these rings is present,
   o, p, q, and r are the same as or different from each other and are each independently an integer from 0 to 4, with the proviso that when each of o, p, q, and r is 2 or more, R11, R12, R13, and R14 may be each independently selected.

In one embodiment of the present invention, the heteroatom in the heteroatom-containing substituent may be one or more selected from O, S, Se, N, and Si.

In another embodiment of the present invention, the heteroatom in the heteroatom-containing substituent may be one or more selected from O, S, and N.

In one embodiment of the present invention, in X1, X2, and X3, any one of X1 and X2 may be N and the other may be C, and X3 may be N; in another embodiment, both X1 and X2 may be C and X3 may be N; in another embodiment, all of X1, X2, and X3 may be N; and in another embodiment, all of X1, X2, and X3 may be C. In this case, C may be actually "CH," and when a heterocycle is bonded to the site, it may be "C".

In one embodiment of the present invention, in rings A, B, and C, any one of these rings may be present and the other rings may not be present; in another embodiment, any two of these rings may be present and the other ring may not be present; and in another embodiment, all of these rings may be present. Rings A, B, and C may be individually present, or may exist in the form of an aromatic ring in all cases in which they exist in combination.

In one embodiment of the present invention, any one of Z1 and Z2 may be a direct bond, and the other may be NR23, O, S, or CR24R25. In this case, R23 and CR24R25 are as defined above.

In one embodiment of the present invention, in rings D, E, F and G, ring D above may be present and rings E, F and G may not be present; in another embodiment, rings D and E may be present and rings F and G may not be present; in another embodiment, any one of rings E, F and G may be present and the other rings may not be present; in another embodiment, any two of rings E, F and G may be present and the other ring may not be present; in another embodiment, all of rings E, F and G may be present and the other ring may not be present; in another embodiment, any one of rings E, F and G and ring D may be present and the other rings may not be present; in another embodiment, any two of rings E, F and G and ring D may be present and the other ring may not be present; and in another embodiment, all of rings D, E, F and G may be present. Rings E, F and G may be individually present, or may exist in the form of an aromatic ring in all cases in which they exist in combination.

In one embodiment of the present invention, Ar1 may be a substituted or unsubstituted C6 to C30 aryl group or a substituted or unsubstituted C2 to C30 heteroaryl group.

In another embodiment of the present invention, Ar1 may be a substituted or unsubstituted C6 to C20 aryl group or a substituted or unsubstituted C2 to C20 heteroaryl group.

In another embodiment of the present invention, Ar1 may be substituted or unsubstituted phenyl, naphthalenyl, biphenyl, anthracenyl, phenanthrenyl,

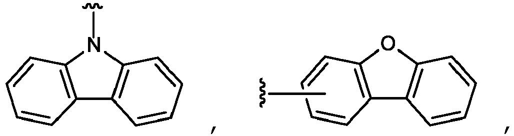

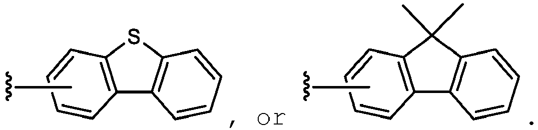

In one embodiment of the present invention, L above may be a substituted or unsubstituted C6 to C30 aryl group or a substituted or unsubstituted C2 to C30 heteroaryl group.

In another embodiment of the present invention, L may be a substituted or unsubstituted C6 to C20 aryl group or a substituted or unsubstituted C2 to C20 heteroaryl group.

In another embodiment of the present invention, L may be substituted or unsubstituted phenylene, naphthalene, biphenylene, anthracenylene, pyridine, or phenanthrenylene.

In one embodiment of the present invention, R1 to R14 may be the same as or different from each other and may be each independently hydrogen, deuterium, halogen, a cyano group, a substituted or unsubstituted C1 to C30 alkyl group, a substituted or unsubstituted C1 to C30 alkoxy group, a substituted or unsubstituted C6 to C60 aryl group, a substituted or unsubstituted C2 to C60 heteroaryl group, or -NR21R22, wherein R21 and R22 may be the same as or different from each other and may be each independently a substituted or unsubstituted C1 to C10 alkyl group, a substituted or unsubstituted C6 to C60 aryl group, or a substituted or unsubstituted C2 to C60 heteroaryl group.

In another embodiment of the present invention, R1 to R14 above may be the same as or different from each other and may be each independently hydrogen, deuterium, halogen, a cyano group, a substituted or unsubstituted C1 to C20 alkyl group, a substituted or unsubstituted C6 to C30 aryl group, a substituted or unsubstituted C2 to C30 heteroaryl group, or -NR21R22, wherein R21 and R22 may be the same as or different from each other and may be each independently a substituted or unsubstituted C1 to C10 alkyl group, a substituted or unsubstituted C6 to C30 aryl group, or a substituted or unsubstituted C2 to C30 heteroaryl group.

In another embodiment of the present invention, R1 to R14 above may be the same as or different from each other and may be each independently hydrogen, deuterium, a substituted or unsubstituted C6 to C20 aryl group, a substituted or unsubstituted C2 to C20 heteroaryl group, or -NR21R22, wherein R21 and R22 may be the same as or different from each other and may be each independently a substituted or unsubstituted C1 to C10 alkyl group, a substituted or unsubstituted C6 to C20 aryl group, or a substituted or unsubstituted C2 to C20 heteroaryl group.

In another embodiment of the present invention, R1 to R14 may be the same as or different from each other and may be each independently hydrogen, deuterium, substituted or unsubstituted phenyl, naphthalenyl, biphenyl, anthracenyl, phenanthrenyl, 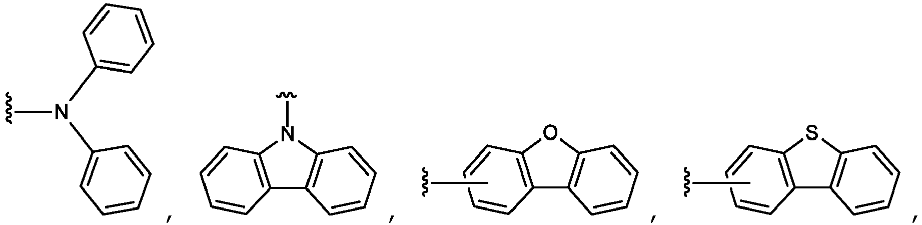 or

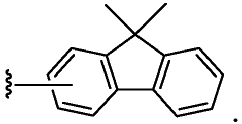

In one embodiment of the present invention, R20 and R23 above may be the same as or different from each other and may be each independently hydrogen, deuterium, a substituted or unsubstituted C1 to C10 alkyl group, a substituted or unsubstituted C6 to C30 aryl group, or a substituted or unsubstituted C2 to C30 heteroaryl group.

In another embodiment of the present invention, R20 and R23 may be the same as or different from each other and may be each independently hydrogen, deuterium, a substituted or unsubstituted C1 to C5 alkyl group, a substituted or unsubstituted C6 to C20 aryl group, or a substituted or unsubstituted C2 to C20 heteroaryl group.

In one embodiment of the present invention, R20 and R23 may be the same as or different from each other and may be each independently selected from the group consisting of hydrogen, deuterium, a substituted or unsubstituted C1 to C5 alkyl group, substituted or unsubstituted phenyl, naphthalenyl, pyridinyl, anthracenyl, carbazole, biphenyl, dibenzothiophene, dibenzofuran, and phenanthrenyl.

In one embodiment of the present invention, R21, R22, R24, and R25 above may be the same as or different from each other and may be each independently hydrogen, deuterium, a substituted or unsubstituted C1 to C10 alkyl group, a substituted or unsubstituted C6 to C30 aryl group, or a substituted or unsubstituted C2 to C30 heteroaryl group; and R24 and R25 may be combined with each other to form a substituted or unsubstituted C6 to C30 aromatic hydrocarbon ring or a substituted or unsubstituted C2 to C30 heterocycle.

In another embodiment of the present invention, R20 to R25 above may be the same as or different from each other and may be each independently a substituted or unsubstituted C1 to C5 alkyl group, a substituted or unsubstituted C6 to C20 aryl group, or a substituted or unsubstituted C2 to C20 heteroaryl group; and R24 and R25 may be combined with each other to form a substituted or unsubstituted C6 to C20 aromatic hydrocarbon ring or a substituted or unsubstituted C2 to C20 heterocycle.

In another embodiment of the present invention, R20 to R25 may be the same as or different from each other and may be each independently selected from the group consisting of a substituted or unsubstituted C1 to C5 alkyl group, substituted or unsubstituted phenyl, naphthalenyl, pyridinyl, anthracenyl, carbazole, biphenyl, dibenzothiophene, dibenzofuran, and phenanthrenyl, wherein R24 and R25 may be combined with each other to form a substituted or unsubstituted fluorenyl group.

In one embodiment of the present invention, the 'substitution' in the definition of R1 to R14; R20 to R25; Arl; and L may be each independently made with one or more substituents selected from the group consisting of C1 to C10 linear or branched alkyl; C2 to C10 linear or branched alkenyl; C2 to C10 linear or branched alkynyl; C3 to C15 cycloalkyl; C2 to C20 heterocycloalkyl; C6 to C30 aryl; C2 to C30 heteroaryl; C1 to C10 alkylamine; C6 to C30 arylamine; and C2 to C30 heteroarylamine.

In another embodiment of the present invention, the 'substitution' in the definition of R1 to R14; R20 to R25; Arl; and L may be each independently made with one or more substituents selected from the group consisting of C1 to C10 linear or branched alkyl; C2 to C10 linear or branched alkenyl; C2 to C10 linear or branched alkynyl; C6 to C30 aryl; C2 to C30 heteroaryl; C6 to C30 arylamine; and C2 to C30 heteroarylamine.

In another embodiment of the present invention, the 'substitution' in the definition of R1 to R14; R20 to R25; Arl; and L may be each independently made with one or more substituents selected from the group consisting of C6 to C30 aryl; C2 to C30 heteroaryl; C6 to C30 arylamine; and C2 to C30 heteroarylamine.

In another embodiment of the present invention, the 'substitution' in the definition of R1 to R14; R20 to R25; Arl; and L may be each independently made with one or more substituents selected from the group consisting of phenyl, naphthalenyl, pyridinyl, anthracenyl, carbazole, biphenyl, dibenzothiophene, dibenzofuran, and phenanthrenyl.

In one embodiment of the present invention, Formula 3 above may be represented by any one of following Formulas 3-1 to 3-3: 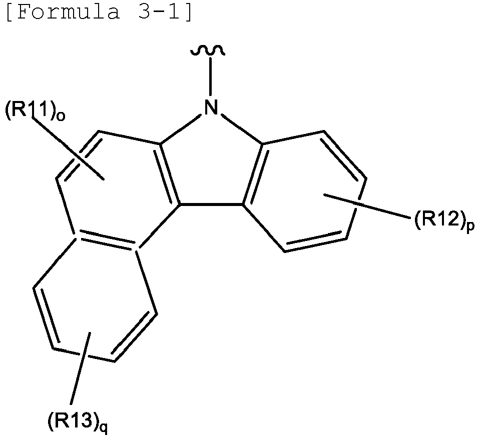 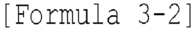 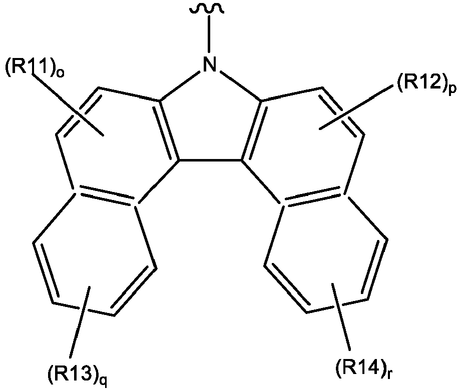 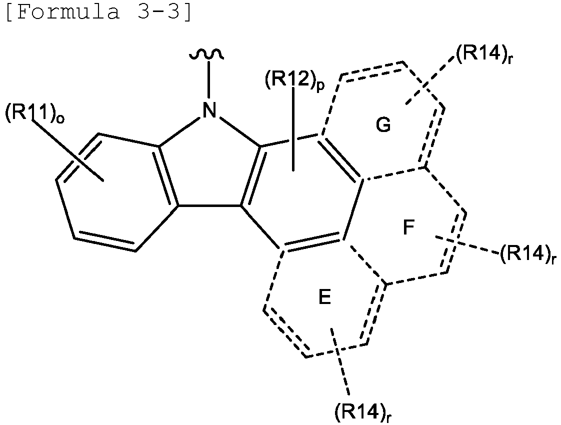 wherein,
each substituent is defined as described above,
rings E, F, and G are each independently present or absent, with the proviso that at least one of these rings is present.

In one embodiment of the present invention, the heterocyclic compound represented by Formula 1 may be a compound represented by any one of the following compounds:

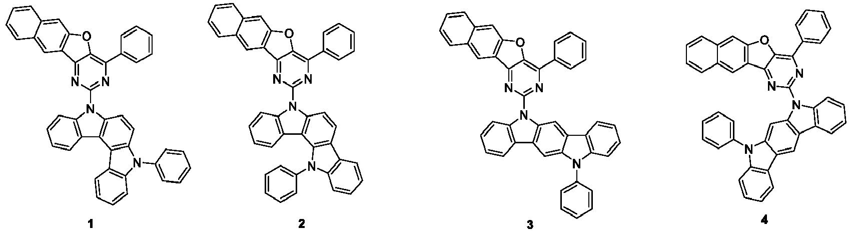

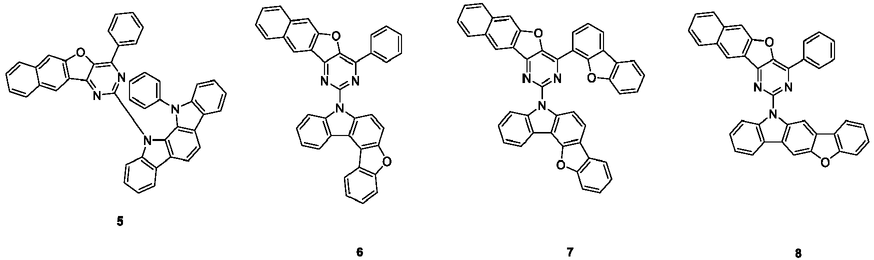

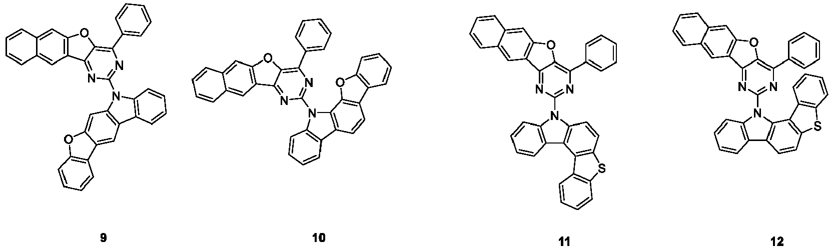

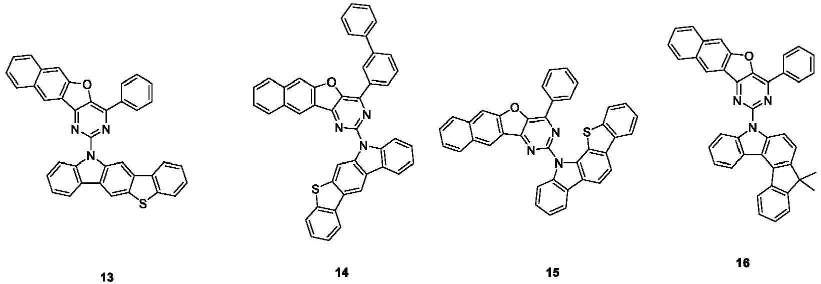

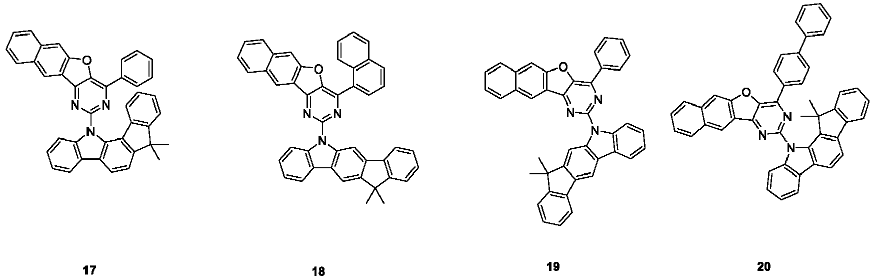

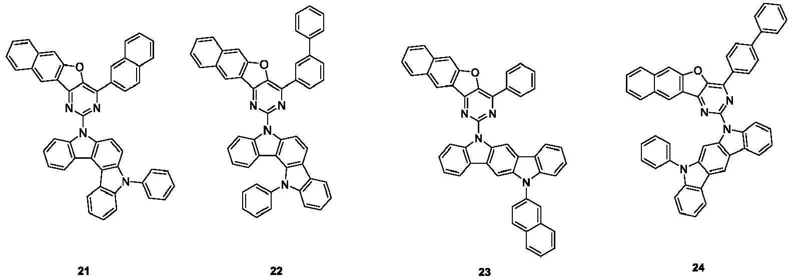

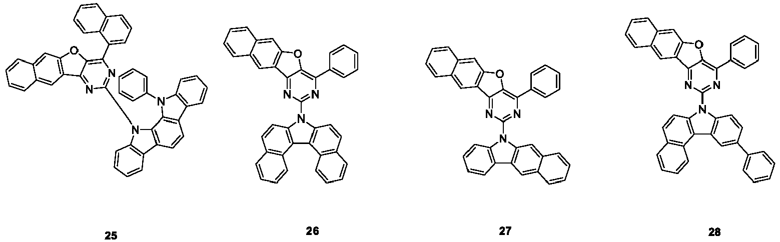

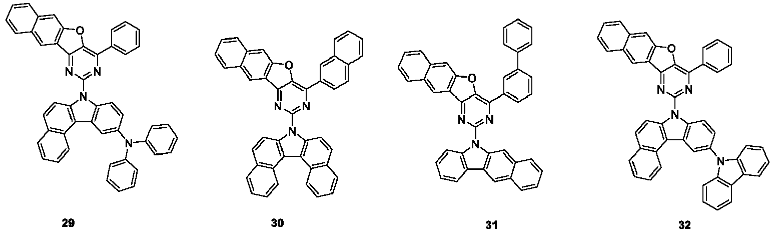

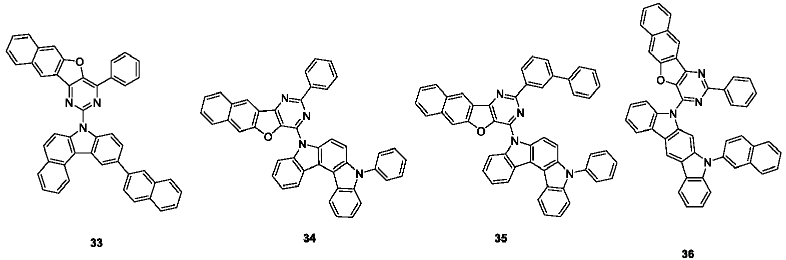

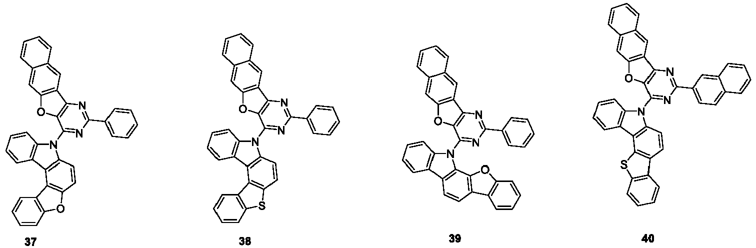

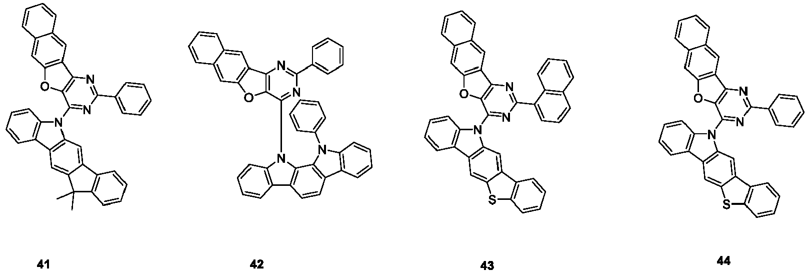

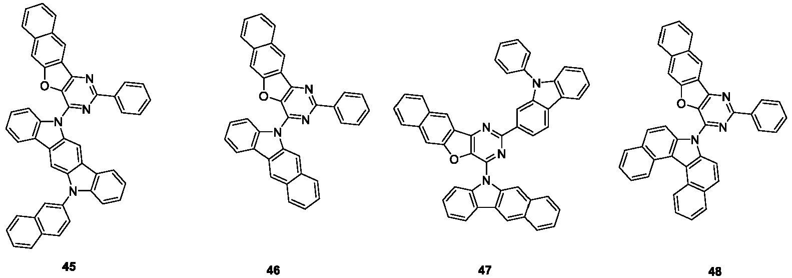

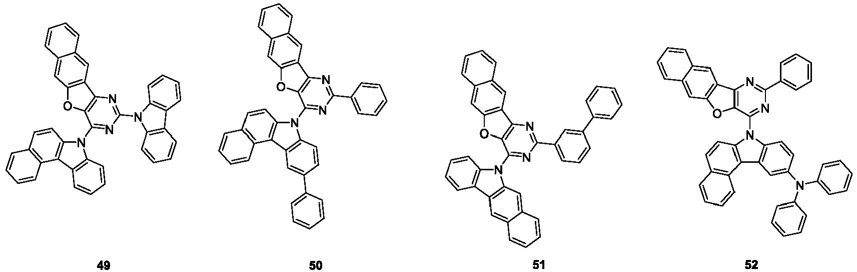

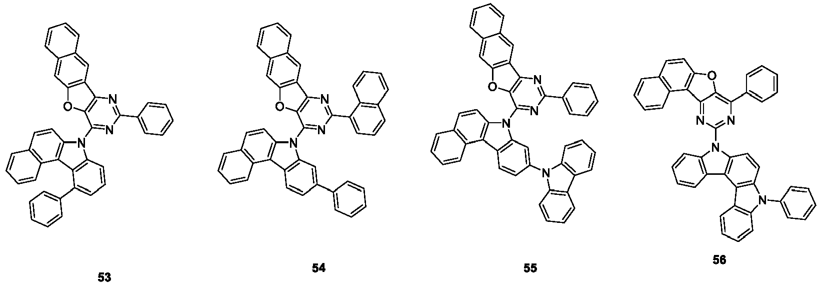

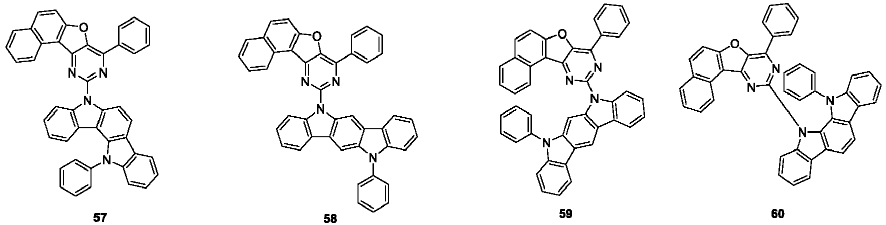

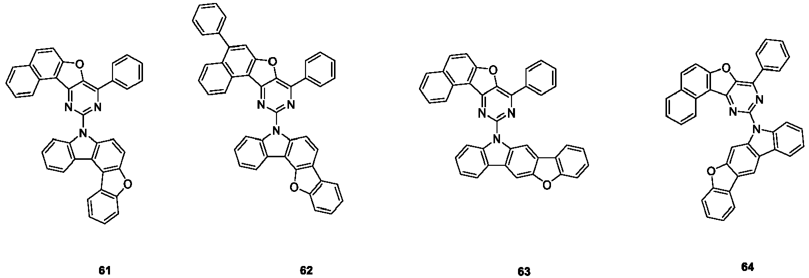

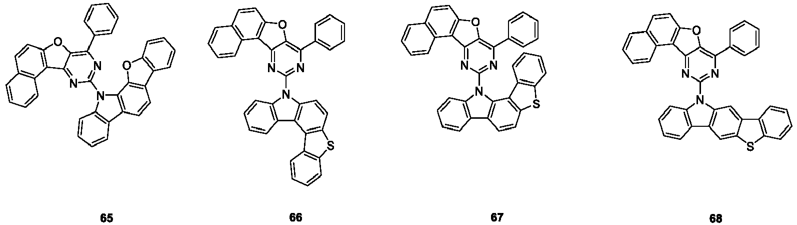

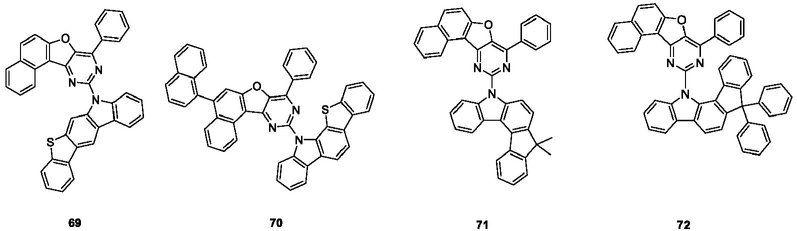

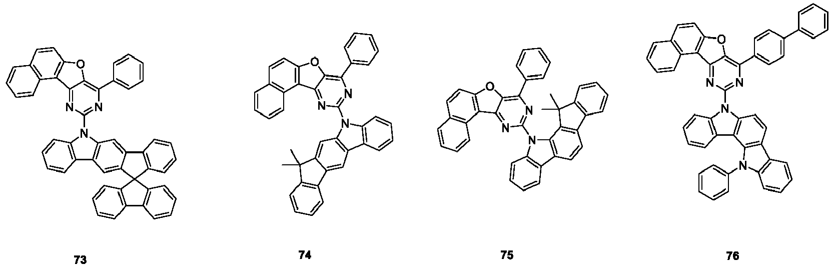

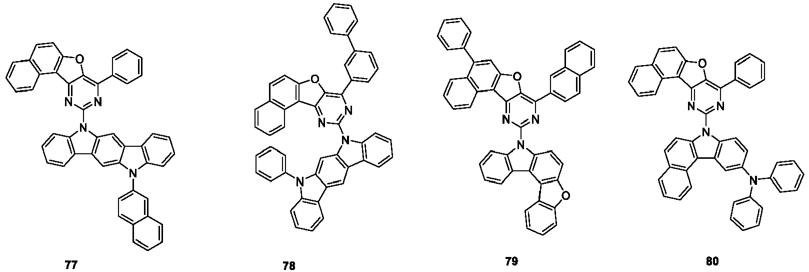

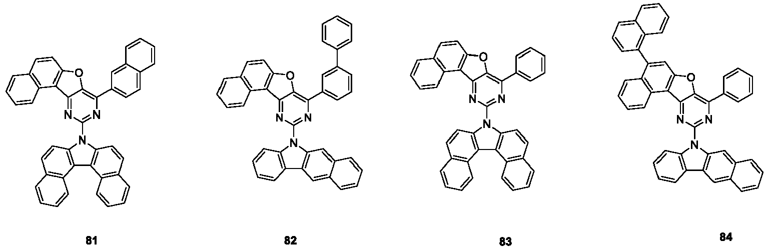

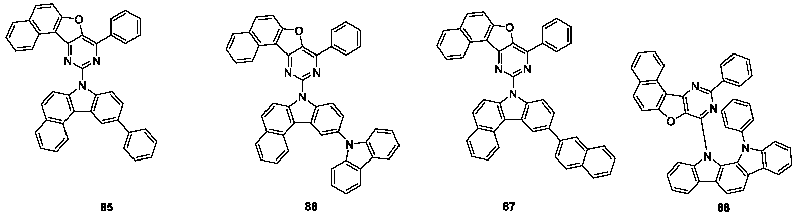

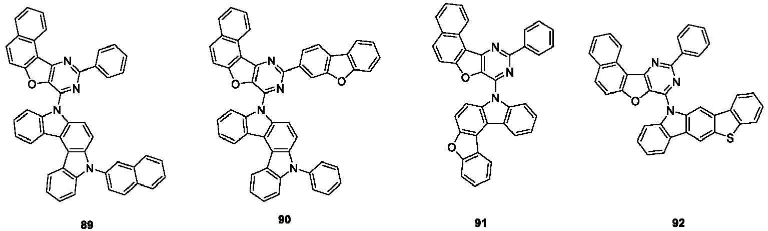

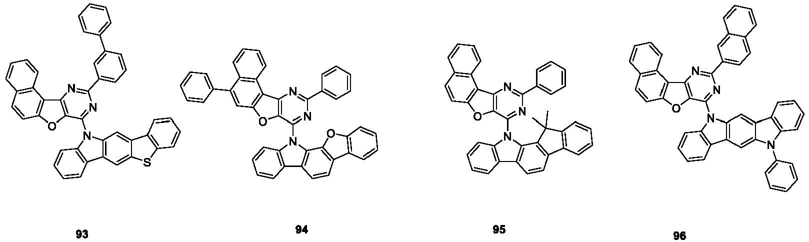

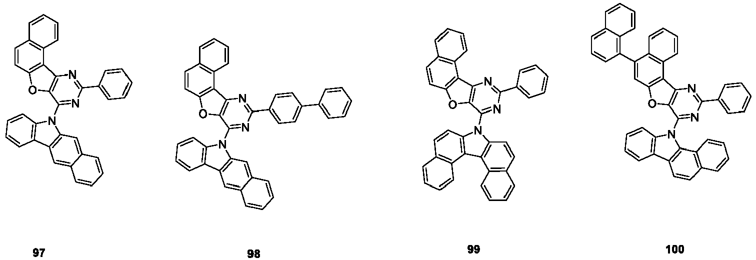

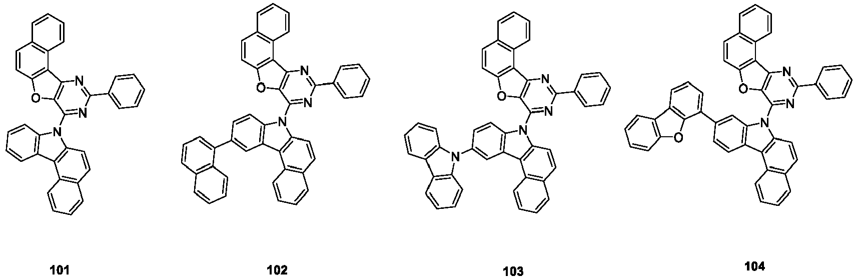

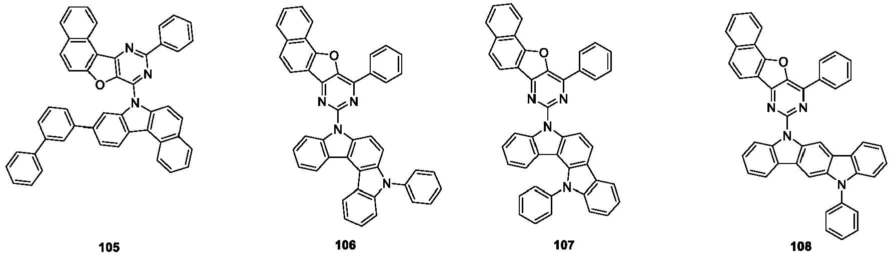

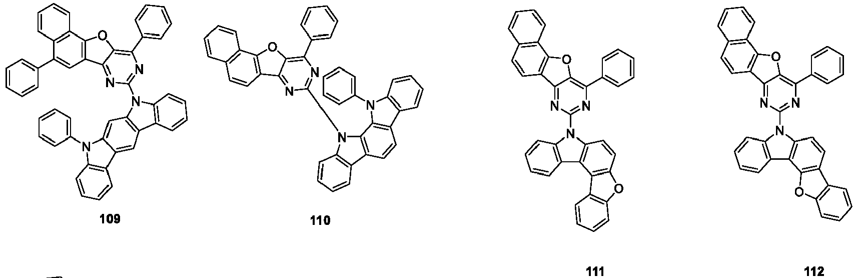

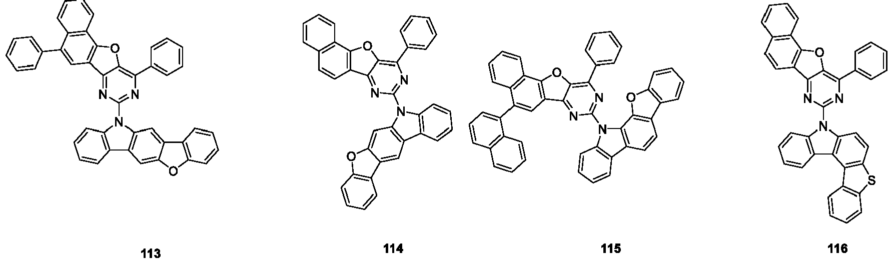

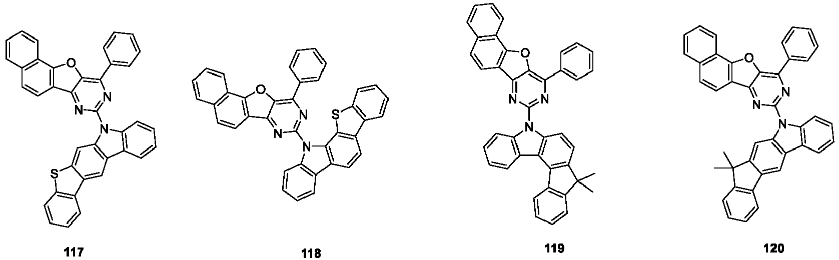

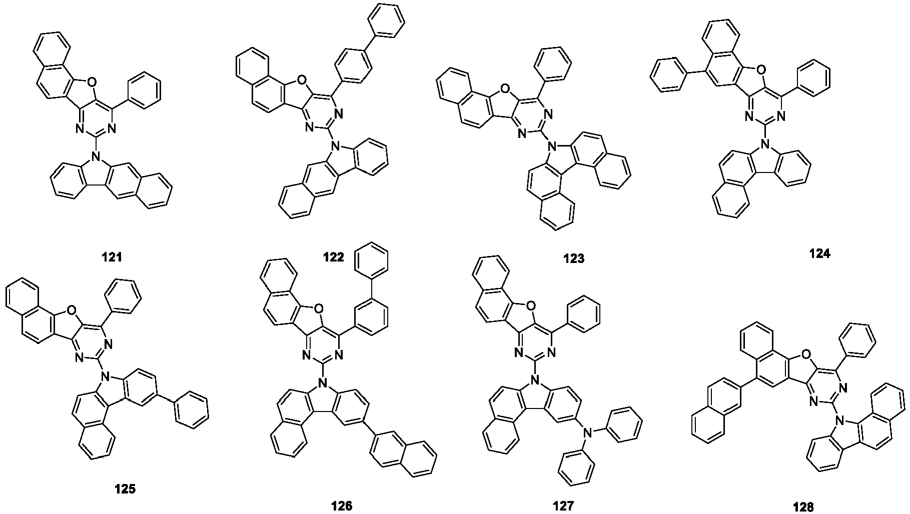

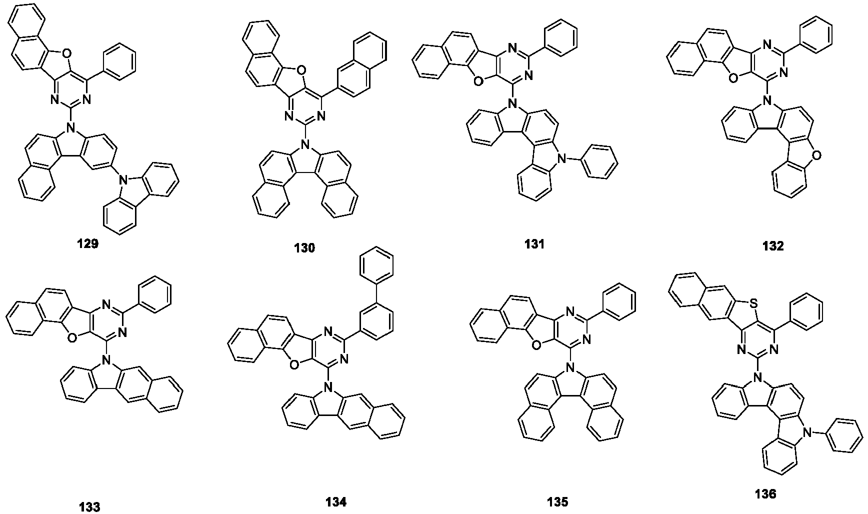

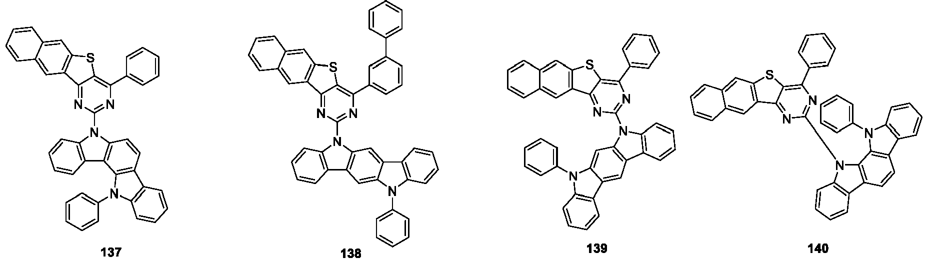

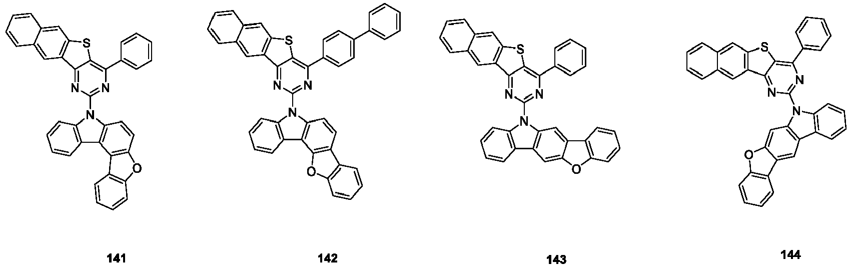

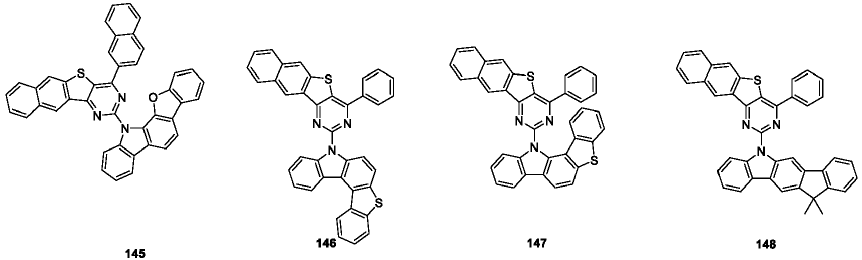

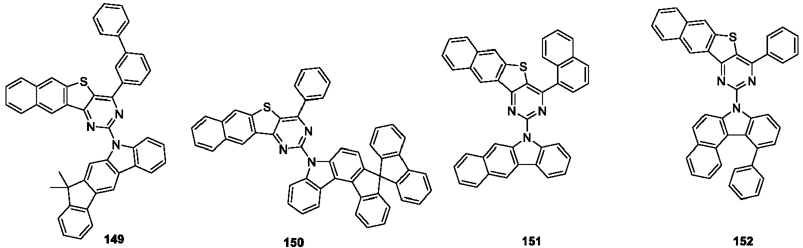

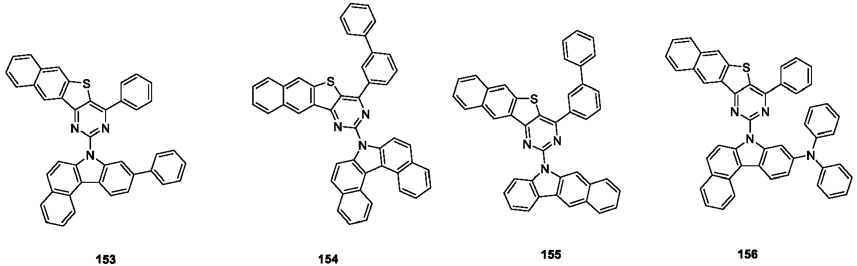

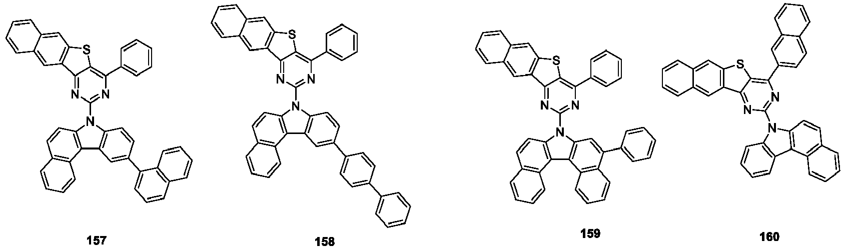

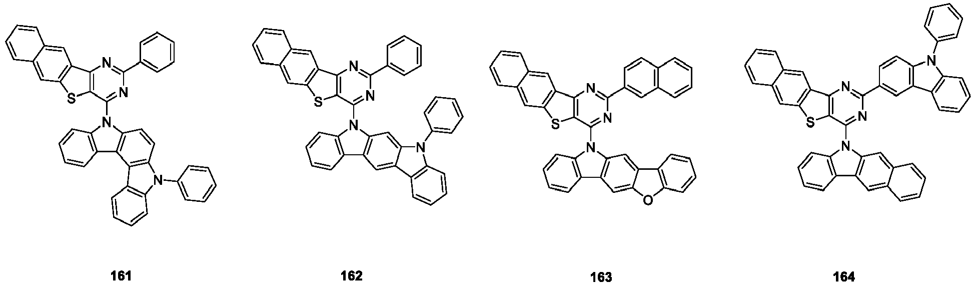

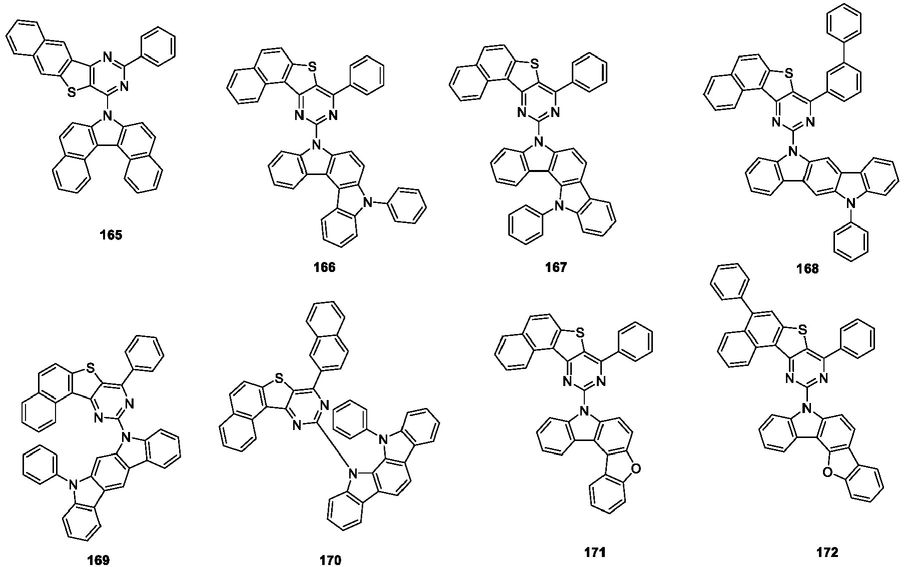

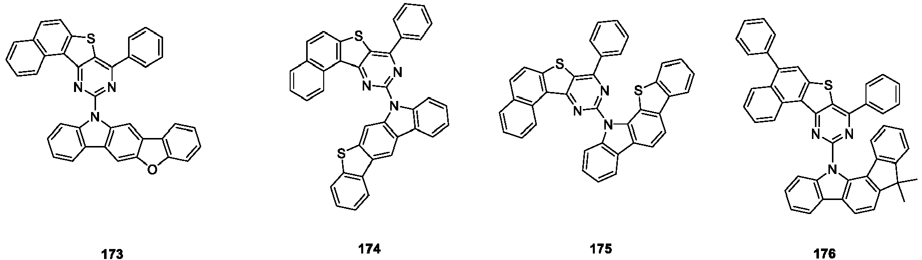

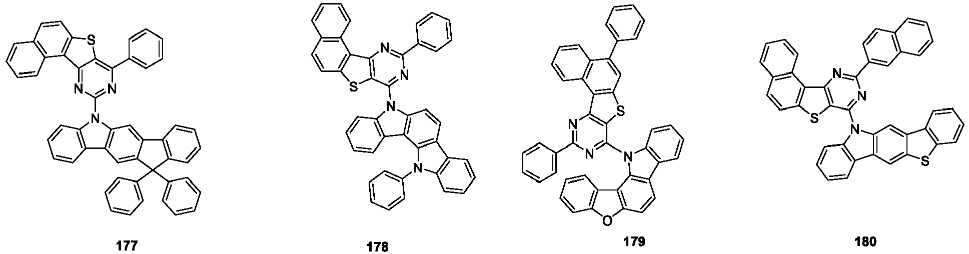

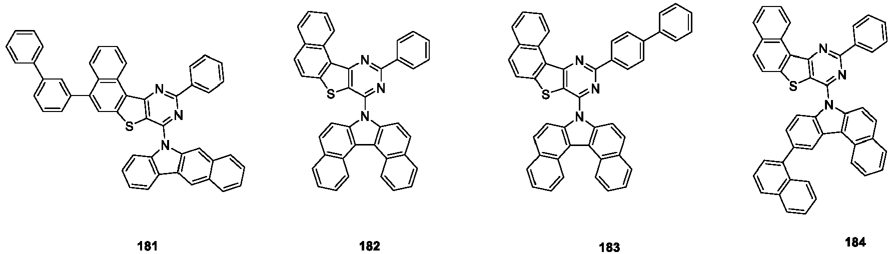

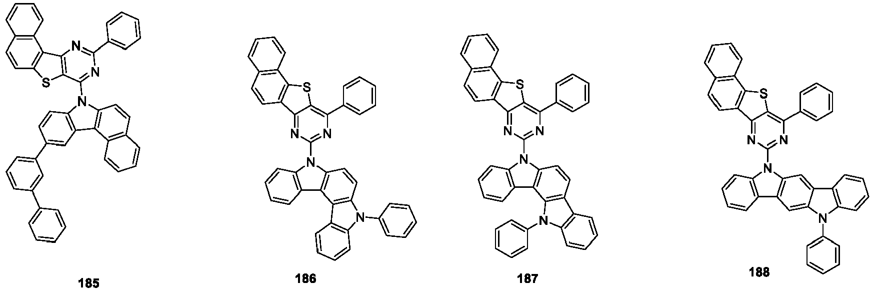

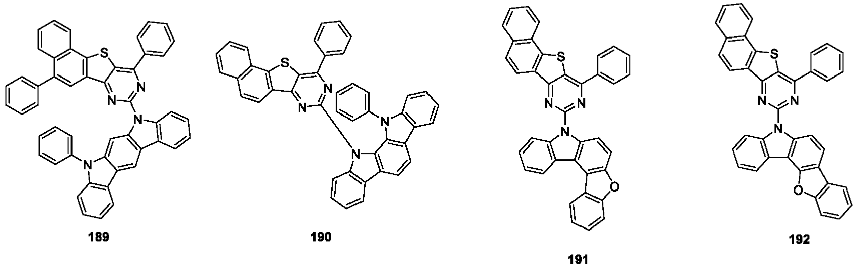

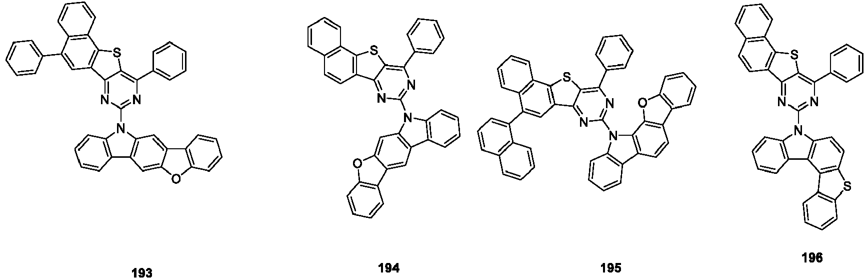

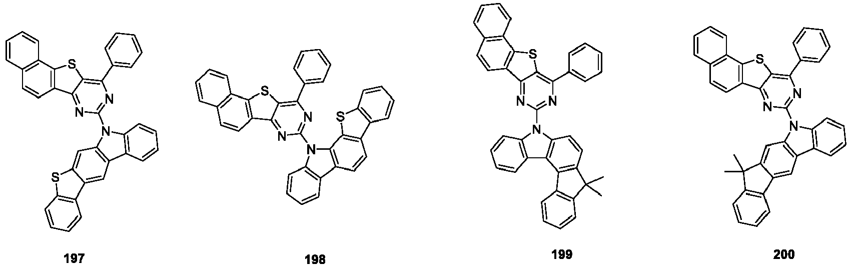

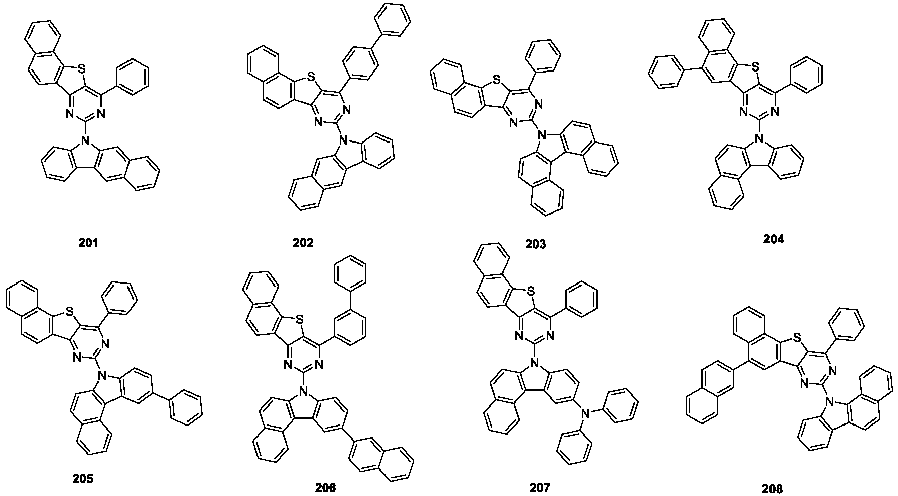

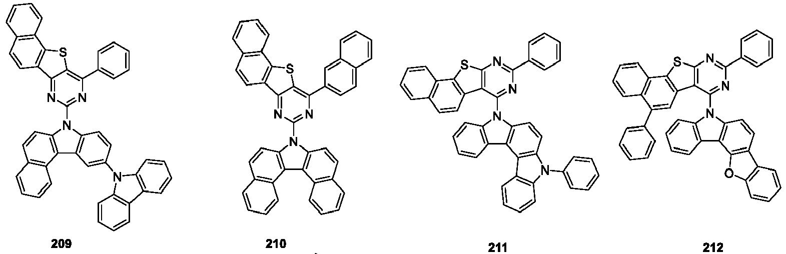

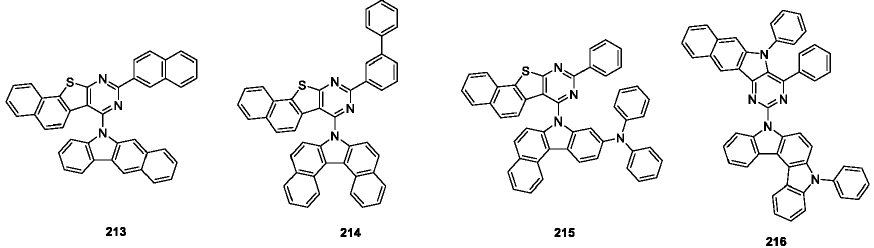

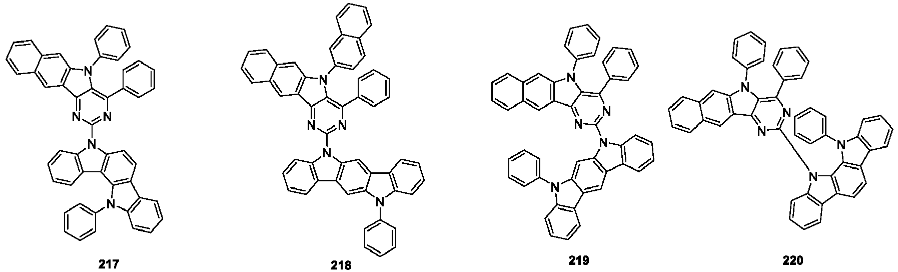

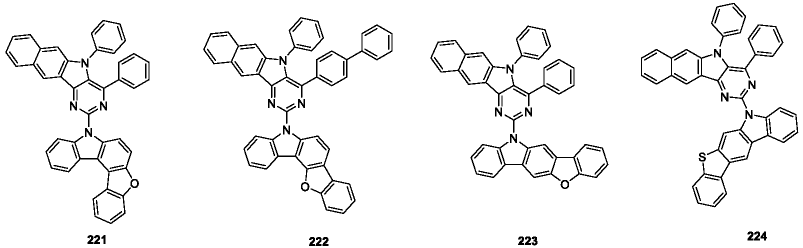

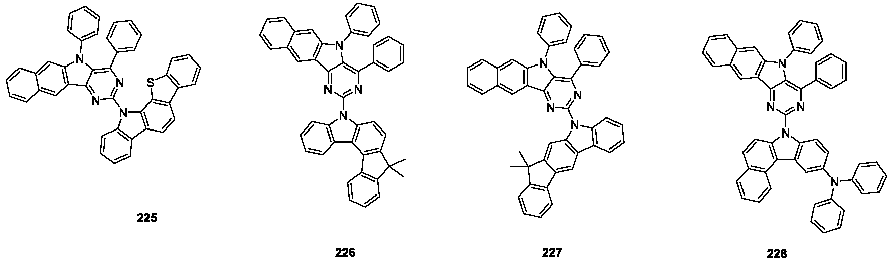

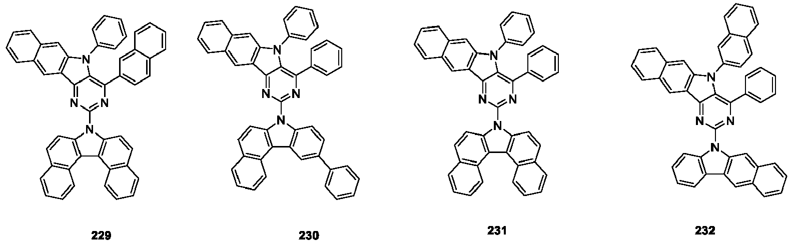

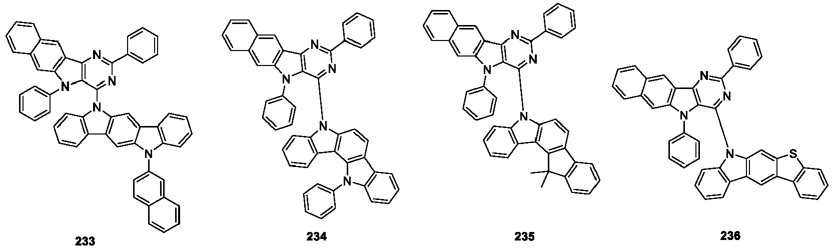

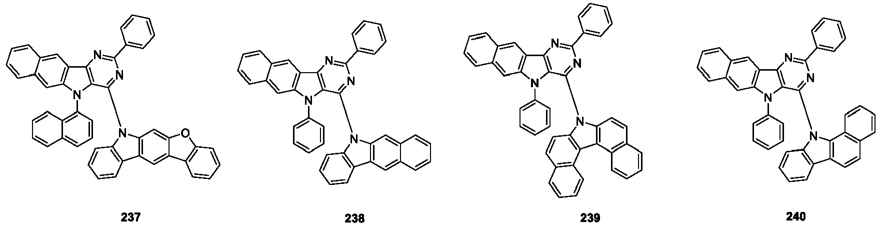

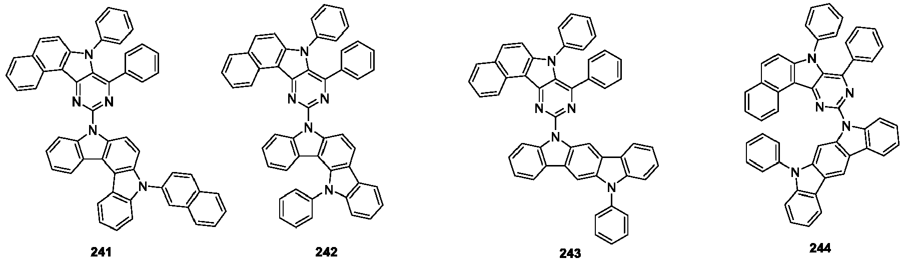

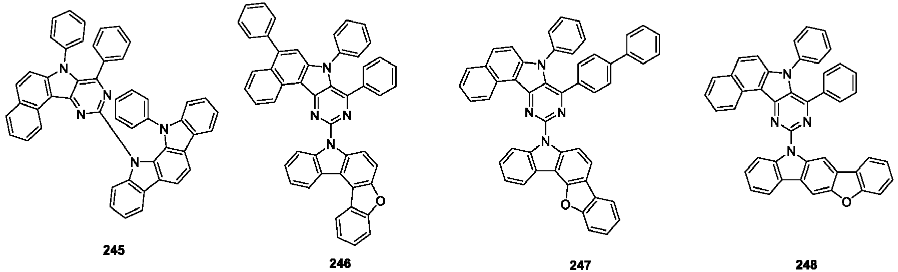

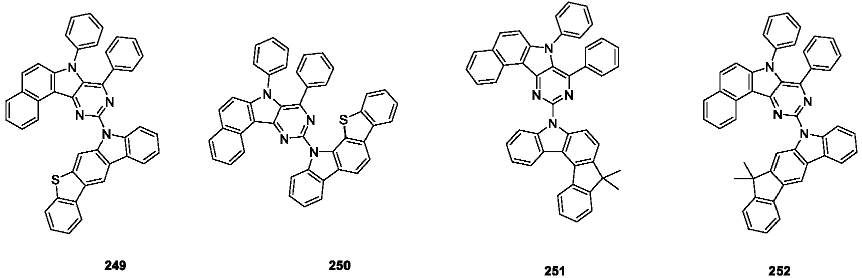

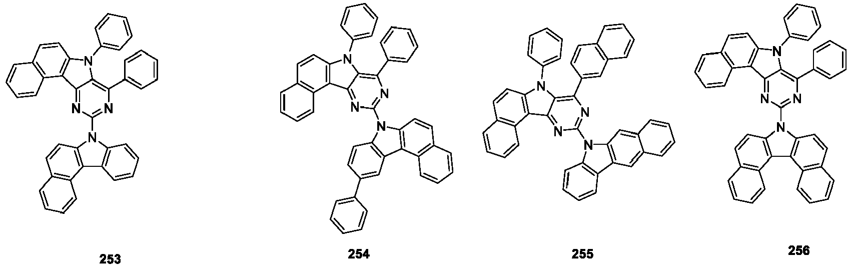

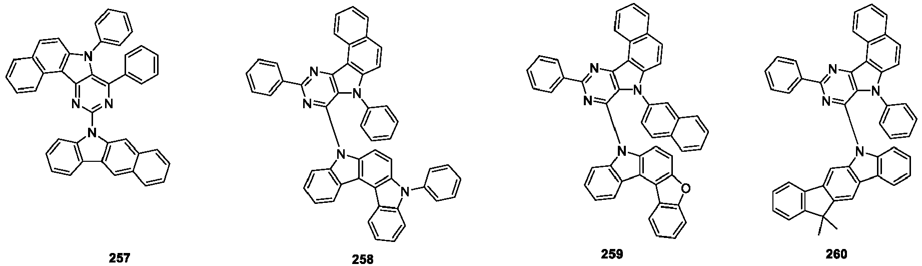

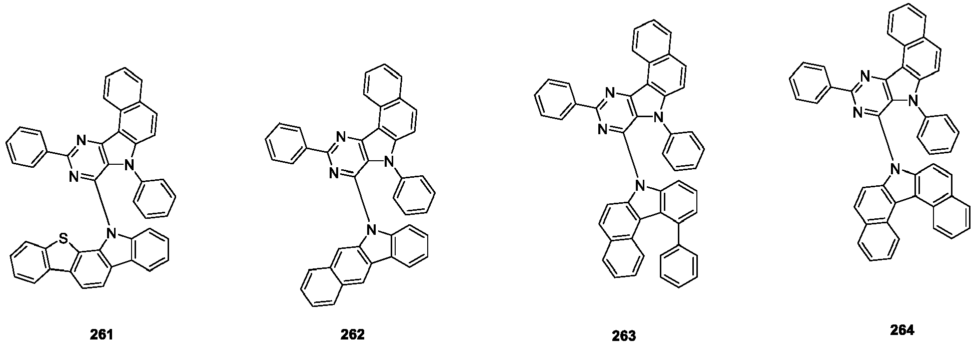

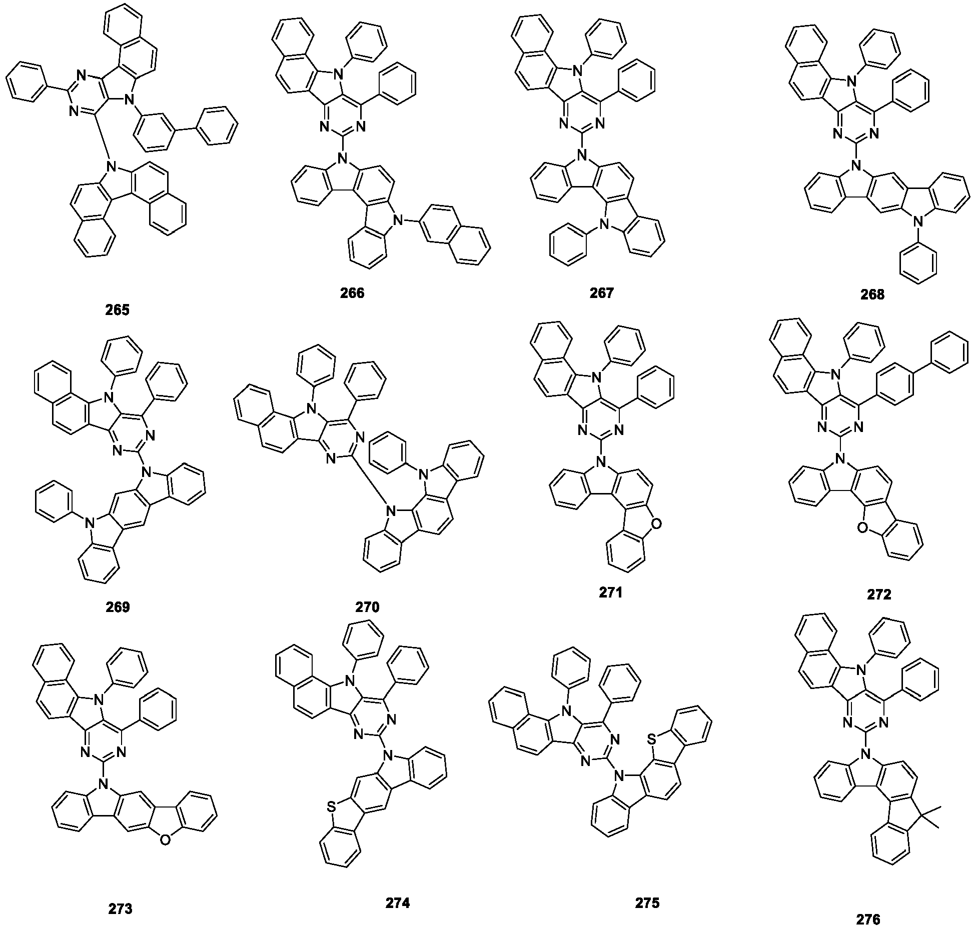

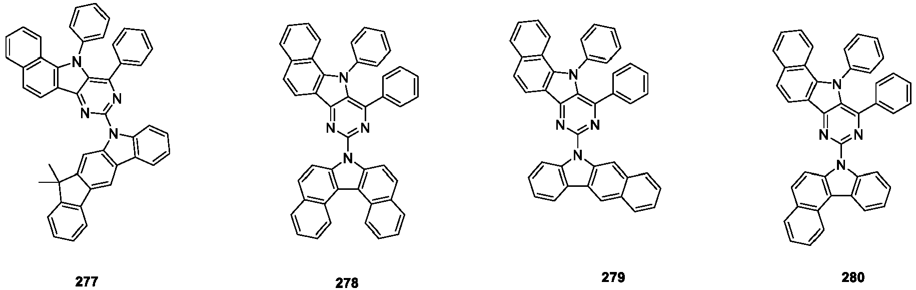

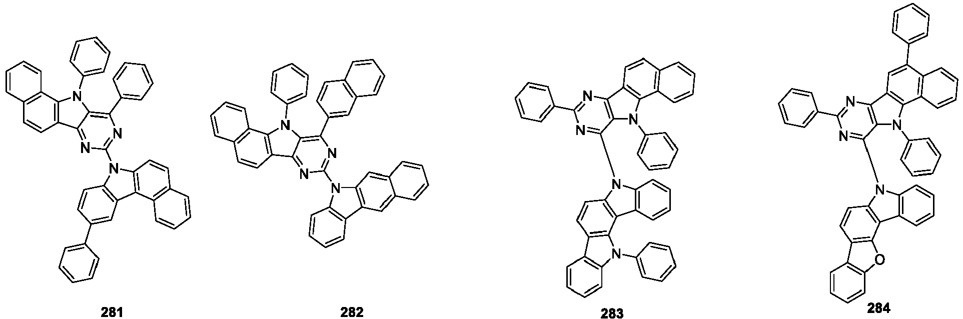

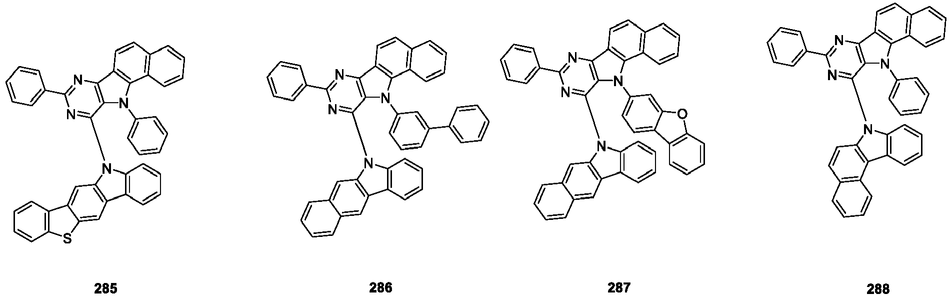

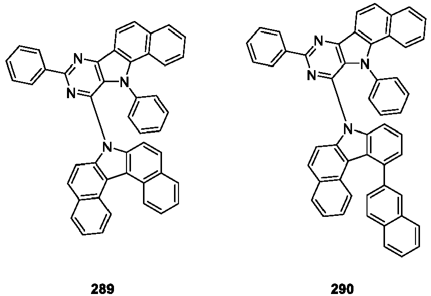

By introducing various substituents into the corresponding structure, the compound of Formula 1 above may be synthesized as a compound having intrinsic properties of the introduced substituent. For example, by introducing into the core structure a substituent mainly used for a material for a hole injection layer, a material for a hole transport layer, a material for an electron-blocking layer, a material for a light-emitting layer, a material for a hole-blocking layer, a material for an electron transport layer, a material for an electron injection layer, and a material for an electron-generating layer, which are used in manufacturing the organic light-emitting device, it is possible to synthesize a material satisfying the conditions required for each organic layer.

In addition, by introducing various substituents into the structure of Formula 1 above, it is possible to finely control the energy band gap, while improving the properties at the interface between organic materials and diversifying the use of the materials.

The heterocyclic compound may be used as one or more use selected from a material for a hole injection layer, a material for a hole transport layer, a material for an electron-blocking layer, a material for a light-emitting layer, a material for a hole-blocking layer, a material for an electron transport layer, and a material for an electron injection layer, which are used in the organic layer of the organic light-emitting device, in particular, may be preferably used as a material for a light-emitting layer, and specifically may be preferably used as a host material.

In addition, the present invention relates to organic light-emitting device, including a first electrode; a second electrode provided to face the first electrode; and one or more organic layers provided between the first electrode and the second electrode, wherein the organic layers comprise the heterocyclic compound represented by Formula 1.

In one embodiment of the present invention, the first electrode may be an anode, and the second electrode may be a cathode.

In another embodiment, the first electrode may be a cathode, and the second electrode may be an anode.

The organic light-emitting device according to one embodiment of the present invention may further comprise one or two more layers selected from the group consisting of a hole injection layer, a hole transport layer, an electron-blocking layer, a light-emitting layer, a hole-blocking layer, an electron transport layer, and an electron injection layer, and may have, but is not limited to, a stacked structure in the order of anode/hole injection layer/hole transport layer/electron-blocking layer/light-emitting layer/hole-blocking layer/electron transport layer/electron injection layer/cathode.

In one embodiment of the present invention, the organic light-emitting device may be a red organic light-emitting device, and the heterocyclic compound represented by Formula 1 may be used as a material of the red organic light-emitting device.

In one embodiment of the present invention, the organic light-emitting device may be a blue organic light-emitting device, and the heterocyclic compound represented by Formula 1 may be used as a material of the blue organic light-emitting device.

In one embodiment of the present invention, the organic light-emitting device may be a green organic light-emitting device, and the heterocyclic compound represented by Formula 1 may be used as a material of the green organic light-emitting device.

Specific details of the heterocyclic compound represented by Formula 1 are as described above.

The organic light-emitting device of the present invention may be manufactured by conventional methods and materials for manufacturing an organic light-emitting device, except that one or more organic layers are formed using the aforementioned heterocyclic compound.

In one embodiment of the present invention, in the red organic light-emitting device, the blue organic light-emitting device, and the green organic light-emitting device, the heterocyclic compound represented by Formula 1 may be used as one or more uses selected from a material for a hole injection layer, a material for a hole transport layer, a material for an electron-blocking layer, a material for a light-emitting layer, a material for a hole-blocking layer, a material for an electron transport layer, and a material for an electron injection layer, in particular, may be used as a material for a light-emitting layer, and specifically may be used as a red host material.

Figs. 1 to 3 accompanied below illustrate the stacking order of the electrodes and the organic layers of the organic light-emitting device according to one embodiment of the present invention. However, it is not intended that the scope of the present invention be limited by these drawings, and the structure of the organic light-emitting device known in the art may also be applied to the present invention.

According to Fig. 1, there is shown an organic light-emitting device in which an anode 200, an organic layer 300, and a cathode 400 are sequentially stacked on a substrate 100. However, it is not limited to such a structure, and an organic light-emitting device in which a cathode, an organic layer, and an anode are sequentially stacked on a substrate may be implemented, as shown in Fig. 2.

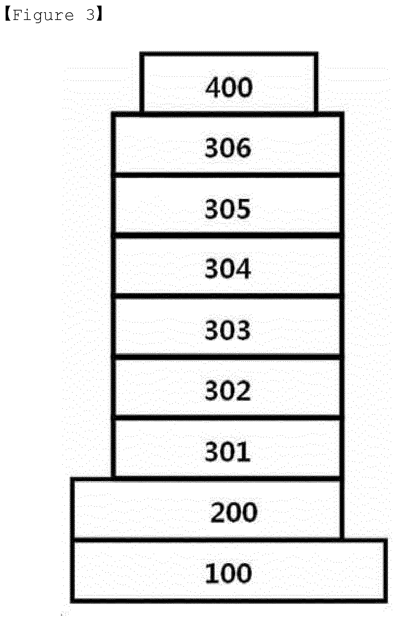

Fig. 3 illustrates a case where the organic layer is composed of multiple layers. The organic light-emitting device according to Fig. 3 comprises a hole injection layer 301, a hole transport layer 302, a light-emitting layer 303, a hole-blocking layer 304, an electron transport layer 305, and an electron injection layer 306. However, the scope of the present invention is not limited by such stacked structures, and the remaining layers except for the light-emitting layer may be omitted, if necessary, and other necessary functional layers such as an electron-blocking layer may be further added.

The heterocyclic compound may be formed into an organic layer by a solution coating method as well as a vacuum deposition method when manufacturing an organic light-emitting device. In this case, the solution coating method refers to, but is not limited to, spin coating, dip coating, inkjet printing, screen printing, spraying, roll coating, and the like.

The organic layer of the organic light-emitting device of the present invention may have a single-layer structure, and may also have a multi-layer structure in which two or more organic layers are stacked. For example, the organic light-emitting device of the present invention may have a structure comprising one or more selected from the group consisting of a hole injection layer, a hole transport layer, an electron-blocking layer, light-emitting layer, a hole-blocking layer, an electron transport layer, an electron injection layer, an electron-generating layer, and the like, as an organic layer. However, the structure of the organic light-emitting device is not limited thereto, and may include a smaller or lager number of organic layers.

In the organic light-emitting device according to one embodiment of the present invention, the materials other than the heterocyclic compound represented by Formula 1 are exemplified below, but these are for illustrative purposes only and are not intended to limit the scope of the present invention, and may be replaced with materials known in the art.

As the anode material, materials having a relatively large work function may be used, and transparent conductive oxides, metals, conductive polymers, or the like may be used. Specific examples of the anode material include, but are not limited to, metals such as vanadium, chromium, copper, zinc, and gold, or alloys thereof; metal oxides such as zinc oxide, indium oxide, indium tin oxide (ITO), and indium zinc oxide (IZO); a combination of metals and oxides such as ZnO:Al or SnO₂:Sb; conductive polymers such as poly(3-methylthiophene), poly[3,4-(ethylene-1,2-dioxy)thiophene] (PEDOT), polypyrrole, and polyaniline; and the like.

As the cathode material, materials having a relatively low work function may be used, and metals, metal oxides, conductive polymers, or the like may be used. Specific examples of the cathode material include, but are not limited to, metals such as magnesium, calcium, sodium, potassium, titanium, indium, yttrium, lithium, gadolinium, aluminum, silver, tin, and lead, or alloys thereof; multilayer-structured materials such as LiF/Al or LiO₂/Al; and the like.

As the hole injection layer material, a known material for the hole injection layer may be used, for example, phthalocyanine compounds such as copper phthalocyanine, and the like, disclosed in U.S. Patent No. 4,356,429, or starburst-type amine derivatives such as tris(4-carbazolyl-9-ylphenyl)amine (TCTA), 4,4',4"-tri[phenyl(m-tolyl)amino]triphenylamine (m-MTDATA), 1,3,5-tris[4-(3-methylphenylphenylamino)phenyl]benzene (m-MTDAPB), soluble conductive polymer polyaniline/dodecylbenzenesulfonic acid or poly(3,4-ethylenedioxythiophene)/poly(4-styrenesulfonate), polyaniline/camphor sulfonic acid or polyaniline/poly(4-styrene-sulfonate), and the like, disclosed in a document [Advanced Material, 6, p.677 (1994)] may be used.

As a material for the hole transport layer, a pyrazoline derivative, an arylamine-based derivative, a stilbene derivative, a triphenyldiamine derivative, or the like may be used, and a low-molecular weight or high-molecular weight material may be used.

As a material for the electron transport layer, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone and derivatives thereof, diphenyldicyanoethylene and derivatives thereof, diphenoquinone derivatives, 8-hydroxyquinoline and derivatives thereof, and the like may be used, and high-molecular weight materials as well as low-molecular weight materials may be used.

As the electron injection layer material, for example, LiF is typically used in the art, but the present invention is not limited thereto.

As a material for the light-emitting layer, a red, green or blue light-emitting material may be used, and a mixture of two or more light-emitting materials may be used, if necessary. In this case, it is possible to use by depositing two or more light-emitting materials as separate sources, or it is possible to use by pre-mixing and depositing them as a single source. In addition, as the light-emitting layer material, a fluorescent material may be used, and a phosphorescent material may also be used. As a material for the light-emitting layer, materials that emit light by combining holes and electrons respectively injected from the anode and the cathode may be used alone, and materials in which the host material and the dopant material together participate in light emission may also be used.

When using by mixing hosts of the material for the light-emitting layer, it is possible to use by mixing hosts of the same type, and it is also possible to use by mixing different types of hosts. For example, it is possible to use by selecting any two or more types of n-type host materials or p-type host materials as a host material for the light-emitting layer.

In the phosphorescent material, those known in the art may be used as the phosphorescent dopant material. For example, phosphorescent dopant materials represented by LL'MX', LL'L"M, LMX'X", L₂MX', and L₃M may be used, but the scope of the present invention is not limited by these examples.

The M may be iridium, platinum, osmium, or the like.

The L is an anionic bidentate ligand coordinated to M by sp² carbon and a heteroatom, and X may function to trap electrons or holes. Non-limiting examples of L include 2-(1-naphthyl)benzoxazole, (2-phenylbenzoxazole), (2-phenylbenzothiazole), (2-phenylbenzothiazole), (7,8-benzoquinoline), (thiophenepyrizine), phenylpyridine, benzothiophenepyrizine, 3-methoxy-2-phenylpyridine, thiophenepyrizine, tolylpyridine, and the like. Non-limiting examples of X' and X" include acetylacetonate (acac), hexafluoroacetylacetonate, salicylidene, picolinate, 8-hydroxyquinolinate, and the like.

Specific examples of the phosphorescent dopant are shown below, but are not limited to these examples:

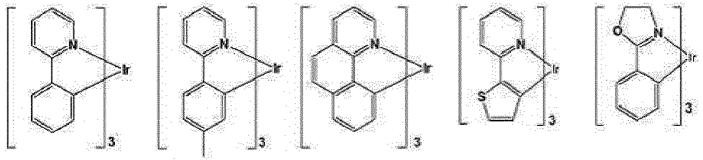

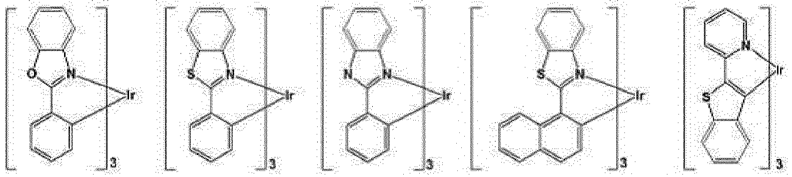

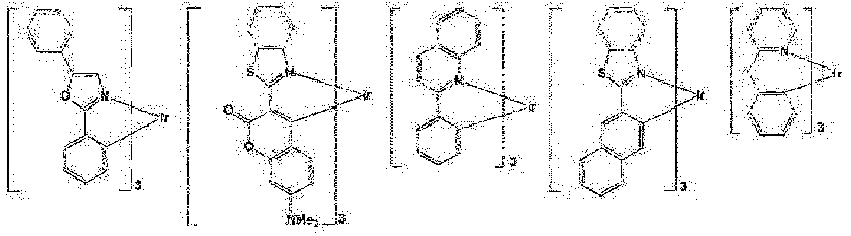

In one embodiment of the present invention, the light-emitting layer includes the heterocyclic compound represented by Formula 1, and may use it together with an iridium-based dopant.

In one embodiment of the present invention, as the iridium-based dopant, the red phosphorescent dopant (piq)₂(Ir) (acac), the green phosphorescent dopant Ir(ppy)₃, and the like may be used.

In one embodiment of the present invention, the content of the dopant may be 1% to 15%, preferably 3% to 10%, more preferably 5% to 10% based on the entire light-emitting layer.

As the material for the electronic-blocking layer, one or more compounds selected from, but not limited to, tris(phenyloyrazole)iridium, 9,9-bis[4-(N,N-bis-biphenyl-4-ylamino)phenyl]-9H-fluorene (BPAPF), bis[4-(p,p-ditolylamino)phenyl]diphenylsilane, NPD (4,4'-bis[N-(1-napthyl)-N-phenylamino]biphenyl), mCP (N,N'-dicarbazolyl-3,5-benzene), and MPMP (bis[4-(N,N-diethylamino)-2-methylphenyl](4-methylphenyl)methane) may be used.

In addition, the electron-blocking layer may include an inorganic compound. For example, it may include, but is not limited to, at least any one of halide compounds such as LiF, NaF, KF, RbF, CsF, FrF, MgF₂, CaF₂, SrF₂, BaF₂, LiCl, NaCl, KCl, RbCl, CsCl and FrCl and oxides such as Li₂O, Li₂O₂, Na₂O, K₂O, Rb₂O, Rb₂O₂, Cs₂O, Cs₂O₂, LiAlO₂, LiBO₂, LiTaO₃, LiNbO₃, LiWO₄, Li₂CO, NaWO₄, KAlO₂, K₂SiO₃, B₂O₅, Al₂O₃ and SiO₂; or a combination thereof.

As the hole-blocking layer material, an oxadiazole derivative, a triazole derivative, a phenanthroline derivative, BCP, an aluminum complex, and the like may be used without limitation.

In the organic light-emitting device of the present invention, as materials not described above, materials known in the art may be used without limitation.

The organic light-emitting device according to one embodiment of the present invention may be a top emission type, a bottom emission type, or a dual emission type depending on the material to be used.

In addition, the present invention relates to a composition for an organic layer of an organic light-emitting device, comprising the heterocyclic compound represented by Formula 1.

Specific details of the heterocyclic compound represented by Formula 1 are as described above.

The composition for an organic layer may be used as a material for a hole injection layer, a material for a hole transport layer, a material for an electron-blocking layer, a material for a light-emitting layer, a material for a hole-blocking layer, a material for an electron transport layer, and a material for an electron injection layer, in particular, may be preferably used as a material for a light-emitting layer, and specifically may be preferably used as a host material.

The composition for an organic layer may further include materials commonly used in the composition for an organic layer in the art, together with the heterocyclic compound represented by Formula 1. For example, it may further comprise a material, and the like, which are included in order to prepare the heterocyclic compound to be used in the deposition process.

In addition, the present invention relates to a method of manufacturing an organic light-emitting device, comprising the steps of: preparing a substrate; forming a first electrode on the substrate; forming one or more organic layers on the first electrode; and forming a second electrode on the organic layer, wherein the step of forming the organic layers comprises the step of forming one or more organic layers using the hetero compound represented by Formula 1 or the composition for an organic layer of the present invention.

In one embodiment of the present invention, the step of forming the organic layers may be formed by depositing the heterocyclic compound represented by Formula 1 or the composition for an organic layer using a thermal vacuum deposition method.

The organic layer including the composition for an organic layer may further include other materials commonly used in the art, if necessary.

### [Best Mode]

The heterocyclic compound represented by Formula 1 according to one embodiment of the present invention may act on the principle similar to that applied to the organic light-emitting device even in an organic electronic device including an organic solar cell, an organic photoreceptor, an organic transistor, and the like.

Hereinafter, preferred examples will be presented to help the understanding of the present invention, but the following examples are provided not to limit the present invention but to facilitate the understanding of the present invention.

### <Preparative Examples>

### Preparative Example 1: Preparation of Compounds 1 and 54

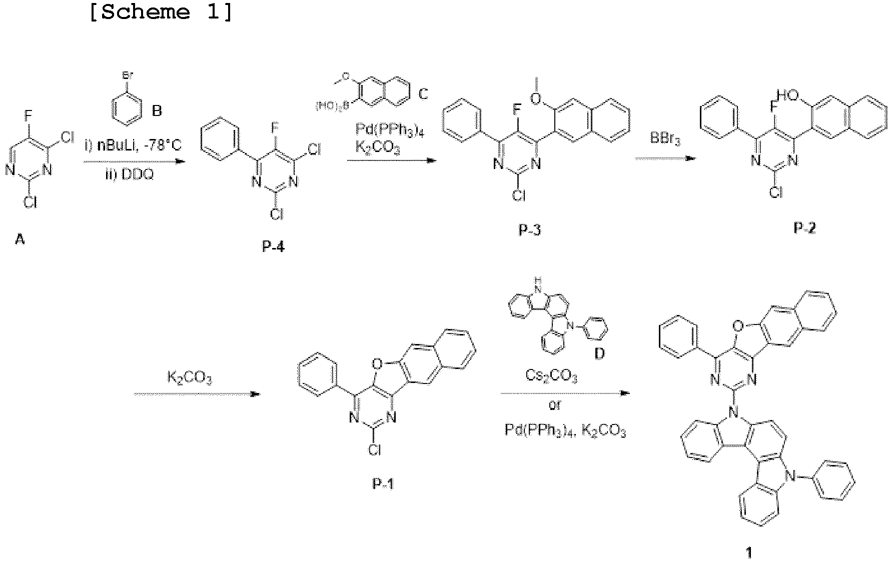

As reagents and Compounds A, B, C, and D used in the above synthesis, commercially available reagents and compounds were used.

### 1) Preparation of Compound P-4

Bromobenzene (20 g, 127.38 mmol) was dissolved in anhydrous THF, and then the temperature was lowered to -78°C. 2.5 M n-BuLi in hexane (56 mL, 140.12 mmol) was slowly added thereto under nitrogen. After 30 minutes, a solution of 2,4-dichloro-5-fluoropyrimidine (21 g, 127.38 mmol) dissolved in anhydrous THF was slowly added thereto. After stirring for 1 hour, the reaction solution was raised to room temperature, and DDQ (29 g, 127.38 mmol) was added thereto and stirred for an additional 1 hour. The reaction was stopped by adding 127 mL of 1 M NaOH (aq) thereto, and it was diluted with an excess of DCM and extracted with water.

The organic layer was dried over MgSO₄, and then filtered through silica gel. The filtered filtrate was subjected to removal of the solvent by a rotary evaporator followed by column chromatography (MC/Hex=1/2) to obtain 20 g of Compound P-4 as an ivory solid in 67% yield.

### 2) Preparation of Compound P-3

2,4-Dichloro-5-fluoro-6-phenylpyrimidine (20 g, 81.96 mmol), (3-methoxynaphthalen-2-yl)boronic acid (16.5 g, 81.96 mmol), Pd(PPh₃)₄ (4.7 g, 4.09 mmol), and K₂CO₃ (22 g, 163.92 mmol) were dissolved in 1,4-dioxane/H₂O (200 mL/50 mL), and then stirred at 100°C for 4 hours. After the reaction was completed, the mixed solution was dissolved in MC and extracted with water, and the organic layer was dried over anhydrous MgSO₄, and then filtered through silica gel. The filtered filtrate was subjected to removal of the solvent by a rotary evaporator followed by column chromatography (MC/Hex=1/1) to obtain 18.5 g of Compound P-3 as a white solid in 62% yield.

### 3) Preparation of Compound P-2

2-Chloro-5-fluoro-4-(3-methoxynaphthalen-2-yl)-6-phenylpyrimidine (18.5 g, 50.71 mmol) was dissolved in 200 mL of DCM, and then the temperature was lowered to 0°C. BBr₃ (4.8 mL, 50.71 mmol) was slowly added thereto, and after 10 minutes, the temperature was raised to room temperature and stirred for 2 hours. After the reaction was completed, the mixed solution was lowered to 0°C, and the reaction was stopped by slowly adding water thereto. The reaction solution was extracted with water, and the organic layer was dried over anhydrous MgSO₄, and then filtered through silica gel. The filtered filtrate was subjected to removal of the solvent by a rotary evaporator to obtain 17 g of Compound P-2 as a yellow solid in 95% yield without further purification.

### 4) Preparation of Compound P-1

3-(2-Chloro-5-fluoro-6-phenylpyrimidin-4-yl)naphthalen-2-ol (17 g, 48.46 mmol) was dissolved in 150 mL of DMA, and then K₂CO₃ (13 g, 96.92 mmol) was placed therein and stirred at 150°C for 3 hours. After the reaction was completed, After the reaction was completed, the mixed solution was subjected to removal of the solvent by a high-pressure rotary evaporator and diluted with an excess of MC, and then extracted with water. The organic layer was dried over anhydrous MgSO₄ and filtered through silica gel, and then the filtered filtrate was subjected to removal of the solvent by a rotary evaporator to obtain 14 g of Compound P-1 as an ivory solid in 89% yield without further purification.

### 5) Preparation of Compound 1

2-Chloro-4-phenylnaphtho[2',3':4,5]puro[3,2-d]pyrimidine (14 g, 42.32 mmol), 5-phenyl-5,8-dihydroindolo[2,3-c]carbazole (14 g, 42.32 mmol), and Cs₂CO₃ (27 g, 84.64 mmol) were dissolved in 150 mL of DMA, and then stirred at 150°C for 4 hours. After the reaction was completed, the mixed solution was subjected to removal of the solvent by a high-pressure rotary evaporator and diluted with an excess of MC, and then extracted with water. The organic layer was dried over anhydrous MgSO₄ and filtered through silica gel, and then the filtered filtrate was subjected to removal of the solvent by a rotary evaporator followed by column chromatography (MC/Hex=1/1) to obtain 13 g of Target Compound 1 as a yellow solid in 50% yield.

### 6) Preparation of Compound 54

2-Chloro-4-(9-phenyl-7H-benzo[c]carbazole-7-yl)naphtho[2',3':4,5]puro[3,2-d]pyrimidine (10 g, 18.31 mmol), naphthalen-1-ylboronic acid (3.1 g, 18.31 mmol), Pd(PPh₃)₄ (1.1 g, 0.92 mmol), and K₂CO₃ (5 g, 36.62 mmol) were dissolved in 1,4-dioxane/H₂O (120 mL/30 mL), and then stirred at 100°C for 4 hours. After the reaction was completed, the mixed solution was cooled to room temperature, and then the precipitated solid was filtered and washed with water, MeOH, and acetone. The dried solid was dissolved in an excess of chloroform and filtered through silica gel. The filtered filtrate was subjected to removal of the solvent by a rotary evaporator followed by recrystallization with CB to obtain 7 g of Target Compound 54 as a yellow solid in 63% yield.

The following target compound was synthesized in the same manner as in Preparative Example 1 above, except that B, C, and D of Table 1 below were used as intermediates.

**[Table 1]**

| No. | Intermediate B | Intermediate C | Intermediate D | Target Compound |
|---|---|---|---|---|
| 1 | 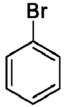 | 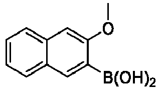 | 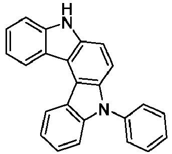 | 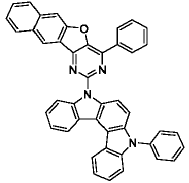 |
| 10 | 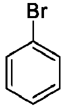 | 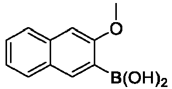 | 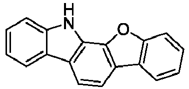 | 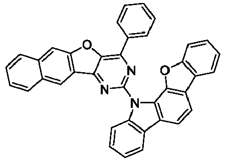 |
| 30 | 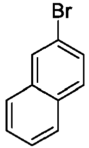 | 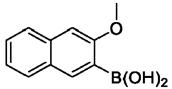 | 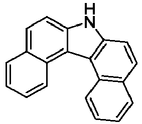 | 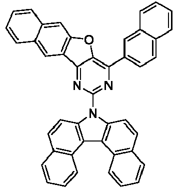 |
| 54 | 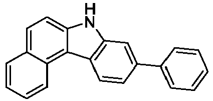 | 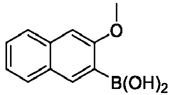 | 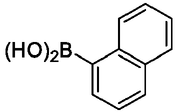 | 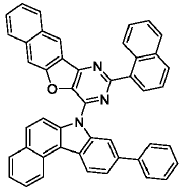 |
| 59 | 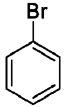 | 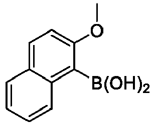 | 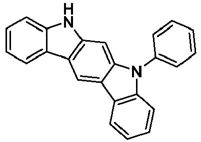 | 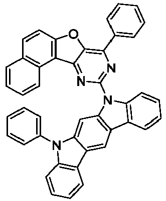 |
| 68 | 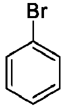 | 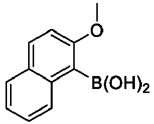 | 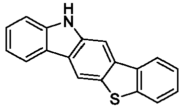 | 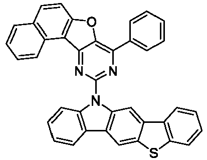 |
| 82 | 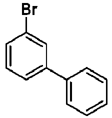 | 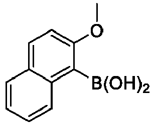 | 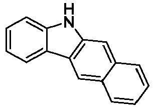 | 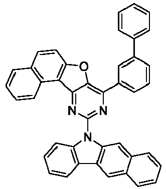 |
| 91 | 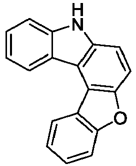 | 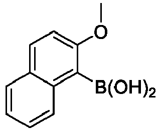 | 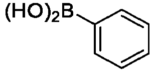 | 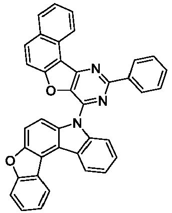 |
| 108 | 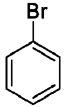 | 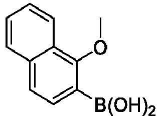 | 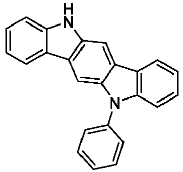 | 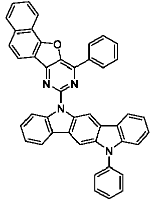 |
| 127 | 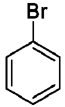 | 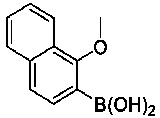 | 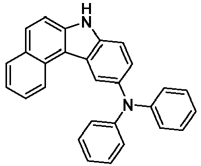 | 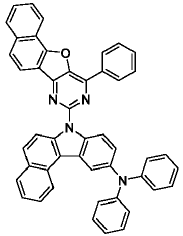 |
| 131 | 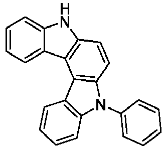 | 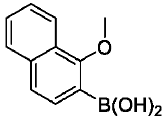 | 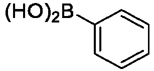 | 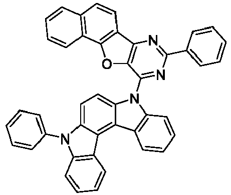 |
| 143 | 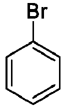 | 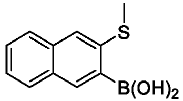 | 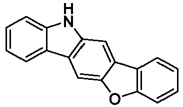 | 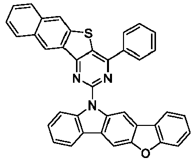 |
| 164 | 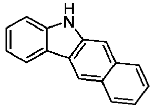 | 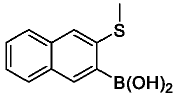 | 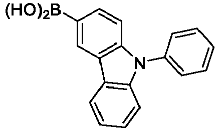 | 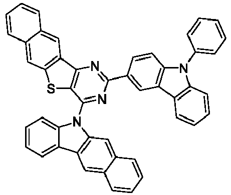 |
| 168 | 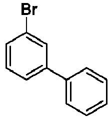 | 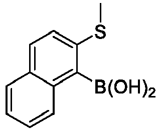 | 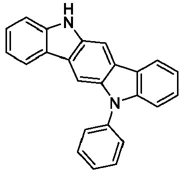 | 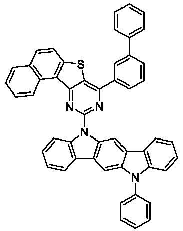 |
| 199 | 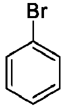 | 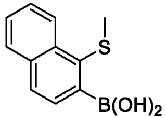 | 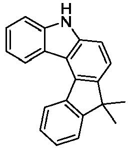 | 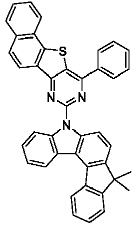 |
| 213 | 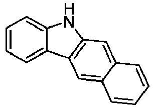 | 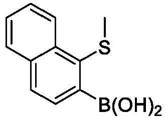 | 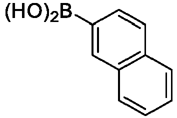 | 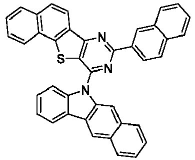 |

### Preparative Example 2: Preparation of Compounds 221 and 233

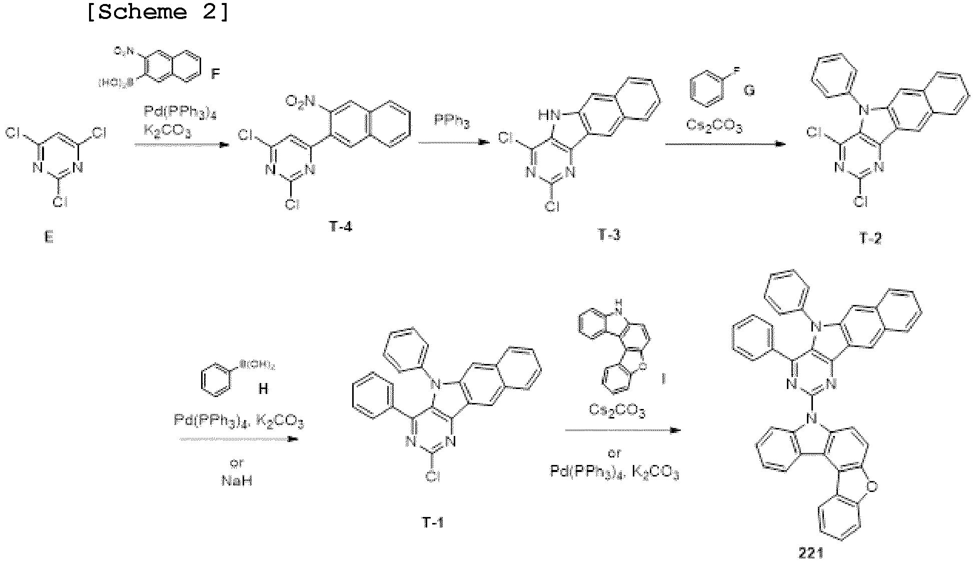

As reagents and Compounds E, F, G, H, and I used in the above synthesis, commercially available reagents and compounds were used.

### 1) Preparation of Intermediate T-4

2,4,6-Trichloropyrimidine (20 g, 109.04 mmol), (3-nitronaphthalen-2-yl)boronic acid (23 g, 109.04 mmol), Pd(PPh₃)₄ (6.3 g, 5.45 mmol), and K₂CO₃ (30 g, 218.08 mmol) were dissolved in 1,4-dioxane/H₂O (250 mL/50 mL), and then stirred at 100°C for 4 hours. After the reaction was completed, the mixed solution was cooled to room temperature, diluted with an excess of MC, and extracted with water, and the organic layer was dried over anhydrous MgSO₄, and then filtered through silica gel. The filtered filtrate was subjected to removal of the solvent by a rotary evaporator followed by column chromatography (MC/Hex=1/2) to obtain 18 g of Intermediate T-4 as a yellow solid in 53% yield.

### 2) Preparation of Intermediate T-3

2,4-Dichloro-6-(3-nitronaphthalen-2-yl)pyrimidine (18 g, 56.22 mmol) and PPh₃ (29 g, 112.44 mmol) were dissolved in 150 mL of DCB, and then stirred at 180°C for 15 hours. After the reaction was completed, the mixed solution was cooled to room temperature, and then subjected to removal the solvent by a high-pressure rotary evaporator, diluted with an excess of MC, and extracted with water. The organic layer was dried over anhydrous MgSO₄, and then filtered through silica gel, and the filtered filtrate was subjected to removal of the solvent by a rotary evaporator followed by column chromatography (MC/Hex=1/1) to obtain 14 g of Intermediate T-34 as a pale yellow solid in 87% yield.

### 3) Preparation of Intermediate T-2

2,4-Dichloro-5H-benzo[f]pyrimido[5,4-b]indole (14 g, 48.48 mmol), fluorobenzene (4.6 g, 48.48 mmol), and Cs₂CO₃ (31 g, 96.96 mmol) were dissolved in 150 mL of DMA, and then stirred at 150°C for 2 hours. After the reaction was completed, the mixed solution was cooled to room temperature, and then the solvent was removed therefrom by a high-pressure rotary evaporator, and it was diluted with an excess of MC and extracted with water. The organic layer was dried over anhydrous MgSO₄, and then filtered through silica

gel, and the filtered filtrate was subjected to removal of the solvent by a rotary evaporator to obtain 16 g of Intermediate T-2 as an ivory solid in 94% yield without further purification.

### 4) Preparation of Intermediate T-1

2,4-Dichloro-5-phenyl-5H-benzo[f]pyrimido[5,4-b]indole (8 g, 21.96 mmol), phenylboronic acid (2.6 g, 21.96 mmol), Pd(PPh₃)₄ (1.2 g, 1,09 mmol), and K₂CO₃ (6 g, 43.92 mmol) were dissolved in 1,4-dioxane/H₂O (100 mL/30 mL), and then stirred at 100°C for 4 hours. After the reaction was completed, the mixed solution was cooled to room temperature, and then the precipitated solid was filtered and washed with water and MeOH. The dried solid was diluted with an excess of chloroform and filtered through silica gel. The filtered filtrate was subjected to removal of the solvent by a rotary evaporator followed by recrystallization with EA/Hex to obtain 7 g of Intermediate T-1 as a pale gray solid in 78% yield.

### 5) Preparation of Compound 221

2-Chloro-4,5-diphenyl-5H-benzo[f]pyrimido[5,4-b]indole (7 g, 17.24 mmol), 8H-benzofuro[2,3-c]carbazole (4.4 g, 17.24 mmol), and Cs₂CO₃ (11 g, 34.48 mmol) were dissolved in 100 mL of DMA, and then stirred at 150°C for 4 hours. After the reaction was completed, the mixed solution was cooled to room temperature, and then a solid was precipitated by adding water thereto. The precipitated solid was filtered and washed with water, MeOH, and acetone. The dried solid was diluted with chloroform and filtered through silica gel, and then the filtered filtrate was subjected to removal of the solvent by a rotary evaporator followed by recrystallization with DCB to obtain 6.4 g of Target Compound 221 as a yellow solid in 62% yield.

### 6) Preparation of Compound 233 T-1

2,4-Dichloro-5-phenyl-5H-benzo[f]pyrimido[5,4-b]indole (8 g, 21.96 mmol) was dissolved in 80 mL of DMF, and then the temperature was lowered to 0°C and NaH (630 mg, 26.35 mmol) was slowly added thereto. After stirring for 30 minutes, 5-(naphthalen-2-yl)-5,11-dihydroindole[3,2-b]carbazole (8.4 g, 21.96 mmol) was slowly added thereto, and after 10 minutes, it was raised to room temperature and stirred for 1 hour. After the reaction was completed, the reaction was stopped by adding water little by little to the solution, and then the precipitated solid was filtered and washed with water and MeOH. The dried solid was diluted with an excess of MC and filtered through silica gel, and the filtered filtrate was subjected to removal of the solvent by a rotary evaporator. The obtained solid was recrystallized with EA to obtain 11.5 g of Compound 233 T-1 as a yellow solid in 76% yield.

### 7) Preparation of Compound 233

5-(2-Chloro-5-phenyl-5H-benzo[f]pyrimido[5,4-b]indol-4-yl)-11-(naphthalen-2-yl)-5,11-dihydroindole[3,2-b]carbazole (11.5 g, 16.19 mmol), phenylboronic acid (2 g, 16.19 mmol), Pd(PPh₃)₄ (0.9 g, 0.81 mmol), and K₂CO₃ (4.5 g, 32.38 mmol) were dissolved in 1,4-dioxane/H₂O (100 mL/30 mL), and then stirred at 100°C for 4 hours. After the reaction was completed, the mixed solution was cooled to room temperature, and then the precipitated solid was filtered and washed with water, MeOH, and acetone. The dried solid was diluted with an excess of chloroform and filtered through silica gel. The filtered filtrate was subjected to removal of the solvent by a rotary evaporator followed by recrystallization with CB to obtain 8 g of Target Compound 233 as a yellow solid in 67% yield.

The following target compound was synthesized in the same manner as in Preparative Example 2 above, except that F, G, H, and I of Table 2 below were used as intermediates.

**[Table 2]**

| No. | Intermediate F | Intermediate G | Intermediate H | Intermediate I | Target Compound |
|---|---|---|---|---|---|
| 221 | 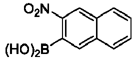 | 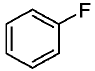 | 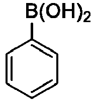 | 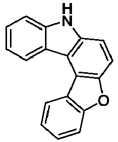 | 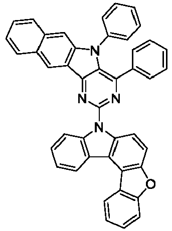 |
| 233 | 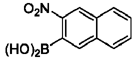 | 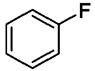 | 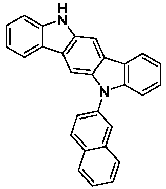 | 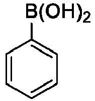 | 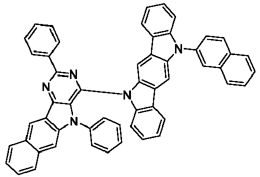 |
| 253 | 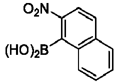 | 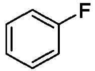 | 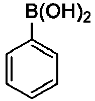 | 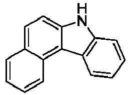 | 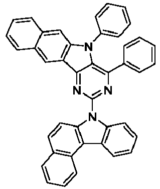 |
| 265 | 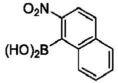 | 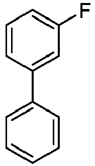 | 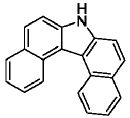 | 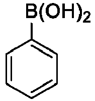 | 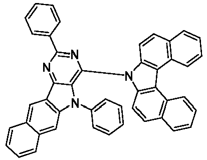 |
| 272 | 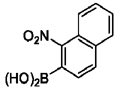 | 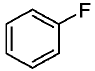 | 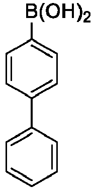 | 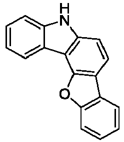 | 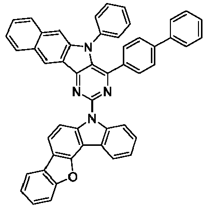 |
| 285 | 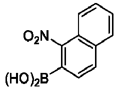 | 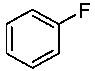 | 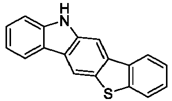 | 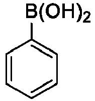 | 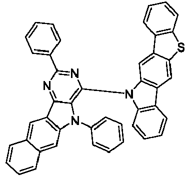 |

The compounds were prepared in the same manner as in the above preparative examples and the synthesis confirmation results are shown in Tables 3 and 4. Table 3 shows the measured values of field desorption-mass spectrometry (FD-MS), and Table 4 shows the measured values of ¹H NMR (CDCl₃, 200 Mz) .

**[Table 3]**

| Compound | FD-MS | Compound | FD-MS | Compound | FD-MS |
|---|---|---|---|---|---|
| 1 | m/z=626.72(C44H26N4O) | 2 | m/z=626.72(C44H26N4O) | 3 | m/z=626.72(C44H26N4O) |
| 4 | m/z=626.72(C44H26N4O) | 5 | m/z=626.72(C44H26N4O) | 6 | m/z=551.61(C38H21N3O2) |
| 7 | m/z=551.61(C38H21N3O2) | 8 | m/z=551.61(C38H21N3O2) | 9 | m/z=551.61(C38H21N3O2) |
| 10 | m/z=551.61(C38H21N3O2) | 11 | m/z=567.67(C38H21N3OS) | 12 | m/z=567.67(C38H21N3OS) |
| 13 | m/z=567.67(C38H21N3OS) | 14 | m/z=567.67(C38H21N3OS) | 15 | m/z=567.67(C38H21N3OS) |
| 16 | m/z=577.69(C41H27N3O) | 17 | m/z=577.69(C41H27N3O) | 18 | m/z=577.69(C41H27N3O) |
| 19 | m/z=676.78(C48H28N4O) | 20 | m/z=702.85(C50H30N4O) | 21 | m/z=676.78(C48H28N4O) |
| 22 | m/z=702.85(C50H30N4O) | 23 | m/z=676.78(C48H28N4O) | 24 | m/z=702.85(C50H30N4O) |
| 25 | m/z=676.78(C48H28N4O) | 26 | m/z=561.64(C40H23N3O) | 27 | m/z=511.58(C36H21N3O) |
| 28 | m/z=587.68(C42H25N3O) | 29 | m/z=678.80(C48H30N4O) | 30 | m/z=611.70(C44H25N3O) |
| 31 | m/z=587.68(C42H25N3O) | 32 | m/z=676.78(C48H28N4O) | 33 | m/z=637.78(C46H27N3O) |
| 34 | m/z=626.72(C44H26N4O) | 35 | m/z=702.85(C50H30N4O) | 36 | m/z=676.78(C48H28N4O) |
| 37 | m/z=551.61(C38H21N3O2) | 38 | m/z=567.67(C38H21N3OS) | 39 | m/z=551.61(C38H21N3O2) |
| 40 | m/z=617.73(C42H23N3OS) | 41 | m/z=577.69(C41H27N3O) | 42 | m/z=626.72(C44H26N4O) |
| 43 | m/z=617.73(C42H23N3OS) | 44 | m/z=561.64(C40H23N3O) | 45 | m/z=676.78(C48H28N4O) |
| 46 | m/z=511.58(C36H21N3O) | 47 | m/z=676.78(C48H28N4O) | 48 | m/z=561.64(C40H23N3O) |
| 49 | m/z=600.68(C42H24N40) | 50 | m/z=587.68(C42H25N3O) | 51 | m/z=587.68(C42H25N3O) |
| 52 | m/z=678.80(C48H30N4O) | 53 | m/z=587.68(C42H25N3O) | 54 | m/z=637.74(C46H27N3O) |
| 55 | m/z=676.78(C48H28N4O) | 56 | m/z=626.72(C44H26N4O) | 57 | m/z=626.72(C44H26N4O) |
| 58 | m/z=626.72(C44H26N4O) | 59 | m/z=626.72(C44H26N4O) | 60 | m/z=626.72(C44H26N4O) |
| 61 | m/z=551.61(C38H21N3O2) | 62 | m/z=551.61(C38H21N3O2) | 63 | m/z=551.61(C38H21N3O2) |
| 64 | m/z=551.61(C38H21N3O2) | 65 | m/z=551.61(C38H21N3O2) | 66 | m/z=567.67(C38H21N3OS) |
| 67 | m/z=567.67(C38H21N3OS) | 68 | m/z=567.67(C38H21N3OS) | 69 | m/z=567.67(C38H21N3OS) |
| 70 | m/z=567.67(C38H21N3OS) | 71 | m/z=577.69(C41H27N3O) | 72 | m/z=701.83(C51H31N3O) |
| 73 | m/z=701.83(C51H31N3O) | 74 | m/z=577.69(C41H27N3O) | 75 | m/z=577.69(C41H27N3O) |
| 76 | m/z=702.85(C50H30N4O) | 77 | m/z=676.78(C48H28N4O) | 78 | m/z=702.85(C50H30N4O) |
| 79 | m/z=677.76(C48H27N3O2) | 80 | m/z=678.80(C48H30N4O) | 81 | m/z=611.70(C44H25N3O) |
| 82 | m/z=587.68(C42H25N3O) | 83 | m/z=561.64(C40H23N3O) | 84 | m/z=637.74(C46H27N3O) |
| 85 | m/z=587.68(C42H25N3O) | 86 | m/z=676.78(C48H28N4O) | 87 | m/z=637.78(C46H27N3O) |
| 88 | m/z=626.72(C44H26N4O) | 89 | m/z=676.78(C48H28N4O) | 90 | m/z=716.80(C50H28N4O2) |
| 91 | m/z=551.61(C38H21N3O2) | 92 | m/z=567.67(C38H21N3OS) | 93 | m/z=643.76(C44H25N3OS) |
| 94 | m/z=627.70(C44H25N3O2) | 95 | m/z=577.69(C41H27N3O) | 96 | m/z=676.78(C48H28N4O) |
| 97 | m/z=511.58(C36H21N3O) | 98 | m/z=587.68(C42H25N3O) | 99 | m/z=561.64(C40H23N3O) |
| 100 | m/z=637.74(C46H27N3O) | 101 | m/z=511.58(C36H21N3O) | 102 | m/z=637.74(C46H27N3O) |
| 103 | m/z=677.76(C48H27N3O2) | 104 | m/z=663.76(C48H29N3O) | 105 | m/z=626.72(C44H26N4O) |
| 106 | m/z=626.72(C44H26N4O) | 107 | m/z=626.72(C44H26N4O) | 108 | m/z=626.72(C44H26N4O) |
| 109 | m/z=702.85(C50H30N4O) | 110 | m/z=626.72(C44H26N4O) | 111 | m/z=551.61(C38H21N3O2) |
| 112 | m/z=551.61(C38H21N3O2) | 113 | m/z=627.70(C44H25N3O2) | 114 | m/z=551.61(C38H21N3O2) |
| 115 | m/z=677.76(C48H27N3O2) | 116 | m/z=567.67(C38H21N3OS) | 117 | m/z=567.67(C38H21N3OS) |
| 118 | m/z=567.67(C38H21N3OS) | 119 | m/z=577.69(C41H27N3O) | 120 | m/z=577.69(C41H27N3O) |
| 121 | m/z=511.58(C36H21N3O) | 122 | m/z=587.68(C42H25N3O) | 123 | m/z=561.64(C40H23N3O) |
| 124 | m/z=587.68(C42H25N3O) | 125 | m/z=587.68(C42H25N3O) | 126 | m/z=713.84(C52H31N3O) |
| 127 | m/z=678.80(C48H30N4O) | 128 | m/z=637.74(C46H27N3O) | 129 | m/z=676.78(C48H28N4O) |
| 130 | m/z=611.70(C44H25N3O) | 131 | m/z=626.72(C44H26N4O) | 132 | m/z=551.61(C38H21N3O2) |
| 133 | m/z=511.58(C36H21N3O) | 134 | m/z=587.68(C42H25N3O) | 135 | m/z=561.64(C40H23N3O) |
| 136 | m/z=542.78(C44H26N4S) | 137 | m/z=542.78(C44H26N4S) | 138 | m/z=718.88(C50H30N4S) |
| 139 | m/z=542.78(C44H26N4S) | 140 | m/z=542.78(C44H26N4S) | 141 | m/z=567.67(C38H21N3OS) |
| 142 | m/z=643.76(C44H25N3OS) | 143 | m/z=567.67(C38H21N3OS) | 144 | m/z=567.67(C38H21N3OS) |
| 145 | m/z=617.73(C42H23N3OS) | 146 | m/z=583.73(C38H21N3S2) | 147 | m/z=583.73(C38H21N3S2) |
| 148 | m/z=593.75(C41H27N3S) | 149 | m/z=669.85(C47H31N3S) | 150 | m/z=715.87(C51H29N3S) |
| 151 | m/z=577.70(C40H23N3S) | 152 | m/z=603.73(C42H25N3S) | 153 | m/z=603.73(C42H25N3S) |
| 154 | m/z=653.80(C46H27N3S) | 155 | m/z=603.73(C42H25N3S) | 156 | m/z=694.86(C48H30N4S) |
| 157 | m/z=653.80(C46H27N3S) | 158 | m/z=679.84(C48H29N3S) | 159 | m/z=653.80(C46H27N3S) |
| 160 | m/z=577.70(C40H23N3S) | 161 | m/z=642.78(C44H26N4S) | 162 | m/z=642.78(C44H26N4S) |
| 163 | m/z=617.73(C42H23N3OS) | 164 | m/z=692.84(C48H28N3S) | 165 | m/z=577.70(C40H23N3S) |
| 166 | m/z=642.78(C44H26N4S) | 167 | m/z=642.78(C44H26N4S) | 168 | m/z=718.88(C50H30N4S) |
| 169 | m/z=642.78(C44H26N4S) | 170 | m/z=692.84(C48H28N3S) | 171 | m/z=567.67(C38H21N3OS) |
| 172 | m/z=643.76(C44H25N3OS) | 173 | m/z=567.67(C38H21N3OS) | 174 | m/z=583.73(C38H21N3S2) |
| 175 | m/z=583.73(C38H21N3S2) | 176 | m/z=669.85(C47H31N3S) | 177 | m/z=717.89(C51H31N3S) |
| 178 | m/z=642.78(C44H26N4S) | 179 | m/z=643.76(C44H25N3OS) | 180 | m/z=633.79(C42H23N3S2) |
| 181 | m/z=679.84(C48H29N3S) | 182 | m/z=577.70(C40H23N3S) | 183 | m/z=653.80(C46H27N3S) |
| 184 | m/z=653.80(C46H27N3S) | 185 | m/z=679.84(C48H29N3S) | 186 | m/z=642.78(C44H26N4S) |
| 187 | m/z=642.78(C44H26N4S) | 188 | m/z=642.78(C44H26N4S) | 189 | m/z=718.88(C50H30N4S) |
| 190 | m/z=642.78(C44H26N4S) | 191 | m/z=567.67(C38H21N3OS) | 192 | m/z=567.67(C38H21N3OS) |
| 193 | m/z=643.76(C44H25N3OS) | 194 | m/z=567.67(C38H21N3OS) | 195 | m/z=693.82(C48H27N3OS) |
| 196 | m/z=583.73(C38H21N3S2) | 197 | m/z=583.73(C38H21N3S2) | 198 | m/z=583.73(C38H21N3S2) |
| 199 | m/z=593.75(C41H27N3S) | 200 | m/z=593.75(C41H27N3S) | 201 | m/z=527.64(C36H21N3S) |
| 202 | m/z=603.73(C42H25N3S) | 203 | m/z=577.70(C40H23N3S) | 204 | m/z=603.73(C42H25N3S) |
| 205 | m/z=603.73(C42H25N3S) | 206 | m/z=729.90(C52H31N3S) | 207 | m/z=694.86(C48H3ON3S) |
| 208 | m/z=653.80(C46H27N3S) | 209 | m/z=692.84(C48H28N3S) | 210 | m/z=627.70(C44H25N3O2) |
| 211 | m/z=642.78(C44H26N4S) | 212 | m/z=643.76(C44H25N3OS) | 213 | m/z=577.70(C40H23N3S) |
| 214 | m/z=653.80(C46H27N3S) | 215 | m/z=694.86(C48H3ON3S) | 216 | m/z=701.83(C50H31N5) |
| 217 | m/z=701.83(C50H31N5) | 218 | m/z=751.89(C54H33N5) | 219 | m/z=701.83(C50H31N5) |
| 220 | m/z=701.83(C50H31N5) | 221 | m/z=626.72(C44H26N4O) | 222 | m/z=702.85(C50H30N4O) |
| 223 | m/z=626.72(C44H26N4O) | 224 | m/z=642.78(C44H26N4S) | 225 | m/z=642.78(C44H26N4S) |
| 226 | m/z=652.80(C47H32N4) | 227 | m/z=652.80(C47H32N4) | 228 | m/z=753.91(C54H35N5) |
| 229 | m/z=686.82(C50H30N4) | 230 | m/z=662.80(C48H30N4) | 231 | m/z=636.76(C46H28N4) |
| 232 | m/z=636.76(C46H28N4) | 233 | m/z=751.89(C54H33N5) | 234 | m/z=701.83(C50H31N5) |
| 235 | m/z=652.80(C47H32N4) | 236 | m/z=642.78(C44H26N4S) | 237 | m/z=676.78(C48H28N4O) |
| 238 | m/z=586.70(C42H26N4) | 239 | m/z=636.76(C46H28N4) | 240 | m/z=586.70(C42H26N4) |
| 241 | m/z=751.89(C54H33N5) | 242 | m/z=701.83(C50H31N5) | 243 | m/z=701.83(C50H31N5) |
| 244 | m/z=701.83(C50H31N5) | 245 | m/z=701.83(C50H31N5) | 246 | m/z=702.82(C50H30N4O) |
| 247 | m/z=702.82(C50H30N4O) | 248 | m/z=626.72(C44H26N4O) | 249 | m/z=642.78(C44H26N4S) |
| 250 | m/z=642.78(C44H26N4S) | 251 | m/z=652.80(C47H32N4) | 252 | m/z=652.80(C47H32N4) |
| 253 | m/z=586.70(C42H26N4) | 254 | m/z=662.80(C48H30N4) | 255 | m/z=636.76(C46H28N4) |
| 256 | m/z=636.76(C46H28N4) | 257 | m/z=586.70(C42H26N4) | 258 | m/z=701.83(C50H31N5) |
| 259 | m/z=676.78(C48H28N4O) | 260 | m/z=652.80(C47H32N4) | 261 | m/z=642.78(C44H26N4S) |
| 262 | m/z=586.70(C42H26N4) | 263 | m/z=662.80(C48H30N4) | 264 | m/z=636.76(C46H28N4) |
| 265 | m/z=712.86(C52H32N4) | 266 | m/z=751.89(C54H33N5) | 267 | m/z=701.83(C50H31N5) |
| 268 | m/z=701.83(C50H31N5) | 269 | m/z=701.83(C50H31N5) | 270 | m/z=701.83(C50H31N5) |
| 271 | m/z=626.72(C44H26N4O) | 272 | m/z=702.82(C50H30N4O) | 273 | m/z=626.72(C44H26N4O) |
| 274 | m/z=642.78(C44H26N4S) | 275 | m/z=642.78(C44H26N4S) | 276 | m/z=652.80(C47H32N4) |
| 277 | m/z=652.80(C47H32N4) | 278 | m/z=636.76(C46H28N4) | 279 | m/z=586.70(C42H26N4) |
| 280 | m/z=586.70(C42H26N4) | 281 | m/z=662.80(C48H30N4) | 282 | m/z=636.76(C46H28N4) |
| 283 | m/z=701.83(C50H31N5) | 284 | m/z=702.82(C50H30N4O) | 285 | m/z=642.78(C44H26N4S) |
| 286 | m/z=662.80(C48H30N4) | 287 | m/z=676.78(C48H28N4O) | 288 | m/z=586.70(C42H26N4) |
| 289 | m/z=636.76(C46H28N4) | 290 | m/z=712.86(C52H32N4) | | |

**[Table 4]**

| **No.** | **¹H NMR (CDCl₃, 300Mz)** |
|---|---|
| **1** | 6.70(m, 6H), 7.07-7.10(m, 4H), 7.20-7.23(m, 8H), 7.33-7.39(m, 3H), 7.66(d, 2H), 7.89(d, 2H), 8.00(s, 1H) |
| **10** | 6.70(m, 6H), 7.07-7.10(m, 3H), 7.20-7.23(m, 5H), 7.33-7.39(m, 3H), 7.41(t, 2H), 7.62(s, 1H), 7.88(d, 1H) |
| **30** | 6.70(m, 6H), 7.08(d, 1H), 7.33-7.39(m, 7H), 7.41-7.55(m, 5H), 7.62(s, 1H), 7.73(d, 2H), 7.77(d, 1H), 7.92(s, 1H), 7.98(d, 1H) |
| **54** | 6.89(m, 6H), 7.27(d, 1H), 7.40-7.59(m, 11H), 7.62(d, 1H), 7.75-7.88(m, 7H), 7.98(d, 1H) |
| **59** | 6.85-6.87(m, 4H), 7.29(t, 1H), 7.38-7.40(m, 5H), 7.51-7.56(m, 8H), 7.74(d, 1H), 7.76(d, 1H), 7.87(d, 1H), 7.93(s, 1H), 7.98(d, 2H), 8.22-8.25(m, 2H) |
| **68** | 7.25(dd, 8H), 7.41(t, 2H), 7.44-7.49(m, 6H), 7.51-7.55(m, 2H), 7.73(d, 2H), 7.99(d, 1H) |
| **82** | 7.25-7 26(dd, 8H), 7.28(s, 1H), 7.42-7.48(m, 3H), 7.51-7.54(m, 6H), 7.62(d, 1H), 7.74(d, 1H), 7.78(d, 1H), 7.87(d, 1H), 7.91(s, 1H), 7.98(d, 1H) |
| **91** | 6.66-6.69(m, 4H), 6.75(s, 1H), 7.07(d, 1H), 7.28-7.30(m, 4H), 7.51-7.54(m, 8H), 7.81-7.88(m, 3H) |
| **108** | 7.35(s, 1H), 7.45(d, 1H), 7.49(t, 1H), 7.51-7.54(m, 7H), 7.60-7.65(m, 10H), 7.83-7.90(m, 3H), 8.20-8.23(m, 3H) |
| **127** | 6.88-6.89(m, 2H), 7.07(d, 1H), 7.44-7.48.(m, 5H), 7.50-7.59(m, 12H), 7.73-7.76(m, 4H), 7.93-7.98(m, 4H), 8.11(d, 1H), 8.28.(d, 1H) |
| **131** | 7.26-7.31(m, 6H), 7.44(t, 2H), 7.51-7.54(m, 7H), 7.60-7.65(m, 6H), 7.67-7.77(m, 2H), 8.00(d, 1H) |
| **143** | 7.20-7.23(m, 4H), 7.33-7.39(m, 7H), 7.42-7.60(m, 6H), 7.73(d, 2H), 7.90(s, 1H), 8.12(d, 1H) |
| **164** | 6.65-6.69(m, 6H), 6.81(t, 1H), 7.20-7.23(m, 4H), 7.38-7.40(m, 8H), 7.45-7.60(m, 7H), 7.75(d, 1H), 7.95(s, 1H) |
| **168** | 6.69-6.71(m, 6H), 7.06-7.11(m, 5H), 7.20-7.23(m, 4H), 7.33-7.39(m, 7H), 7.42-7.60(m, 4H), 7.73(d, 2H), 7.89(s, 1H), 7.98(d, 1H) |
| **199** | 1.72(s, 6H), 7.06-7.12 (m, 5H), 7.25-7.27 (m, 4H), 7.40-7.61(m, 9H), 7.73-7.77(m, 4H), 7.87-7.89(m, 3H), 7.98(d, 1H) |
| **213** | 7.16-7.18(m, 4H), 7.20-7.23(m, 5H), 7.33-7.39(m, 7H), 7.42-7.60(m, 3H), 7.63(d, 2H), 7.73(d, 1H), 7.98(d, 1H) |
| **221** | 7.06-7.10(m, 5H), 7.20-7.23(m, 7H), 7.33-7.39(m, 8H), 7.66(d, 2H), 7.89(d, 1H), 8.01-8.06(m, 2H), 8.22(d, 1H) |
| **233** | 7.15-7.18(m, 4H), 7.21-7.30(m, 6H), 7.38-7.45(m, 8H), 7.51-7.55(m, 2H), 7.58-7.60(m, 3H), 7.62(s, 1H), 7.65(d, 1H), 7.69-7.72(m, 5H), 7.90(d, 1H), 8.06(d, 1H), 8.22(d, 1H) |
| **253** | 7.32-7.45(m, 12H), 7.51-7.55(m, 6H), 7.66(d, 1H), 7.77(d, 1H), 7.80-7.85(m, 3H), 7.89(d, 1H), 7.92(t, 1H), 8.23(d, 1H) |
| **265** | 7.28-7.40(m, 10H), 7.51-7.56(m, 6H), 7.62-7.66(m, 6H), 7.71-7.75(m, 5H), 7.77(d, 1H), 7.80(s, 1H), 7.87-7.89(m, 2H), 8.24(d, 1H) |
| **272** | 7.17(d, 1H), 7.30-7.41(m, 7H), 7.51-7.57(m, 9H), 7.62-7.66(m, 2H), 7.77(d, 2H), 7.80-7.83(m, 4H), 7.87-7.92(m, 4H), 8.23(d, 1H) |
| **285** | 7.25-7.27(m, 6H), 7.38-7.41(m, 10H), 7.48-7.52(m, 6H), 7.65(d, 1H), 7.77(d, 1H), 7.90(d, 1H), 7.93(s, 1H) |

### Experimental Example 1: Manufacturing of organic light-emitting devices

A glass substrate coated with a thin film of indium tin oxide (ITO) to a thickness of 1500 Å was washed with distilled water ultrasonic waves. After washing with distilled water was completed, it was ultrasonically washed with a solvent such as acetone, methanol, isopropyl alcohol, and the like, and dried, and then treated with ultraviolet ozone (UVO) for 5 minutes using ultraviolet (UV) in a UV cleaner. Thereafter, the substrate was transferred to a plasma cleaner (PT), and then plasma-treated in a vacuum for the work function of ITO and the removal of the residual film, and transferred to a thermal deposition equipment for organic deposition.

2-TNATA (4,4',4"-tris[2-naphthyl(phenyl)amino]triphenylamine) and NPB (N,N'-di(1-naphthyl)-N,N'-diphenyl-(1,1'-biphenyl)-4,4'-diamine) were formed on the ITO transparent electrode (anode) as a hole injection layer and a hole transport layer, which are a common layer, respectively.

A light-emitting layer was thermally vacuum deposited thereon as follows. The light-emitting layer was formed by doping (piq)₂(Ir) (acac) to the host at 3% using the compound described in Table 5 below as a red host and (piq)₂(Ir) (acac) as a red phosphorescent dopant and depositing it to 500 Å. Thereafter, BCP was deposited to 60 Å as a hole-blocking layer, and Alq₃ was deposited to 200 Å thereon as an electron transport layer. Finally, lithium fluoride (LiF) was deposited to a thickness of 10 Å on the electron transport layer to form an electron injection layer, and then an aluminum (Al) cathode was deposited to a thickness of 1,200 Å on the electron injection layer to form a cathode, thereby manufacturing an organic light-emitting device.

On the other hand, all organic compounds required for manufacturing OLED devices were vacuum sublimated and purified under 10⁻⁶ to 10⁻⁸ torr for each material before use in OLED manufacturing.

For the organic light-emitting device manufactured as described above, electroluminescence (EL) properties were measured with M7000 from McScience Inc., and based on the measured results, T₉₀ was measured when the reference luminance was 6,000 cd/m² through the lifetime measuring device (M6000) manufactured by McScience Inc. The properties of the organic light-emitting device of the present invention are as shown in Table 5 below.

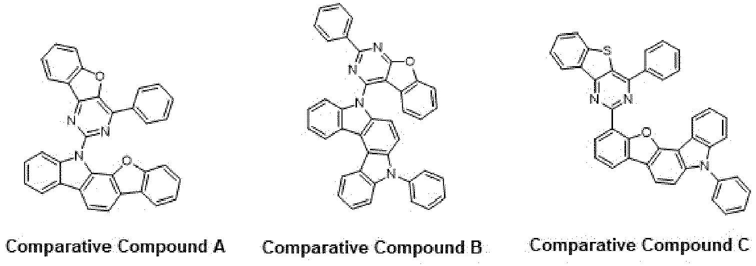

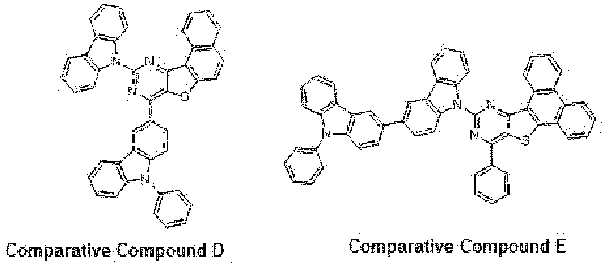

**[Table 5]**

| | Compound | Ratio (N:P) | Driving Voltage (V) | Efficiency (cd/A) | Color Coordinates (x, y) | Lifetime (T₉₀) |
|---|---|---|---|---|---|---|
| Comparative Example 1 | Comparative Compound A | - | 5.65 | 14.5 | 0.672, 0.328 | 50 |
| Comparative Example 2 | Comparative Compound B | - | 5.54 | 13.1 | 0.676, 0.324 | 45 |
| Comparative Example 3 | Comparative Compound C | - | 5.50 | 13.7 | 0.680, 0.320 | 57 |
| Comparative Example 4 | Comparative Compound D | - | 5.70 | 14.5 | 0.677, 0.322 | 55 |
| Comparative Example 5 | Comparative Compound E | - | 5.49 | 12.9 | 0.680, 0.320 | 30 |
| Example 1 | Compound 1 | - | 3.99 | 22.3 | 0.679, 0.321 | 105 |
| Example 2 | Compound 10 | - | 4.29 | 21.0 | 0.684, 0.316 | 98 |
| Example 3 | Compound 54 | - | 4.10 | 19.8 | 0.689, 0.311 | 118 |
| Example 4 | Compound 82 | - | 4.05 | 22.8 | 0.689, 0.311 | 120 |
| Example 5 | Compound 108 | - | 3.97 | 21.1 | 0.685, 0.314 | 95 |
| Example 6 | Compound 131 | - | 3.94 | 19.5 | 0.678, 0.321 | 97 |
| Example 7 | Compound 164 | - | 3.97 | 18.7 | 0.679, 0.321 | 116 |
| Example 8 | Compound 213 | - | 4.21 | 18.5 | 0.685, 0.315 | 126 |
| Example 9 | Compound 221 | - | 3.96 | 22.2 | 0.681, 0.319 | 91 |
| Example 10 | Compound 265 | - | 4.00 | 21.5 | 0.692, 0.308 | 120 |
| Example 11 | Compound 10: Compound H4 | (5:5) | 4.26 | 22.3 | 0.689, 0.311 | 90 |
| Example 12 | Compound 82: Compound H11 | (6:4) | 3.82 | 23.1 | 0.685, 0.314 | 105 |
| Example 13 | Compound 168: Compound H7 | (7:3) | 4.00 | 21.6 | 0.678, 0.321 | 88 |
| Example 14 | Compound 221: Compound H3 | (7:3) | 3.97 | 22.5 | 0.679, 0.321 | 85 |
| Example 15 | Compound 233: Compound H14 | (8:2) | 3.80 | 23.3 | 0.685, 0.315 | 96 |

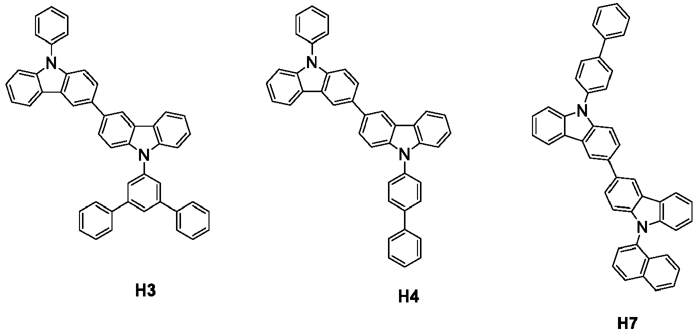

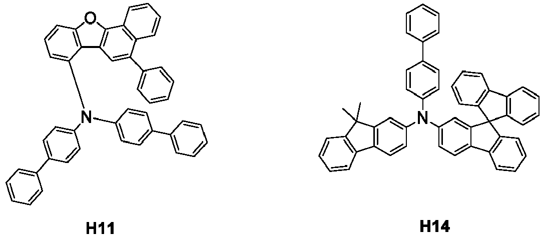

### [Description of Symbols]

| | | | |
|---|---|---|---|
| 100: | Substrate | 200: | Anode |
| 300: | Organic layer | 301: | Hole injection layer |
| 302: | Hole transport layer | 303: | Light-emitting layer |
| 304: | Hole-blocking layer | 305: | Electron transport layer |
| 306: | Electron injection layer | 400: | Cathode |

## Claims

1. A heterocyclic compound represented by following Formula 1: 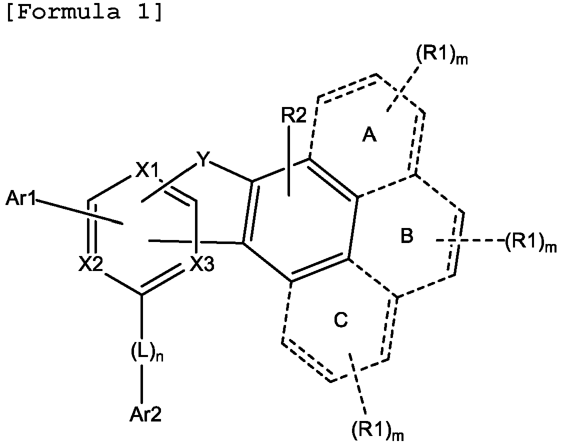 wherein,
X1, X2, and X3 are the same as or different from each other and are each independently N or C,
Y is NR20, O, or S, wherein R20 is hydrogen, deuterium, a substituted or unsubstituted C1 to C10 alkyl group, a substituted or unsubstituted C6 to C60 aryl group, or a substituted or unsubstituted C2 to C60 heteroaryl group,
Rings A, B, and C are each independently present or absent, with the proviso that at least one of these rings is present,
L is a direct bond, a substituted or unsubstituted C6 to C60 arylene group, or a substituted or unsubstituted C2 to C60 heteroarylene group,
Ar1 is a substituted or unsubstituted C6 to C60 aryl group or a substituted or unsubstituted C2 to C60 heteroaryl group,
R1 and R2 above are the same as or different from each other and are each independently hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; or -NR21R22, wherein R21 and R22 are the same as or different from each other and are each independently a substituted or unsubstituted C1 to C10 alkyl group, a substituted or unsubstituted C6 to C60 aryl group, or a substituted or unsubstituted C2 to C60 heteroaryl group, with the proviso that when two or more of R1 are present, each R1 is the same as or different from each other,
m is each independently an integer from 0 to 4, with the proviso that when m is 2 or more, each R1 is the same as or different from each other,
n is an integer from 0 to 3, with the proviso that when n is 2 or more, each L is the same as or different from each other, and
Ar2 is represented by following Formula 2 or 3: 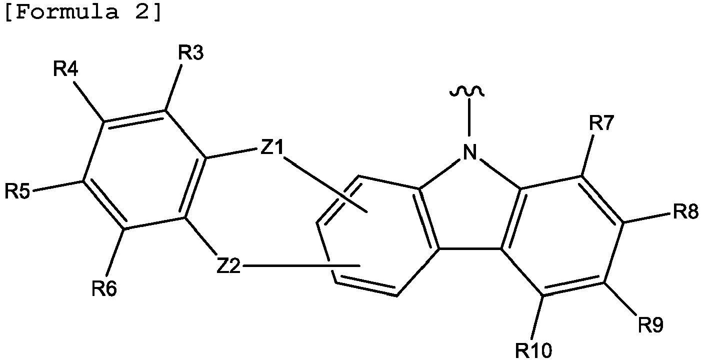 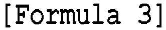 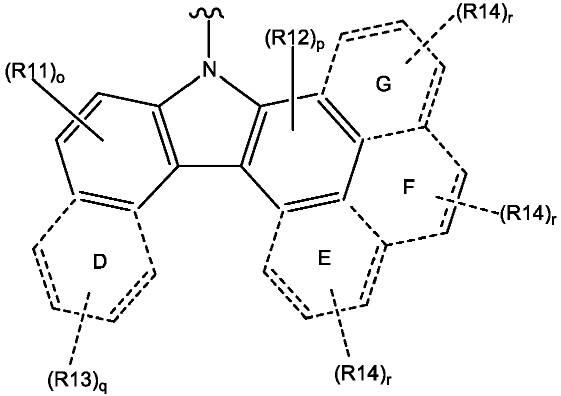 wherein,
Z1 and Z2 are the same as or different from each other and are each independently a direct bond, NR23, O, S, or CR24R25, wherein R23 is hydrogen, deuterium, a substituted or unsubstituted C1 to C10 alkyl group, a substituted or unsubstituted a C6 to C60 aryl group, or a substituted or unsubstituted C2 to C60 heteroaryl group; R24 and R25 are the same as or different from each other and are each independently a substituted or unsubstituted C1 to C10 alkyl group, a substituted or unsubstituted C6 to C60 aryl group, or a substituted or unsubstituted C2 to C60 heteroaryl group; and R24 and R25 may be combined with each other to form a substituted or unsubstituted C6 to C60 aromatic hydrocarbon ring or a substituted or unsubstituted C2 to C60 heterocycle,
R3 to R14 are the same as or different from each other and are each independently hydrogen; deuterium, halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; or -NR21R22, wherein R21 and R22 are the same as or different from each other and are each independently a substituted or unsubstituted C1 to C10 alkyl group, a substituted or unsubstituted C6 to C60 aryl group, or a substituted or unsubstituted C2 to C60 heteroaryl group, with the proviso that when two or more of R14 are present, each R14 is the same as or different from each other,
rings D, E, F and G are each independently present or absent, with the proviso that at least one of these rings is present,
o, p, q, and r are the same as or different from each other and are each independently an integer from 0 to 4, with the proviso that when each of o, p, q, and r is 2 or more, R11, R12, R13, and R14 is each independently selected.

2. The heterocyclic compound according to claim 1, **characterized in that** R1 to R14 are the same as or different from each other and are each independently hydrogen, deuterium, a substituted or unsubstituted C6 to C60 aryl group, a substituted or unsubstituted C2 to C60 heteroaryl group, or -NR21R22, wherein R21 and R22 are the same as or different from each other and are each independently a substituted or unsubstituted C6 to C60 aryl group or a substituted or unsubstituted C2 to C60 heteroaryl group.

3. The heterocyclic compound according to claim 1, **characterized in that** Ar1 is a substituted or unsubstituted C6 to C30 aryl group or a substituted or unsubstituted C2 to C30 heteroaryl group.

4. The heterocyclic compound according to claim 1, **characterized in that** Formula 3 is represented by any one of following Formulas 3-1 to 3-3: 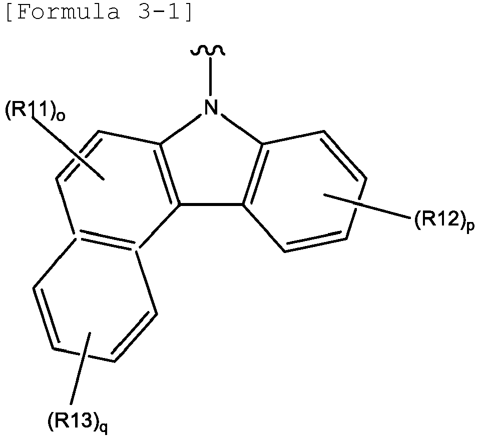 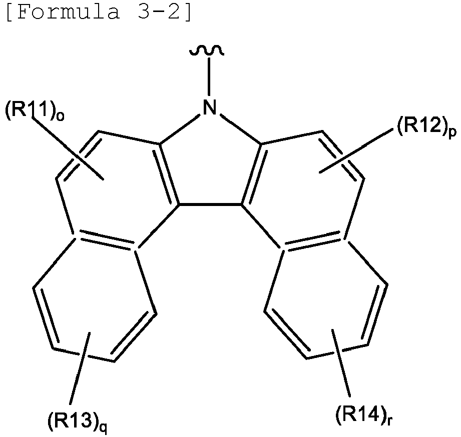 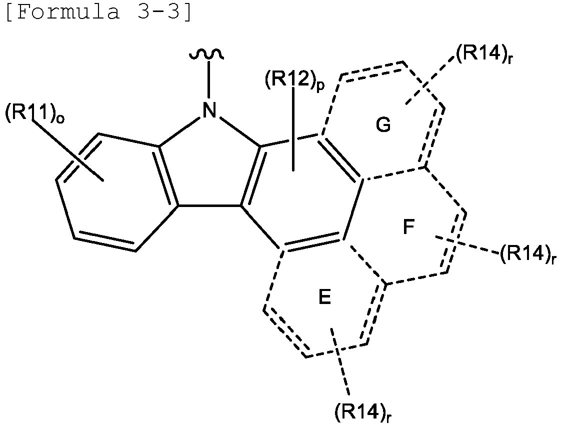 wherein,
each substituent is as defined in Formula 3,
rings E, F and G are each independently present or absent, with the proviso that at least one of these rings is present.

5. The heterocyclic compound according to claim 1, **characterized in that** any one of X1 and X2 is N and the other is C, and X3 is N.

6. The heterocyclic compound according to claim 1, **characterized in that** the heterocyclic compound represented by Formula 1 is a compound represented by any one of the following compounds:

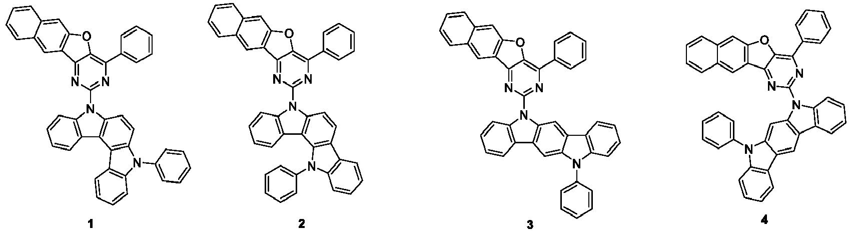

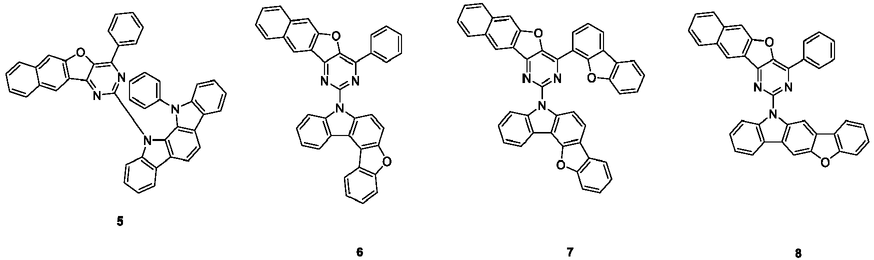

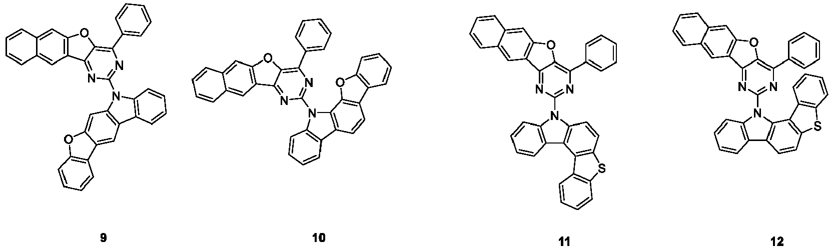

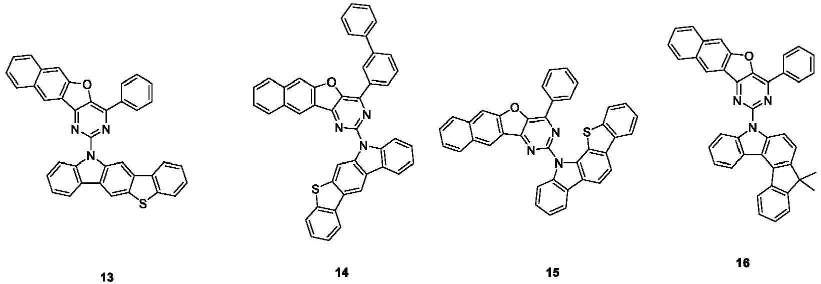

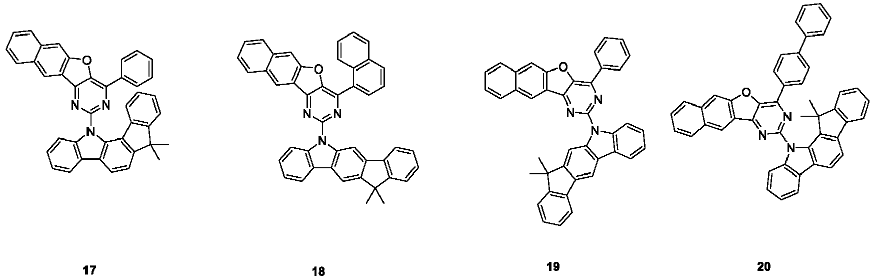

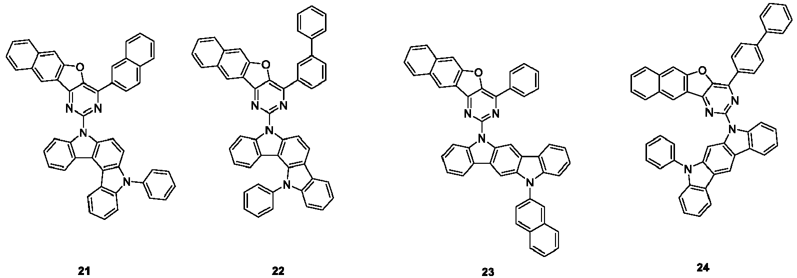

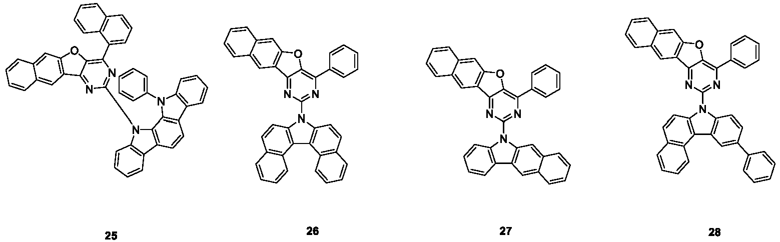

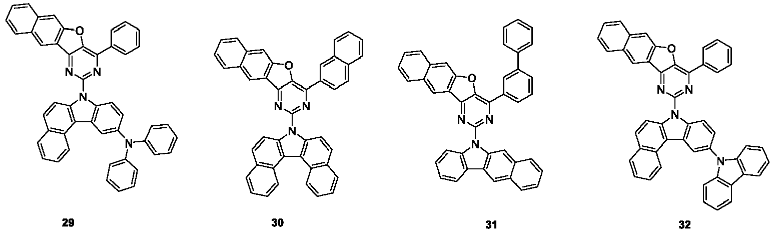

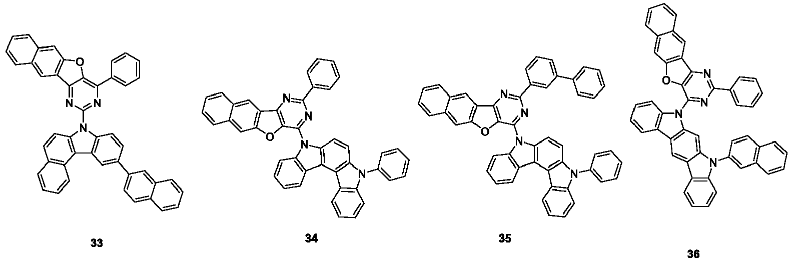

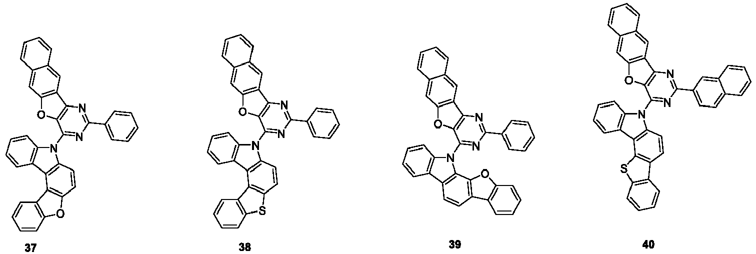

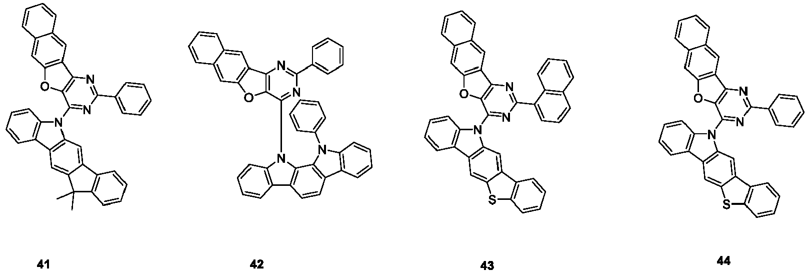

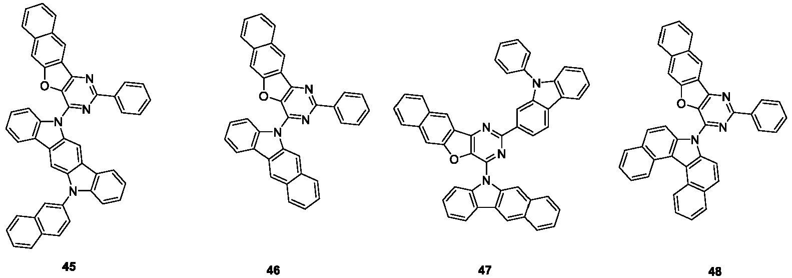

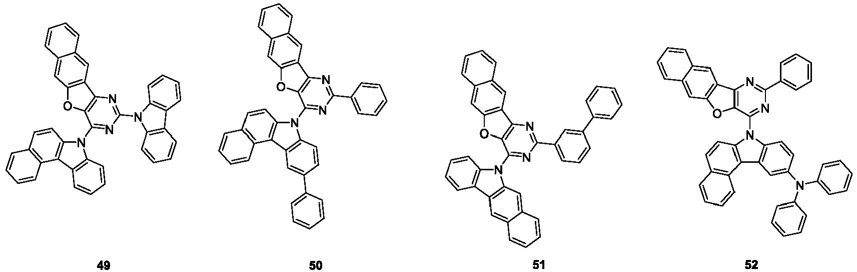

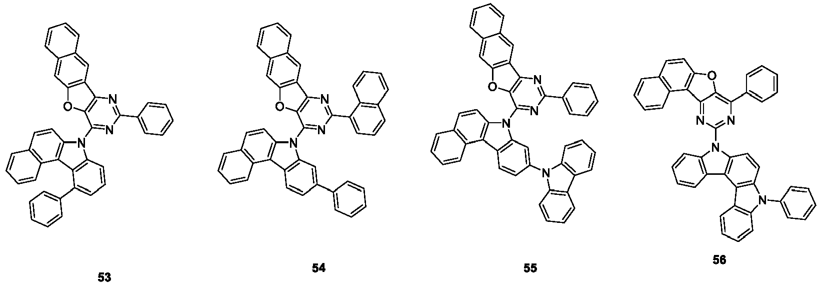

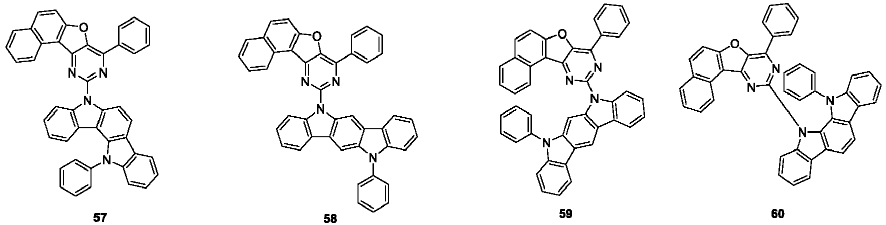

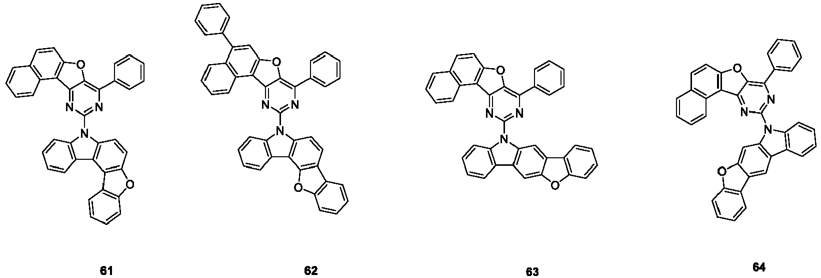

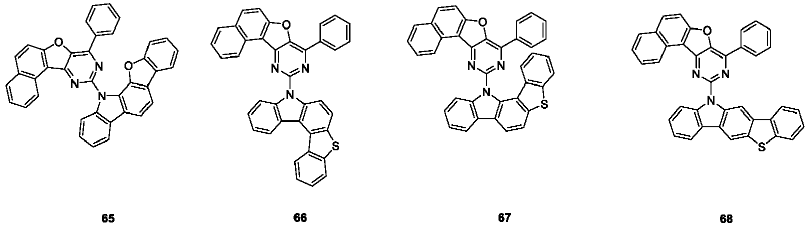

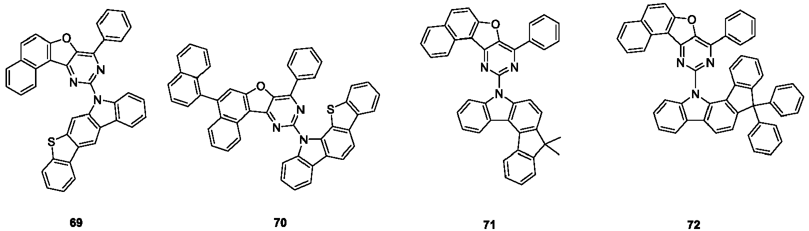

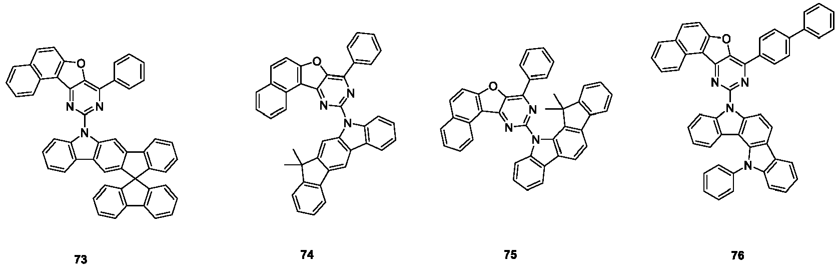

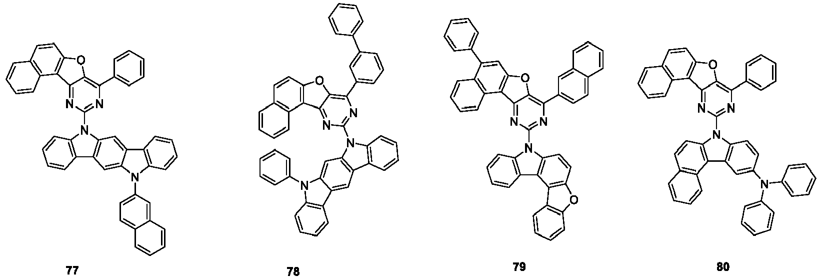

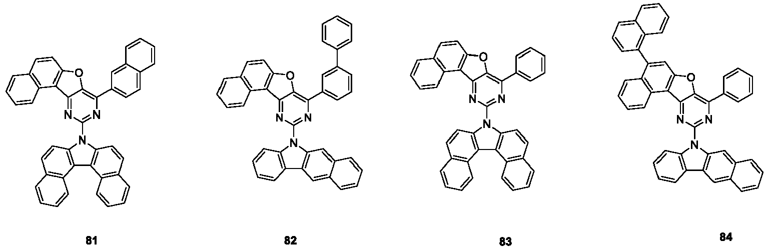

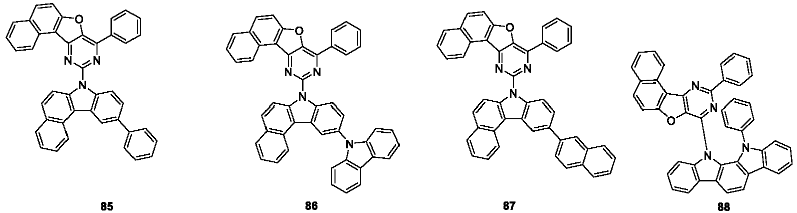

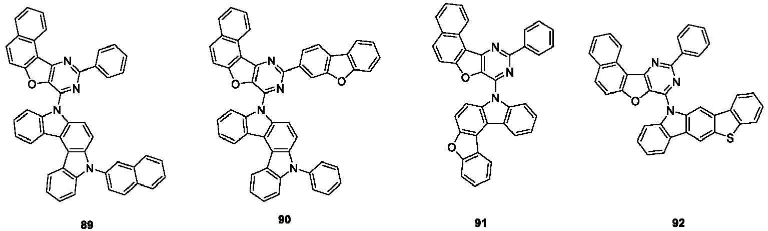

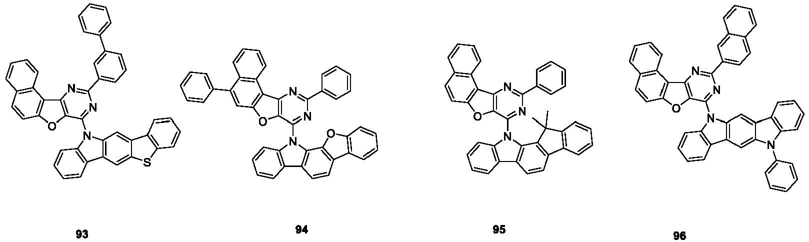

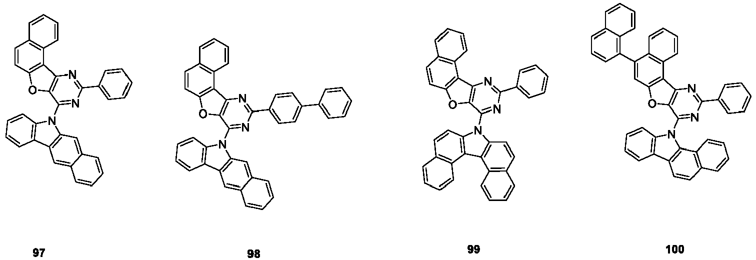

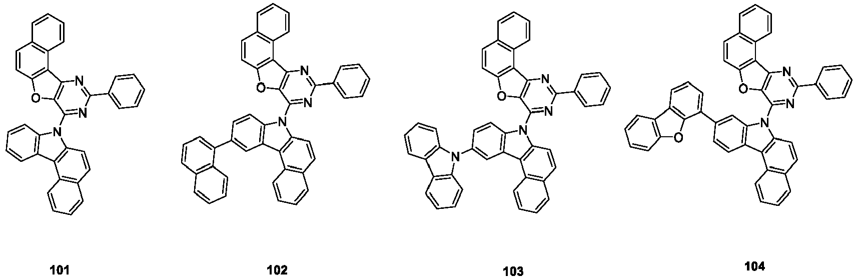

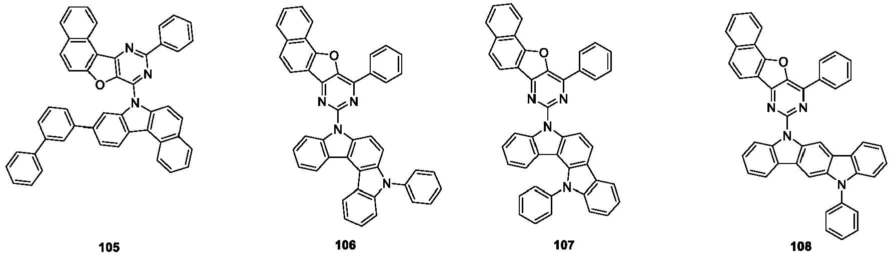

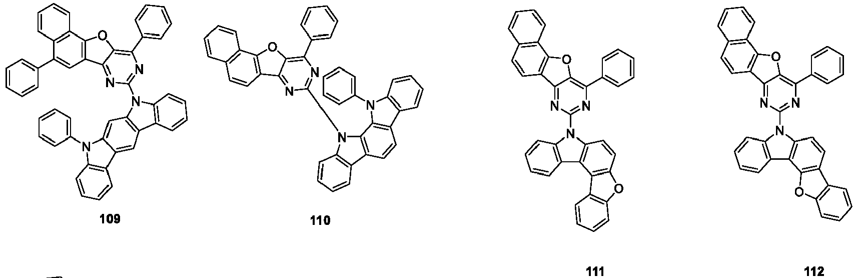

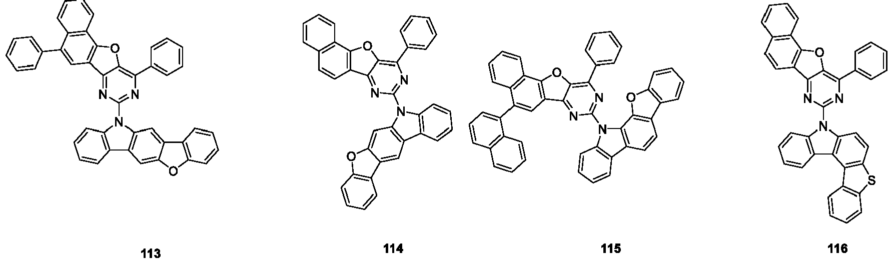

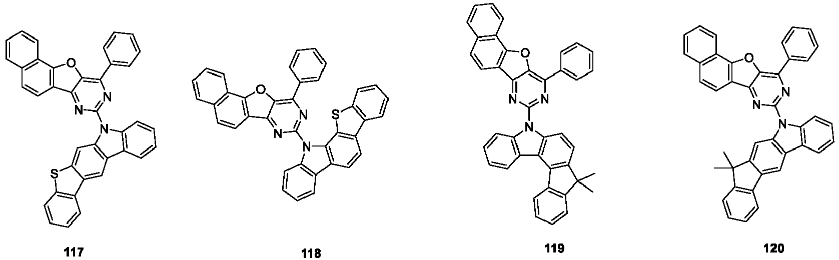

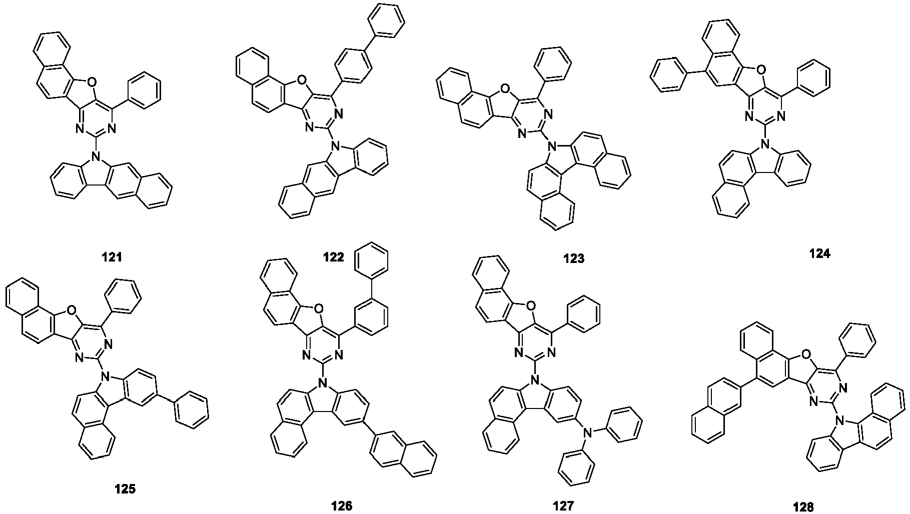

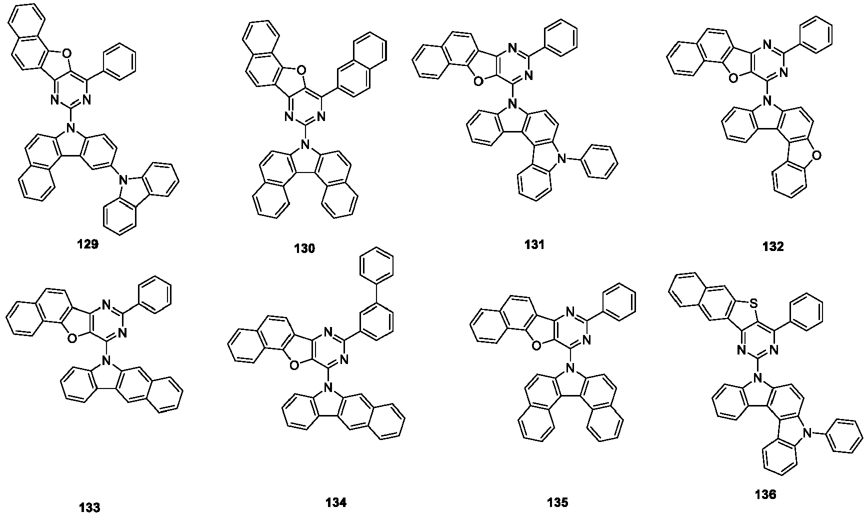

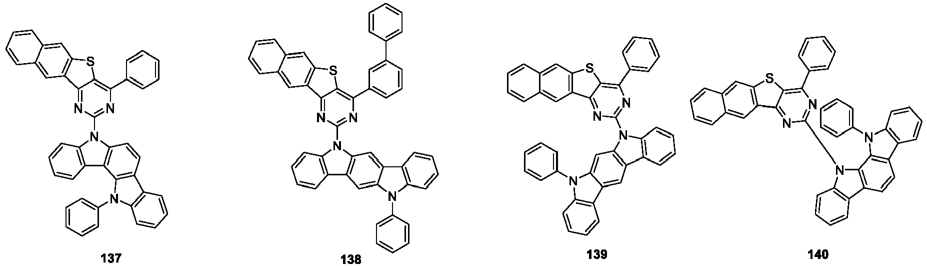

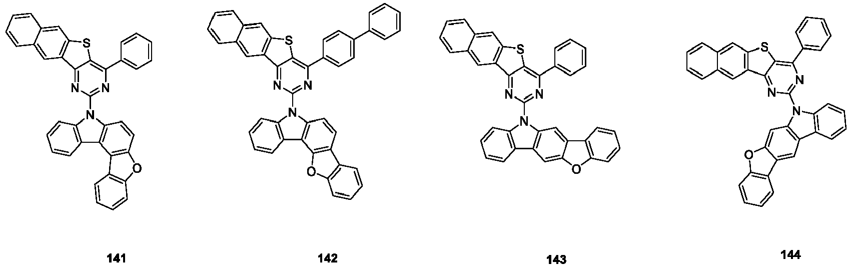

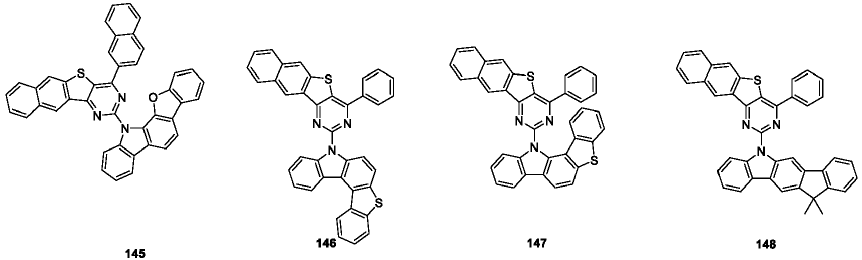

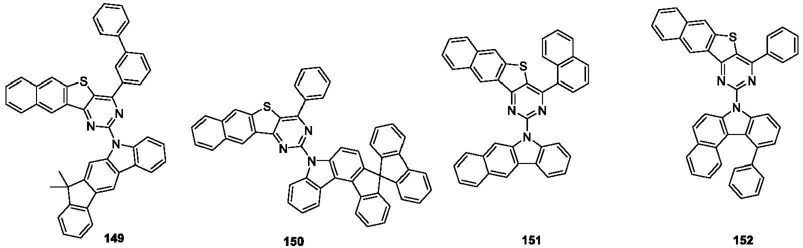

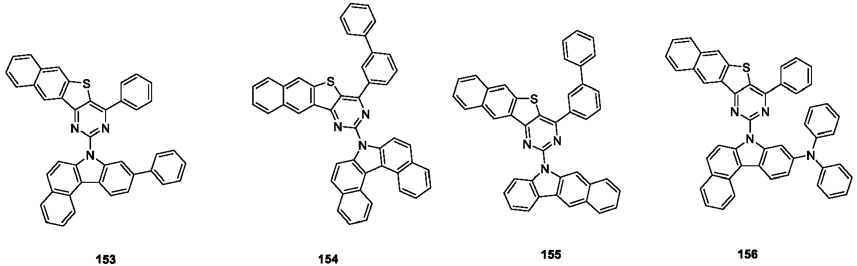

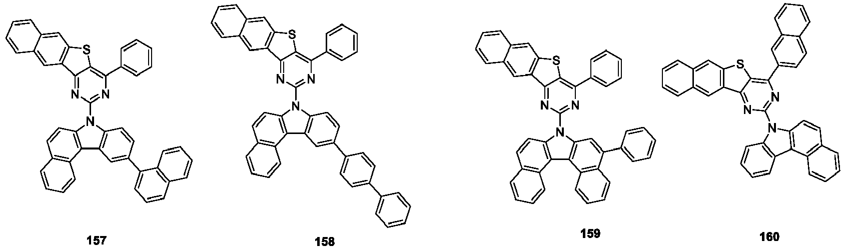

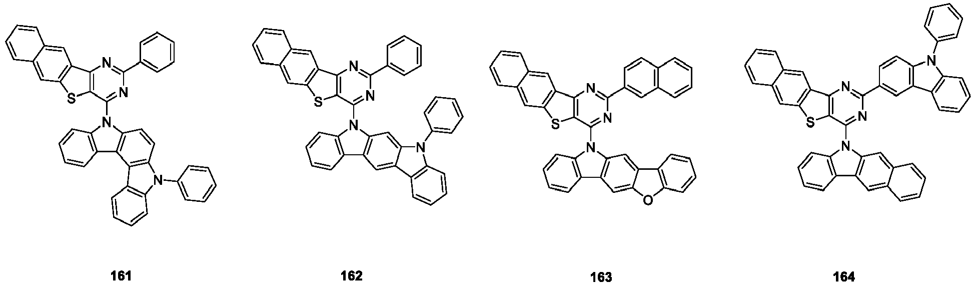

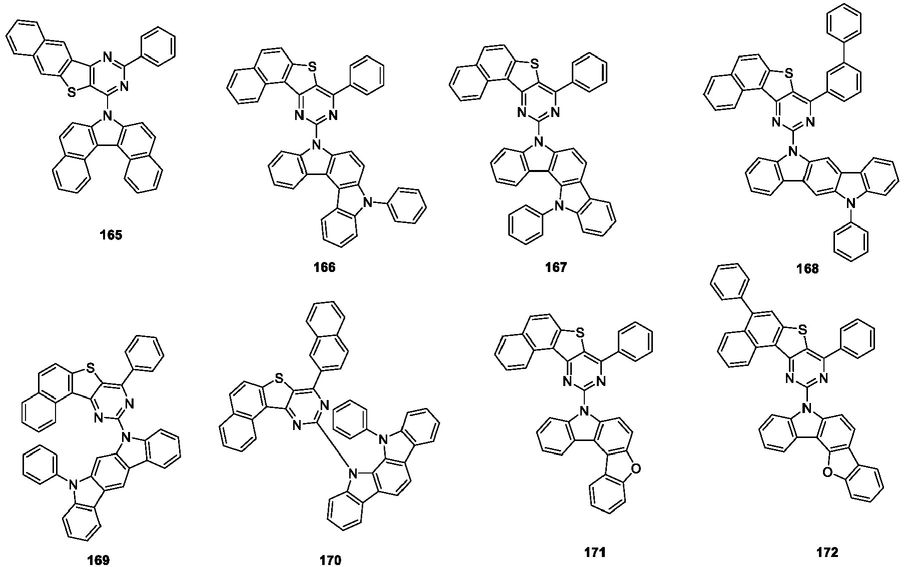

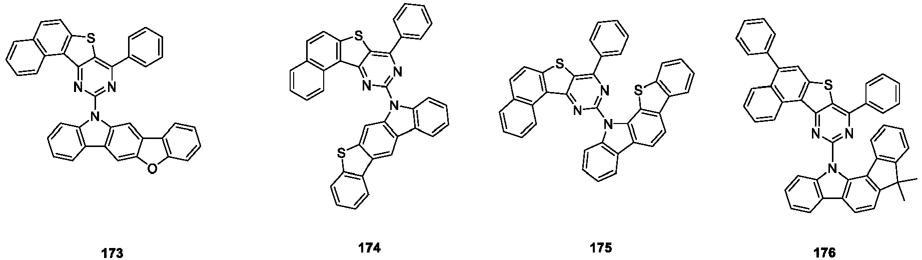

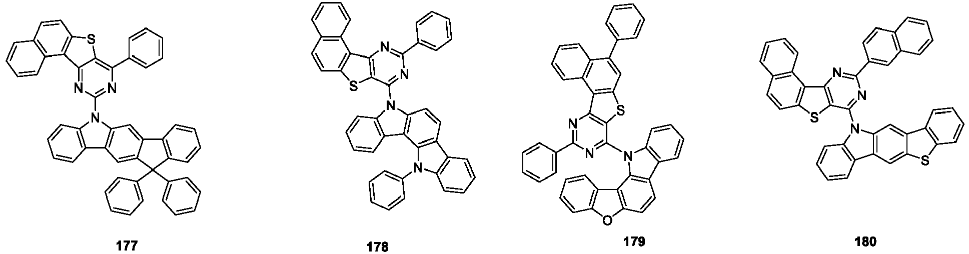

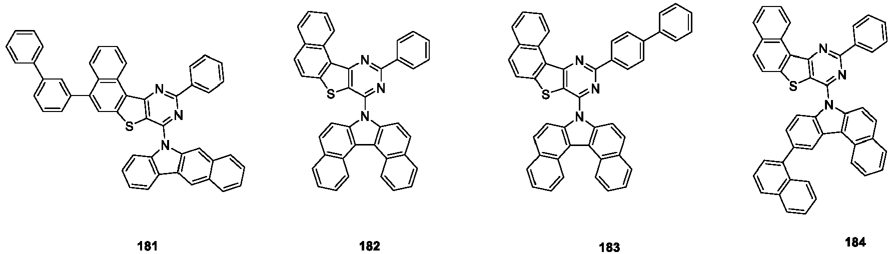

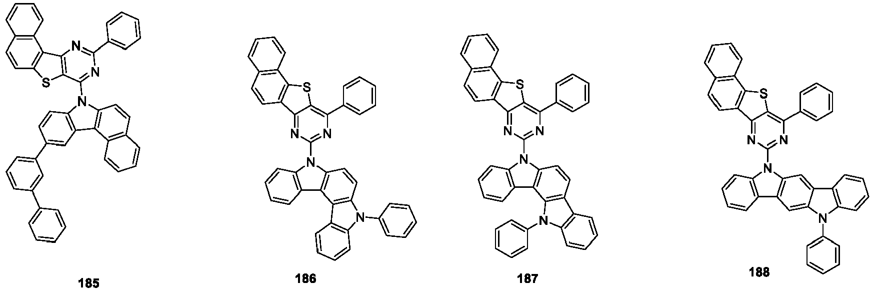

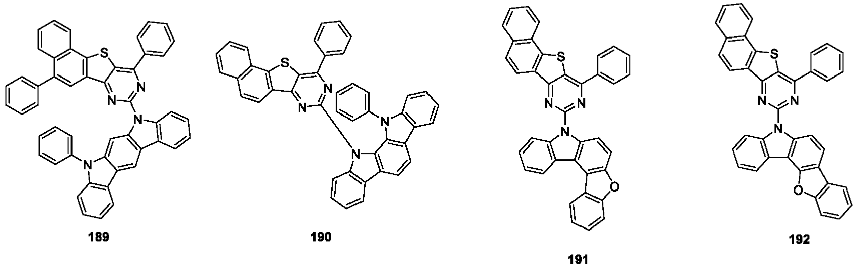

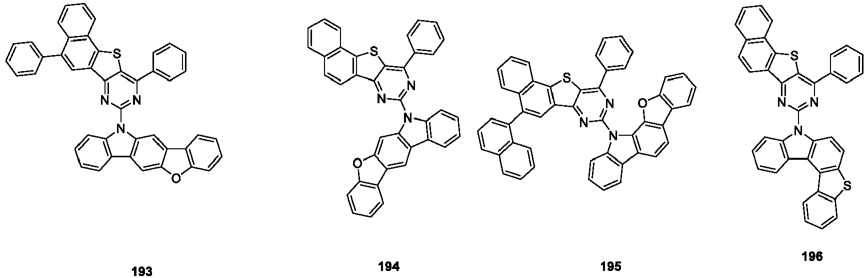

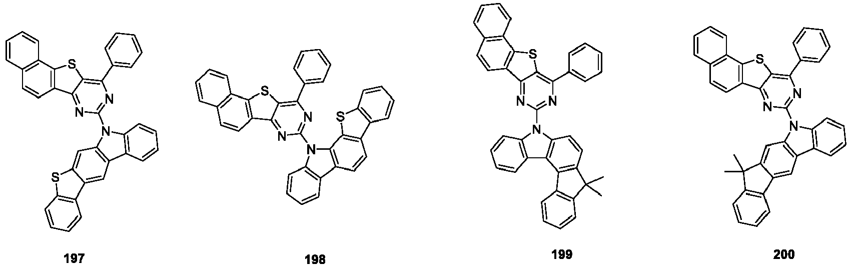

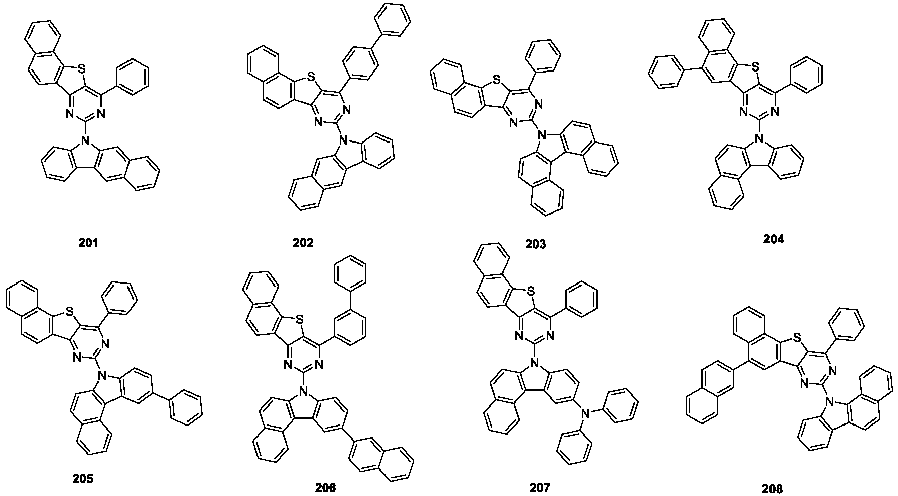

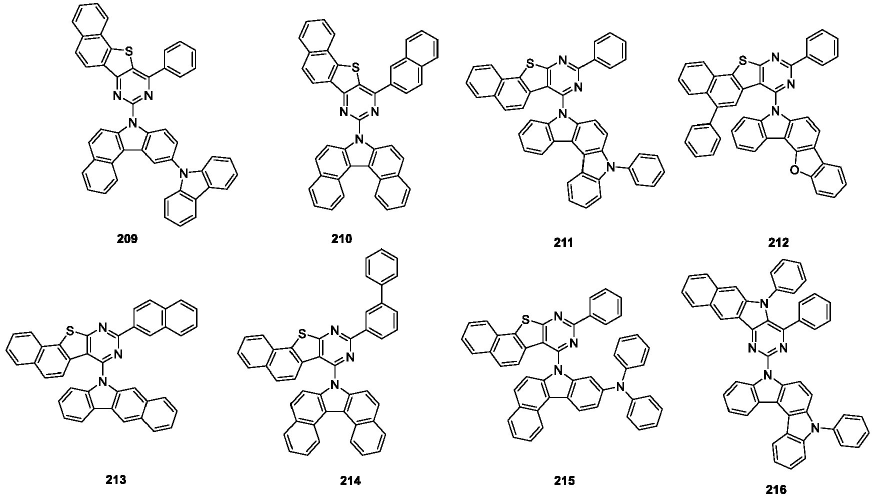

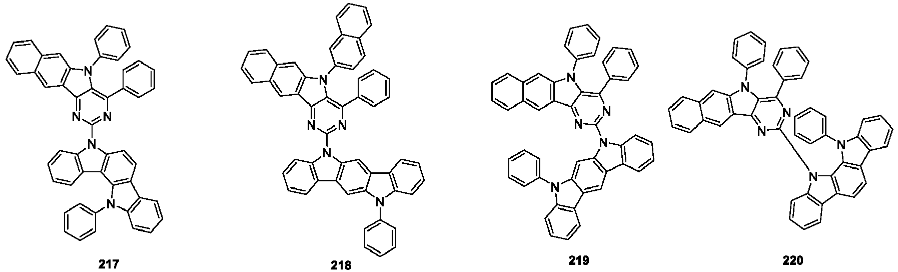

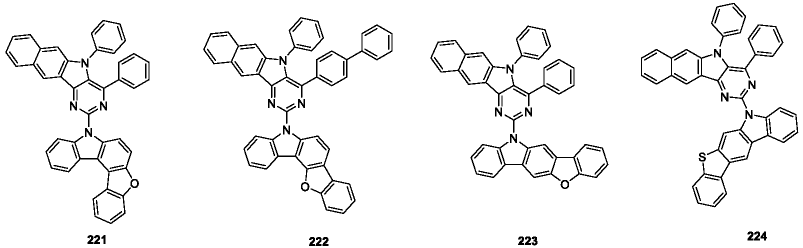

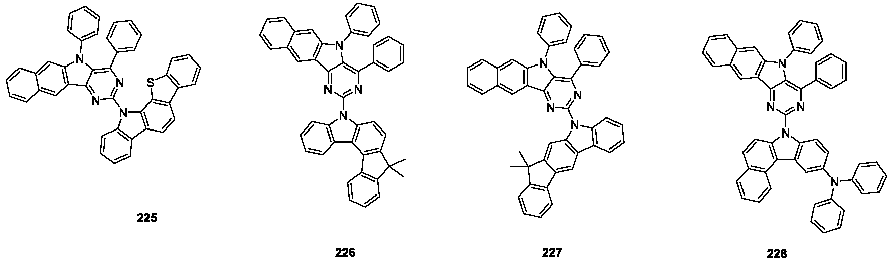

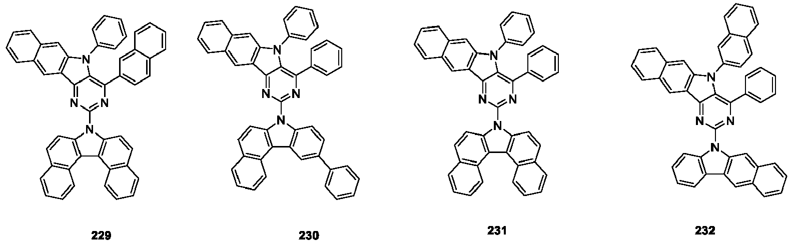

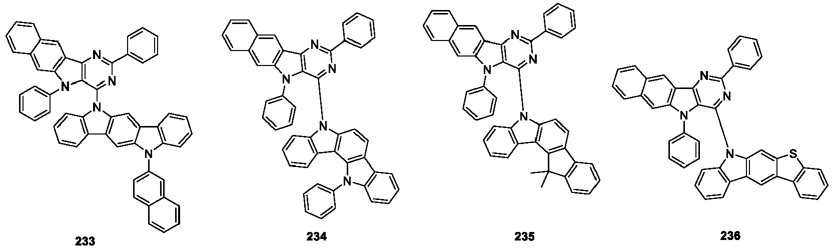

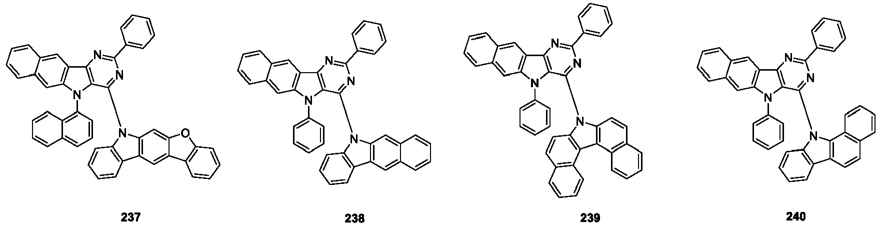

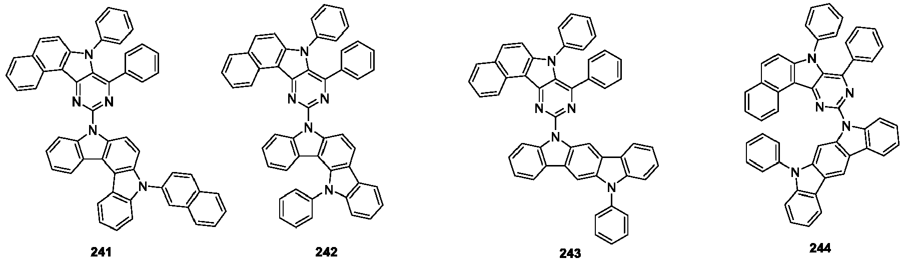

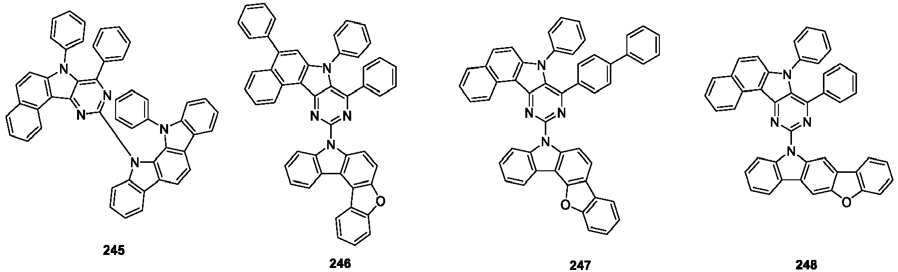

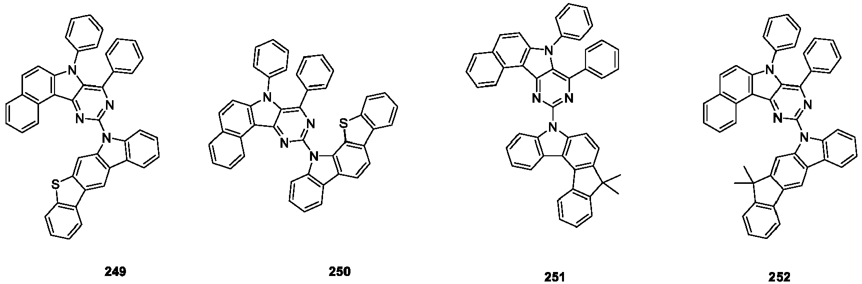

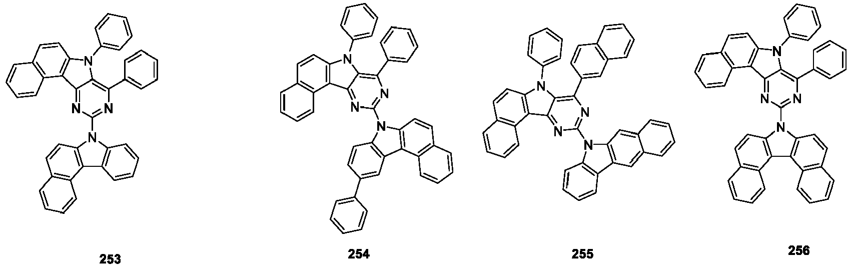

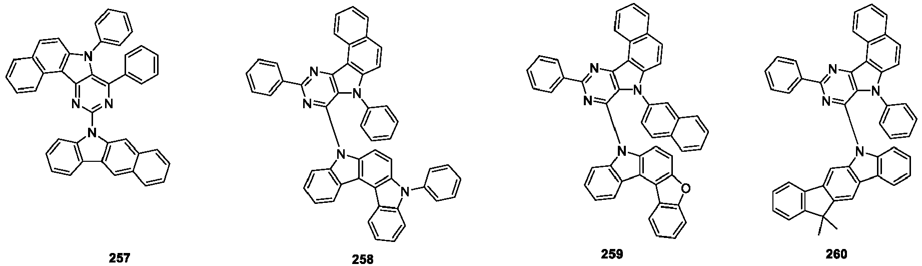

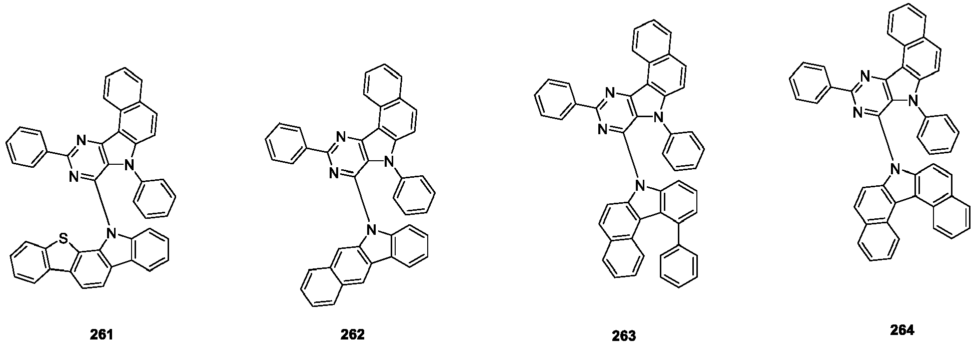

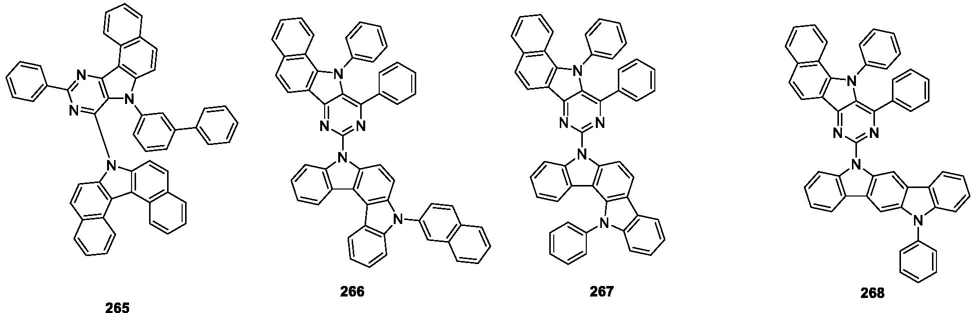

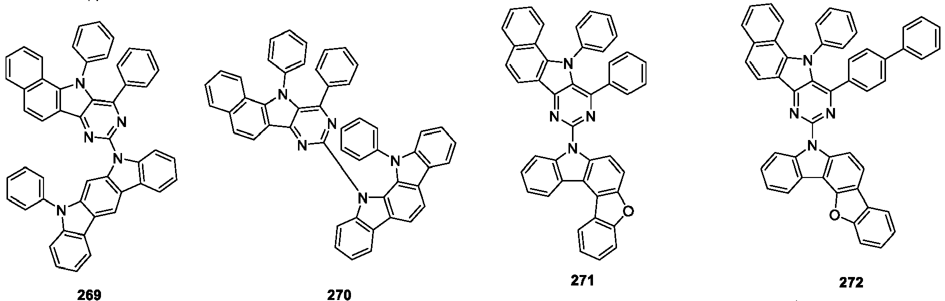

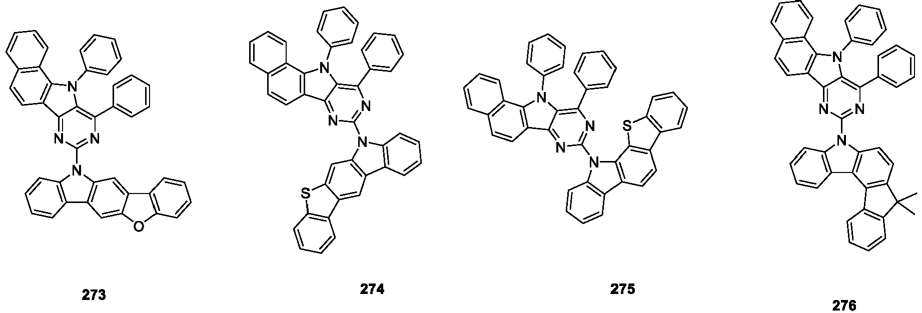

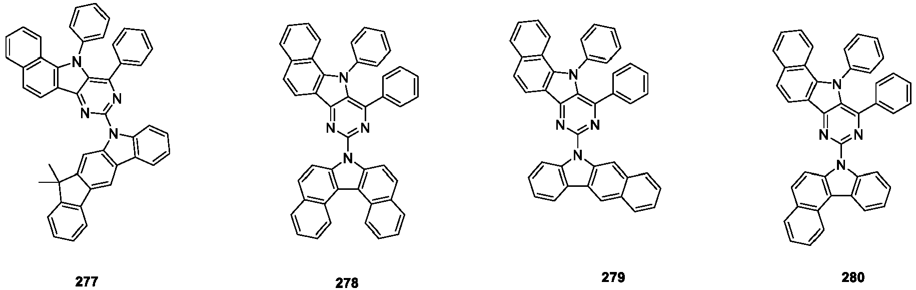

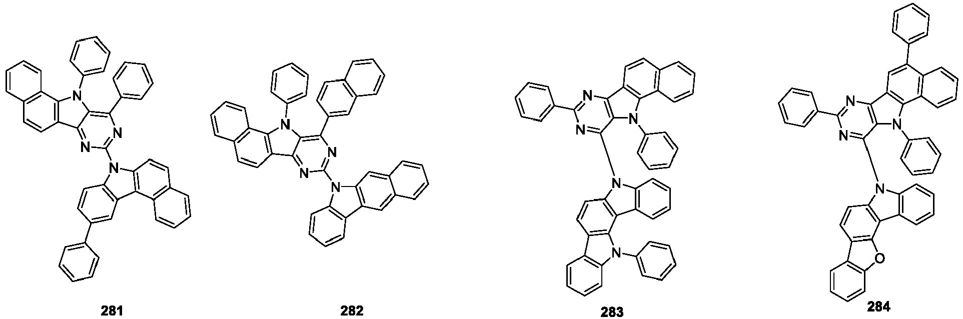

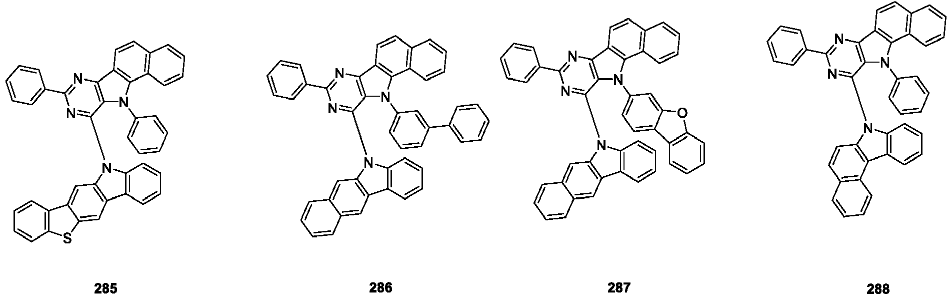

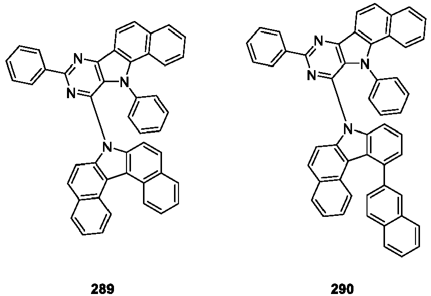

7. An organic light-emitting device comprising:
a first electrode;
a second electrode provided to face the first electrode; and
one or more organic layers provided between the first electrode and the second electrode, wherein the organic layers comprise the heterocyclic compound according to any one of claims 1 to 6.

8. The organic light-emitting device according to claim 7, characterize in that the organic layer comprises a light-emitting layer, wherein the light-emitting layer comprises the heterocyclic compound.

9. The organic light-emitting device according to claim 8, characterize in that the light-emitting layer comprises a host material, wherein the host material comprises the heterocyclic compound.

10. The organic light-emitting device according to claim 8, characterize in that the organic layer further comprises one or more layers selected from an electron injection layer, an electron transport layer, a hole-blocking layer, an electron-blocking layer, a hole transport layer, and a hole injection layer.

11. The organic light-emitting device according to claim 10, characterize in that the organic layer comprises an electron injection layer, an electron transport layer, and a hole-blocking layer, and one or more of the layers comprises the heterocyclic compound.

12. The organic light-emitting device according to claim 10, characterize in that the organic layer comprises an electron-blocking layer, a hole transport layer, and a hole injection layer, and one or more of the layers comprises the heterocyclic compound.
